(19) Europäisches Patentamt
European Patent Office
Office européen des brevets

(11) EP 3 144 323 A2

(12) # EUROPEAN PATENT APPLICATION

(43) Date of publication:
**22.03.2017 Bulletin 2017/12**

(21) Application number: **16191498.1**

(22) Date of filing: **08.12.2010**

(51) Int Cl.:
***C07K 16/30*** *(2006.01)* ***A61K 39/395*** *(2006.01)*
***G01N 33/53*** *(2006.01)* ***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2009 EP 09178474**
**26.07.2010 EP 10170797**

(62) Document number(s) of the earlier application(s) in accordance with Art. 76 EPC:
**10798996.4 / 2 510 012**

(27) Previously filed application:
**08.12.2010 PCT/EP2010/069216**

(71) Applicant: **Bayer Pharma Aktiengesellschaft**
**13353 Berlin (DE)**

(72) Inventors:
- **LINDEN, Lars**
**58285 Gevelsberg (DE)**
- **CAO, Yong-Jiang**
**13127 Berlin (DE)**
- **LEDER, Gabriele**
**14163 Berlin (DE)**
- **STELTE-LUDWIG, Beatrix**
**42489 Wülfrath (DE)**
- **HARRENGA, Axel**
**42113 Wuppertal (DE)**
- **FINNERN, Ricarda**
**52074 Aachen (DE)**
- **DITTMER, Frank**
**40627 Düsseldorf (DE)**
- **MAYER-BARTSCHMID, Anke**
**42489 Wülfrath (DE)**
- **FRANZ, Juergen**
**58456 Witten (DE)**
- **GREVEN, Simone**
**41541 Dormagen (DE)**
- **WILLUDA, Jörg**
**16548 Glienicke/ Nordbahn (DE)**
- **TEBBE, Jan**
**50733 Köln (DE)**

(74) Representative: **BIP Patents**
**c/o Bayer Intellectual Property GmbH**
**Alfred-Nobel-Straße 10**
**40789 Monheim am Rhein (DE)**

Remarks:
This application was filed on 29.09.2016 as a divisional application to the application mentioned under INID code 62.

(54) ## ANTI-C4.4A ANTIBODIES AND USES THEREOF

(57) The present invention provides recombinant antigen-binding regions and antibodies and functional fragments containing such antigen-binding regions that are specific for the membrane-anchored, 29 kDa polypeptide named C4.4a , which is over expressed in tumors.

C4.4a has a high abundance in metastases of several cancer types. The antibodies, accordingly, can be used to treat these and other disorders and conditions. Antibodies of the invention also can be used in the diagnostics field, as well as for further investigating the role of C4.4a in the progression of disorders associated with cancer. The invention also provides nucleic acid sequences encoding the foregoing antibodies, vectors containing the same, pharmaceutical compositions and kits with instructions for use.

EP 3 144 323 A2

SEQUENCE LISTING

<110> Bayer Schering Pharma AG

<120> Anti-C4.4a Antibodies and Uses Thereof

<130> BHC 09 1 039

<160> 345

<170> PatentIn version 3.5

<210> 1
<211> 278
<212> PRT
<213> Homo Sapiens

<400> 1

```
Leu Glu Cys Tyr Ser Cys Val Gln Lys Ala Asp Asp Gly Cys Ser Pro
1               5                   10                  15

Asn Lys Met Lys Thr Val Lys Cys Ala Pro Gly Val Asp Val Cys Thr
            20                  25                  30

Glu Ala Val Gly Ala Val Glu Thr Ile His Gly Gln Phe Ser Leu Ala
        35                  40                  45

Val Arg Gly Cys Gly Ser Gly Leu Pro Gly Lys Asn Asp Arg Gly Leu
    50                  55                  60

Asp Leu His Gly Leu Leu Ala Phe Ile Gln Leu Gln Gln Cys Ala Gln
65                  70                  75                  80

Asp Arg Cys Asn Ala Lys Leu Asn Leu Thr Ser Arg Ala Leu Asp Pro
                85                  90                  95

Ala Gly Asn Glu Ser Ala Tyr Pro Pro Asn Gly Val Glu Cys Tyr Ser
            100                 105                 110

Cys Val Gly Leu Ser Arg Glu Ala Cys Gln Gly Thr Ser Pro Pro Val
        115                 120                 125

Val Ser Cys Tyr Asn Ala Ser Asp His Val Tyr Lys Gly Cys Phe Asp
    130                 135                 140

Gly Asn Val Thr Leu Thr Ala Ala Asn Val Thr Val Ser Leu Pro Val
145                 150                 155                 160

Arg Gly Cys Val Gln Asp Glu Phe Cys Thr Arg Asp Gly Val Thr Gly
                165                 170                 175
```

Pro Gly Phe Thr Leu Ser Gly Ser Cys Cys Gln Gly Ser Arg Cys Asn
180 185 190

Ser Asp Leu Arg Asn Lys Thr Tyr Phe Ser Pro Arg Ile Pro Pro Leu
195 200 205

Val Arg Leu Pro Pro Pro Glu Pro Thr Thr Val Ala Ser Thr Thr Ser
210 215 220

Val Thr Thr Ser Thr Ser Ala Pro Val Arg Pro Thr Ser Thr Thr Lys
225 230 235 240

Pro Met Pro Ala Pro Thr Ser Gln Thr Pro Arg Gln Gly Val Glu His
245 250 255

Glu Ala Ser Arg Asp Glu Glu Pro Arg Leu Thr Gly Gly Ala Ala Gly
260 265 270

His Gln Asp Arg Ser Asn
275

<210> 2
<211> 293
<212> PRT
<213> Mus musculus

<400> 2

Leu Glu Cys Tyr Ser Cys Val Gln Lys Ala Asp Asp Gly Cys Ser Pro
1 5 10 15

His Arg Met Lys Thr Val Lys Cys Gly Pro Gly Val Asp Val Cys Thr
20 25 30

Glu Ala Val Gly Ala Val Glu Thr Ile His Gly Gln Phe Ser Val Ala
35 40 45

Val Arg Gly Cys Gly Ser Gly Ile Pro Gly Lys Asn Asp Arg Gly Leu
50 55 60

Asp Leu His Gly Leu Leu Ala Phe Phe Gln Leu Gln Gln Cys Ser Glu
65 70 75 80

Asp Arg Cys Asn Ala Lys Leu Asn Leu Thr Leu Arg Gly Leu Asn Pro
85 90 95

Ala Gly Asn Glu Ser Ala Tyr Glu Pro Asn Gly Ala Glu Cys Tyr Ser
100 105 110

Cys Val Gly Leu Ser Arg Glu Lys Cys Gln Gly Ser Met Pro Pro Val
115 120 125

Val Asn Cys Tyr Asn Ala Ser Gly Arg Val Tyr Lys Gly Cys Phe Asp
130 135 140

Gly Asn Val Thr Leu Thr Ala Ala Asn Val Thr Val Ser Leu Pro Val
145 150 155 160

Arg Gly Cys Val Gln Asp Glu Thr Cys Thr Arg Asp Gly Val Thr Gly
165 170 175

Pro Gly Phe Thr Leu Ser Gly Ser Cys Cys Gln Gly Pro Arg Cys Asn
180 185 190

Ala Asp Leu Arg Asn Lys Thr Tyr Phe Ser Pro Arg Ile Pro Pro Leu
195 200 205

Val Leu Leu Pro Pro Pro Thr Thr Ala Ala Pro Ser Thr Arg Ala Gln
210 215 220

Asn Ser Ser Ser Thr Thr Ser Thr Ala Ala Pro Thr Thr Thr Thr Ser
225 230 235 240

Ile Ile Lys Pro Thr Thr Ala Gln Ala Ser His Thr Ser Pro His Glu
245 250 255

Met Asp Leu Glu Val Ile Gln Glu Glu Gly Ala Ser Leu Ser Gly Gly
260 265 270

Ala Ala Gly His Gly Gly Thr Ala Gly His Gly Gly Ala Ala Gly His
275 280 285

Gln Asp Arg Ser Asn
290

<210> 3
<211> 346
<212> PRT
<213> Homo Sapiens

<400> 3

Met Asp Pro Ala Arg Lys Ala Gly Ala Gln Ala Met Ile Trp Thr Ala
1 5 10 15

Gly Trp Leu Leu Leu Leu Leu Leu Arg Gly Gly Ala Gln Ala Leu Glu
20 25 30

```
Cys Tyr Ser Cys Val Gln Lys Ala Asp Asp Gly Cys Ser Pro Asn Lys
        35                  40                  45

Met Lys Thr Val Lys Cys Ala Pro Gly Val Asp Val Cys Thr Glu Ala
    50                  55                  60

Val Gly Ala Val Glu Thr Ile His Gly Gln Phe Ser Leu Ala Val Arg
65                  70                  75                  80

Gly Cys Gly Ser Gly Leu Pro Gly Lys Asn Asp Arg Gly Leu Asp Leu
                85                  90                  95

His Gly Leu Leu Ala Phe Ile Gln Leu Gln Gln Cys Ala Gln Asp Arg
            100                 105                 110

Cys Asn Ala Lys Leu Asn Leu Thr Ser Arg Ala Leu Asp Pro Ala Gly
        115                 120                 125

Asn Glu Ser Ala Tyr Pro Pro Asn Gly Val Glu Cys Tyr Ser Cys Val
    130                 135                 140

Gly Leu Ser Arg Glu Ala Cys Gln Gly Thr Ser Pro Pro Val Val Ser
145                 150                 155                 160

Cys Tyr Asn Ala Ser Asp His Val Tyr Lys Gly Cys Phe Asp Gly Asn
                165                 170                 175

Val Thr Leu Thr Ala Ala Asn Val Thr Val Ser Leu Pro Val Arg Gly
            180                 185                 190

Cys Val Gln Asp Glu Phe Cys Thr Arg Asp Gly Val Thr Gly Pro Gly
        195                 200                 205

Phe Thr Leu Ser Gly Ser Cys Cys Gln Gly Ser Arg Cys Asn Ser Asp
    210                 215                 220

Leu Arg Asn Lys Thr Tyr Phe Ser Pro Arg Ile Pro Pro Leu Val Arg
225                 230                 235                 240

Leu Pro Pro Pro Glu Pro Thr Thr Val Ala Ser Thr Thr Ser Val Thr
                245                 250                 255

Thr Ser Thr Ser Ala Pro Val Arg Pro Thr Ser Thr Thr Lys Pro Met
            260                 265                 270

Pro Ala Pro Thr Ser Gln Thr Pro Arg Gln Gly Val Glu His Glu Ala
        275                 280                 285
```

Ser Arg Asp Glu Glu Pro Arg Leu Thr Gly Gly Ala Ala Gly His Gln
290 295 300

Asp Arg Ser Asn Ser Gly Gln Tyr Pro Ala Lys Gly Gly Pro Gln Gln
305 310 315 320

Pro His Asn Lys Gly Cys Val Ala Pro Thr Ala Gly Leu Ala Ala Leu
325 330 335

Leu Leu Ala Val Ala Ala Gly Val Leu Leu
340 345

<210> 4
<211> 363
<212> PRT
<213> Mus musculus

<400> 4

Met Asp Ala Ala Arg Arg Gly Asp Thr Gln Pro Val Met Trp Thr Thr
1 5 10 15

Gly Trp Leu Leu Leu Leu Pro Leu Leu Leu Cys Glu Gly Ala Gln Ala
20 25 30

Leu Glu Cys Tyr Ser Cys Val Gln Lys Ala Asp Asp Gly Cys Ser Pro
35 40 45

His Arg Met Lys Thr Val Lys Cys Gly Pro Gly Val Asp Val Cys Thr
50 55 60

Glu Ala Val Gly Ala Val Glu Thr Ile His Gly Gln Phe Ser Val Ala
65 70 75 80

Val Arg Gly Cys Gly Ser Gly Ile Pro Gly Lys Asn Asp Arg Gly Leu
85 90 95

Asp Leu His Gly Leu Leu Ala Phe Phe Gln Leu Gln Gln Cys Ser Glu
100 105 110

Asp Arg Cys Asn Ala Lys Leu Asn Leu Thr Leu Arg Gly Leu Asn Pro
115 120 125

Ala Gly Asn Glu Ser Ala Tyr Glu Pro Asn Gly Ala Glu Cys Tyr Ser
130 135 140

Cys Val Gly Leu Ser Arg Glu Lys Cys Gln Gly Ser Met Pro Pro Val
145 150 155 160

```
Val Asn Cys Tyr Asn Ala Ser Gly Arg Val Tyr Lys Gly Cys Phe Asp
                165                 170                 175

Gly Asn Val Thr Leu Thr Ala Ala Asn Val Thr Val Ser Leu Pro Val
            180                 185                 190

Arg Gly Cys Val Gln Asp Glu Thr Cys Thr Arg Asp Gly Val Thr Gly
        195                 200                 205

Pro Gly Phe Thr Leu Ser Gly Ser Cys Cys Gln Gly Pro Arg Cys Asn
    210                 215                 220

Ala Asp Leu Arg Asn Lys Thr Tyr Phe Ser Pro Arg Ile Pro Pro Leu
225                 230                 235                 240

Val Leu Leu Pro Pro Pro Thr Thr Ala Ala Pro Ser Thr Arg Ala Gln
                245                 250                 255

Asn Ser Ser Ser Thr Thr Ser Thr Ala Ala Pro Thr Thr Thr Thr Ser
            260                 265                 270

Ile Ile Lys Pro Thr Thr Ala Gln Ala Ser His Thr Ser Pro His Glu
        275                 280                 285

Met Asp Leu Glu Val Ile Gln Glu Glu Gly Ala Ser Leu Ser Gly Gly
    290                 295                 300

Ala Ala Gly His Gly Gly Thr Ala Gly His Gly Gly Ala Ala Gly His
305                 310                 315                 320

Gln Asp Arg Ser Asn Met Glu Lys Tyr Pro Gly Lys Gly Gly Ala Gln
                325                 330                 335

Ile Pro Ala Lys Gly Gly Ser Gly Thr Leu Gly Ser Trp Leu Ser Ala
            340                 345                 350

Val Leu Leu Thr Val Val Ala Gly Ala Met Leu
        355                 360
```

<210> 5
<211> 9
<212> PRT
<213> Artificial Sequence

<220>
<223> C4.4a binder

<400> 5

Phe Ser Asn Ala Trp Met Ser Trp Val
1               5

<210>  6
<211>  9
<212>  PRT
<213>  Artificial Sequence

<220>
<223>  C4.4a binder

<400>  6

Phe Ser Asp Tyr Gln Met Thr Trp Ile
1               5

<210>  7
<211>  9
<212>  PRT
<213>  Artificial Sequence

<220>
<223>  C4.4a binder

<400>  7

Phe Gly His Tyr Tyr Met Phe Trp Ile
1               5

<210>  8
<211>  8
<212>  PRT
<213>  Artificial Sequence

<220>
<223>  C4.4a binder

<400>  8

Phe Ser Ser Asn Tyr Met Ser Trp
1               5

<210>  9
<211>  20
<212>  PRT
<213>  Artificial Sequence

<220>
<223>  C4.4a binder

<400>  9

Val Ser Tyr Ile Ser Ser Ser Gly Ser Thr Ile Tyr Tyr Ala Asp Ser
1               5                   10                  15

Val Lys Gly Arg
            20

<210> 10
<211> 20
<212> PRT
<213> Artificial Sequence

<220>
<223> C4.4a binder

<400> 10

```
Val Ser Gly Val Ser Trp Asn Gly Ala Arg Thr His Tyr Ala Asp Ser
1               5                   10                  15


Val Lys Gly Arg
            20
```

<210> 11
<211> 20
<212> PRT
<213> Artificial Sequence

<220>
<223> C4.4a binder

<400> 11

```
Val Ser Ala Ile Ser Gly Ser Gly Tyr Ser Thr His Tyr Ala Asp Ser
1               5                   10                  15


Val Lys Gly Arg
            20
```

<210> 12
<211> 20
<212> PRT
<213> Artificial Sequence

<220>
<223> C4.4a binder

<400> 12

```
Val Ser Ala Ile Ser Ser Ser Gly Ser Ser Thr Tyr Tyr Ala Asp Ser
1               5                   10                  15


Val Lys Gly Arg
            20
```

<210> 13
<211> 10
<212> PRT
<213> Artificial Sequence

<220>
<223> C4.4a binder

<400> 13

Ala Arg Glu Gly Leu Trp Ala Phe Asp Tyr
1 5 10

<210> 14
<211> 15
<212> PRT
<213> Artificial Sequence

<220>
<223> C4.4a binder

<400> 14

Ala Lys Gly Asp Tyr Leu Val Tyr Ser Ala Tyr Tyr Phe Asp Ser
1 5 10 15

<210> 15
<211> 13
<212> PRT
<213> Artificial Sequence

<220>
<223> C4.4a binder

<400> 15

Ala Arg Leu Pro Tyr Gly Ser Gln Ser Gly Val Asp Tyr
1 5 10

<210> 16
<211> 17
<212> PRT
<213> Artificial Sequence

<220>
<223> C4.4a binder

<400> 16

Ala Arg Glu Ser Gly Gly Ser Gly Pro Asn Tyr Tyr Tyr Gly Met Asp
1 5 10 15

Val

<210> 17
<211> 14
<212> PRT
<213> Artificial Sequence

<220>
<223> C4.4a binder

<400> 17

Thr Gly Ser Ser Ser Asn Ile Gly Ala Gly Tyr Val Val His

1 5 10

<210> 18
<211> 13
<212> PRT
<213> Artificial Sequence

<220>
<223> C4.4a binder

<400> 18

Ser Gly Ser Ser Ser Asn Val Gly Ser Asn Pro Val Asn
1 5 10

<210> 19
<211> 14
<212> PRT
<213> Artificial Sequence

<220>
<223> C4.4a binder

<400> 19

Thr Gly Ser Ser Ser Asn Ile Gly Ala Gly Tyr Val Val His
1 5 10

<210> 20
<211> 14
<212> PRT
<213> Artificial Sequence

<220>
<223> C4.4a binder

<400> 20

Thr Gly Ser Ser Ser Asn Ile Gly Ala Gly Tyr Val Val His
1 5 10

<210> 21
<211> 7
<212> PRT
<213> Artificial Sequence

<220>
<223> C4.4a binder

<400> 21

Asp Asn Asn Lys Arg Pro Ser
1 5

<210> 22
<211> 7
<212> PRT
<213> Artificial Sequence

<220>
<223> C4.4a binder

<400> 22

Arg Asn Asn Gln Arg Pro Ser
1 5

<210> 23
<211> 7
<212> PRT
<213> Artificial Sequence

<220>
<223> C4.4a binder

<400> 23

Ser Asn Asn Gln Arg Pro Ser
1 5

<210> 24
<211> 7
<212> PRT
<213> Artificial Sequence

<220>
<223> C4.4a binder

<400> 24

Ser Asn Asn Gln Arg Pro Ser
1 5

<210> 25
<211> 12
<212> PRT
<213> Artificial Sequence

<220>
<223> C4.4a binder

<400> 25

Cys Ala Ala Trp Asp Asp Arg Leu Asn Gly Pro Val
1 5 10

<210> 26
<211> 12
<212> PRT
<213> Artificial Sequence

<220>
<223> C4.4a binder

<400> 26

Cys Ala Ala Trp Asp Asp Arg Leu Asn Gly Trp Val

1 5 10

<210> 27
<211> 10
<212> PRT
<213> Artificial Sequence

<220>
<223> C4.4a binder

<400> 27

Cys Gln Ser Tyr Asp Ser Ser His Val Leu
1 5 10

<210> 28
<211> 12
<212> PRT
<213> Artificial Sequence

<220>
<223> C4.4a binder

<400> 28

Cys Gln Ser Tyr Asp Arg Ser Leu Arg Gly Trp Val
1 5 10

<210> 29
<211> 113
<212> PRT
<213> Artificial Sequence

<220>
<223> C4.4a binder

<400> 29

Gln Ser Val Leu Thr Gln Pro Pro Ser Ala Ser Gly Thr Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Trp Asp Asp Arg
85 90 95

Leu Asn Gly Pro Val Phe Gly Gly Gly Thr Lys Leu Thr Val Leu Gly
100 105 110

Gln

<210> 30
<211> 112
<212> PRT
<213> Artificial Sequence

<220>
<223> C4.4a binder

<400> 30

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Val Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Arg Leu
85 90 95

Asn Gly Trp Val Phe Gly Gly Gly Thr Lys Leu Thr Val Leu Gly Gln
100 105 110

<210> 31
<211> 111
<212> PRT
<213> Artificial Sequence

<220>
<223> C4.4a binder

<400> 31

Gln Ser Val Leu Thr Gln Pro Pro Ser Ala Ser Gly Thr Pro Gly Gln
1 5 10 15

Arg Val Thr Ile Ser Cys Thr Gly Ser Ser Ser Asn Ile Gly Ala Gly

20 25 30

Tyr Val Val His Trp Tyr Gln Gln Leu Pro Gly Thr Ala Pro Lys Leu
35 40 45

Leu Ile Tyr Ser Asn Asn Gln Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Gln Ser Tyr Asp Ser Ser
85 90 95

His Val Leu Phe Gly Gly Gly Thr Lys Leu Thr Val Leu Gly Gln
100 105 110

<210> 32
<211> 113
<212> PRT
<213> Artificial Sequence

<220>
<223> C4.4a binder

<400> 32

Gln Ser Val Leu Thr Gln Pro Pro Ser Ala Ser Gly Thr Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Ser Asn Asn Gln Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Gln Ser Tyr Asp Arg Ser
85 90 95

Leu Arg Gly Trp Val Phe Gly Gly Gly Thr Lys Leu Thr Val Leu Gly
100 105 110

Gln

<210> 33
<211> 117
<212> PRT
<213> Artificial Sequence

<220>
<223> C4.4a binder

<400> 33

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Thr Ser
        115
```

<210> 34
<211> 122
<212> PRT
<213> Artificial Sequence

<220>
<223> C4.4a binder

<400> 34

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
```

35 40 45

Ser Gly Val Ser Trp Asn Gly Ala Arg Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ala Tyr Tyr Phe Asp Ser Trp
100 105 110

Gly Gln Gly Thr Leu Val Thr Val Thr Ser
115 120

<210> 35
<211> 120
<212> PRT
<213> Artificial Sequence

<220>
<223> C4.4a binder

<400> 35

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Gly His Tyr
20 25 30

Tyr Met Phe Trp Ile Arg Gln Ala Pro Gly Lys Gly Leu Glu Trp Val
35 40 45

Ser Ala Ile Ser Gly Ser Gly Tyr Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Leu Pro Tyr Gly Ser Gln Ser Gly Val Asp Tyr Trp Gly Gln
100 105 110

Gly Thr Leu Val Thr Val Thr Ser
115 120

<210> 36
<211> 124
<212> PRT
<213> Artificial Sequence

<220>
<223> C4.4a binder

<400> 36

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Asn
            20                  25                  30

Tyr Met Ser Trp Val Arg Gln Ala Pro Gly Lys Gly Leu Glu Trp Val
        35                  40                  45

Ser Ala Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Ser Gly Gly Ser Gly Pro Asn Tyr Tyr Tyr Gly Met Asp
            100                 105                 110

Val Trp Gly Gln Gly Thr Leu Val Thr Val Thr Ser
        115                 120

<210> 37
<211> 339
<212> DNA
<213> Artificial Sequence

<220>
<223> C4.4a binder

<400> 37
cagtctgtgc tgactcagcc accctcagcg tctgggaccc ctgggcagag ggtcaccatc 60

tcctgcactg ggagcagctc caacattggg gcgggttatg ttgtacattg gtatcagcag 120

ctcccaggaa cggcccccaa actcctcatc tatgacaata ataagcgacc ctcaggggtc 180

cctgaccgat tctctggctc caagtctggc acctcagcct ccctggccat cagtgggctc 240

cggtccgagg atgaggctga ttattactgt gcagcatggg atgacaggct gaatggtccg 300

gtgttcggcg gaggaaccaa gttaaccgtc ctaggtcag 339

<210> 38
<211> 336
<212> DNA
<213> Artificial Sequence

<220>
<223> C4.4a binder

<400> 38
cagtctgtgc tgactcagcc accctcagcg tctgggaccc ccgggcagag ggtcaccatc 60

tcttgttctg gaagcagctc caacgtcggg agtaatcctg taaactggta tcagcagctc 120

ccaggaacgg cccccaaact cctcatctat aggaataatc agcggccctc aggggtccct 180

gaccgattct ctggctccaa gtctggcacc tcagcctccc tggccatcag tgggctccgg 240

tccgaggatg aggctgatta ttactgtgca gcatgggatg acaggctgaa tggttgggtg 300

ttcggcggag gaaccaagct gacggtccta ggtcag 336

<210> 39
<211> 333
<212> DNA
<213> Artificial Sequence

<220>
<223> C4.4a binder

<400> 39
cagtctgtgc tgactcagcc accctcagcg tctgggaccc ccgggcagag ggtcaccatc 60

tcctgcactg ggagcagctc caacattggg gcgggttatg ttgtacattg gtatcagcag 120

ctcccaggaa cggcccccaa actcctcatc tatagtaata atcagcggcc ctcaggagtc 180

cctgaccgat tctctggctc caagtctggc acctcagcct ccctggccat cagtgggctc 240

cggtccgagg atgaggctga ttattactgc cagtcctatg acagcagcca tgttttattc 300

ggcggaggaa ccaagctgac ggtcctaggt cag 333

<210> 40
<211> 339
<212> DNA
<213> Artificial Sequence

<220>
<223> C4.4a binder

<400> 40
cagtctgtgc tgactcagcc accctcagcg tctgggaccc ccgggcagag ggtcaccatc 60

tcctgcactg ggagcagctc caacattggg gcgggttatg ttgtacattg gtatcagcag 120

ctcccaggaa cggcccccaa actcctcatc tatagtaata atcagcggcc ctcaggggtc 180

cctgaccgat tctctggctc caagtctggc acctcagcct ccctggccat cagtgggctc 240

cggtccgagg atgaggctga ttattactgc cagtcctatg acagaagcct gcgtggttgg 300

gtgttcggcg gaggaaccaa gctgacggtc ctaggtcag 339

<210> 41
<211> 351
<212> DNA
<213> Artificial Sequence

<220>
<223> C4.4a binder

<400> 41
gaggtgcagc tgttggagtc tgggggaggc ttggtacagc ctggggggtc cctgagactc 60

tcctgtgcag cctctggatt caccttcagt aacgcctgga tgagctgggt ccgccaggct 120

ccagggaagg ggctggagtg ggtttcatac attagtagta gtggtagtac catatactac 180

gcagactctg tgaagggccg attcaccatc tccagagaca attccaagaa cacgctgtat 240

ctgcaaatga acagcctgag agccgaggac actgccgtgt attactgtgc gagagaaggg 300

ttatgggcct ttgactactg gggccagggt accctggtca ccgtgactag t 351

<210> 42
<211> 366
<212> DNA
<213> Artificial Sequence

<220>
<223> C4.4a binder

<400> 42
gaggtgcagc tgttggagtc tgggggaggc ttggtacagc ctggggggtc cctgagactc 60

tcctgtgcag cctctggatt caccttcagt gactatcaga tgacctggat ccgccagact 120

ccagggaagg ggctggagtg ggtatcgggt gttagttgga atggcgctag gacgcactat 180

gcagactctg tgaagggccg attcaccatc tccagagaca attccaagaa cacgctgtat 240

ctacaaatga acagcctgag agccgaggac actgccgtgt attactgtgc gaagggcgac 300

tacctggttt actccgcata ctactttgac tcctggggcc agggtaccct ggtcaccgtg 360

actagt 366

<210> 43
<211> 360
<212> DNA
<213> Artificial Sequence

<220>
<223> C4.4a binder

<400> 43
gaggtgcagc tgttggagtc tgggggaggc ttggtacagc ctggggggtc cctgagactc 60

tcctgtgcag cctctggatt caccttcggt cactactata tgttctggat ccgtcaggct 120

ccagggaagg ggctggagtg ggtctcagct attagtggta gtggttatag cacacactac 180

gcagactccg tgaagggccg gttcaccatc tccagagaca attccaagaa cacgctgtat 240

ctgcaaatga acagcctgag agccgaggac actgccgtgt attactgtgc gagactgcca 300

tatggttcgc agagtggcgt tgactactgg ggccagggta ccctggtcac cgtgactagt 360

<210> 44
<211> 372
<212> DNA
<213> Artificial Sequence

<220>
<223> C4.4a binder

<400> 44
gaggtgcagc tgttggagtc tgggggaggc ttggtacagc ctggggggtc cctgagactc 60

tcctgtgcag cctctggatt caccttcagt agcaactaca tgagctgggt ccgccaggct 120

ccagggaagg ggctggagtg ggtctcagct attagtagta gtggtagtag cacatactac 180

gcagactccg tgaagggccg gttcaccatc tccagagaca attccaagaa cacgctgtat 240

ctgcaaatga acagcctgag agccgaggac actgccgtgt attactgtgc gagagaatct 300

ggtgggagcg gaccgaacta ctactacggt atggacgtct ggggccaagg taccctggtc 360

accgtgacta gt 372

<210> 45
<211> 9
<212> PRT
<213> Artificial Sequence

<220>
<223> C4.4a binder

<400> 45

Phe Ser Asn Ala Trp Met Ser Trp Val
1 5

<210> 46
<211> 20
<212> PRT
<213> Artificial Sequence

<220>
<223> C4.4a binder

<400> 46

Val Ser Tyr Ile Ser Ser Ser Gly Ser Thr Ile Tyr Tyr Ala Asp Ser
1 5 10 15

Val Lys Gly Arg
20

<210> 47
<211> 10
<212> PRT
<213> Artificial Sequence

<220>
<223> C4.4a binder

<400> 47

Ala Arg Glu Gly Leu Trp Ala Phe Asp Tyr
1 5 10

<210> 48
<211> 14
<212> PRT
<213> Artificial Sequence

<220>
<223> C4.4a binder

<400> 48

Thr Gly Ser Ser Ser Asn Ile Gly Ala Gly Tyr Val Val His
1 5 10

<210> 49
<211> 7
<212> PRT
<213> Artificial Sequence

<220>
<223> C4.4a binder

<400> 49

Asp Asn Asn Lys Arg Pro Ser
1 5

<210> 50
<211> 12
<212> PRT
<213> Artificial Sequence

<220>
<223> C4.4a binder

<400> 50

Cys Ala Ala Trp Asp Asp Arg Leu Asn Gly Pro Val
1 5 10

<210> 51
<211> 117
<212> PRT

<213> Artificial Sequence

<220>
<223> C4.4a binder

<400> 51

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Tyr Trp Gly Gln Gly Thr Leu
100 105 110

Val Thr Val Ser Ser
115

<210> 52
<211> 113
<212> PRT
<213> Artificial Sequence

<220>
<223> C4.4a binder

<400> 52

Glu Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110

Gln

<210> 53
<211> 351
<212> DNA
<213> Artificial Sequence

<220>
<223> C4.4a binder

<400> 53
gaggtgcagc tgctggaaag cggcggagga ctggtgcagc ctggaggcag cctgagactg 60

tcttgcgccg ccagcggctt caccttcagc aacgcctgga tgagctgggt ccgacaggct 120

cctggcaagg gcctggaatg ggtgtcctac atcagcagca gcggcagcac catctactac 180

gccgacagcg tgaagggccg gttcaccatc agccgggaca acagcaagaa caccctgtac 240

ctgcagatga acagcctgcg ggccgaggac accgccgtgt actactgcgc cagagaaggc 300

ctgtgggcct tcgactactg gggccagggc accctggtca ccgtgtctag c 351

<210> 54
<211> 339
<212> DNA
<213> Artificial Sequence

<220>
<223> C4.4a binder

<400> 54
gagagcgtgc tgacccagcc tcctagcgtg tccggcgctc ctggccagag agtgaccatc 60

agctgcaccg gcagcagcag caacatcgga gccggctacg tggtgcactg gtatcagcag 120

ctgcccggca ccgcccccaa gctgctgatc tacgacaaca acaagcggcc tagcggcgtg 180

cccgacagat tcagcggcag caagagcggc accagcgcca gcctggccat cagcggcctg 240

agaagcgagg acgaggccga ctactactgc gccgcctggg acgacagact gaacggccct 300

gtgttcggcg gaggcaccaa gctgaccgtg ctgggacag 339

<210> 55

<211> 9
<212> PRT
<213> Artificial Sequence

<220>
<223> C4.4a binder

<400> 55

Phe Ser Asn Ala Trp Met Ser Trp Val
1 5

<210> 56
<211> 20
<212> PRT
<213> Artificial Sequence

<220>
<223> C4.4a binder

<400> 56

Val Ser Tyr Ile Ser Ser Ser Gly Ser Thr Ile Tyr Tyr Ala Asp Ser
1 5 10 15

Val Lys Gly Arg
20

<210> 57
<211> 10
<212> PRT
<213> Artificial Sequence

<220>
<223> C4.4a binder

<400> 57

Ala Arg Glu Gly Leu Trp Ala Phe Asp Tyr
1 5 10

<210> 58
<211> 14
<212> PRT
<213> Artificial Sequence

<220>
<223> C4.4a binder

<400> 58

Thr Gly Ser Ser Ser Asn Ile Gly Ala Gly Tyr Val Val His
1 5 10

<210> 59
<211> 7
<212> PRT
<213> Artificial Sequence

<220>
<223> C4.4a binder

<400> 59

Asp Asn Asn Lys Arg Pro Ser
1 5

<210> 60
<211> 12
<212> PRT
<213> Artificial Sequence

<220>
<223> C4.4a binder

<400> 60

Cys Ala Ala Tyr Asp Asp Ser Leu Ser Gly Pro Val
1 5 10

<210> 61
<211> 117
<212> PRT
<213> Artificial Sequence

<220>
<223> C4.4a binder

<400> 61

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Tyr Trp Gly Gln Gly Thr Leu
100 105 110

Val Thr Val Ser Ser

115

<210> 62
<211> 113
<212> PRT
<213> Artificial Sequence

<220>
<223> C4.4a binder

<400> 62

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Ser
85 90 95

Leu Ser Gly Pro Val Phe Gly Gly Gly Thr Lys Leu Thr Val Leu Gly
100 105 110

Gln

<210> 63
<211> 351
<212> DNA
<213> Artificial Sequence

<220>
<223> C4.4a binder

<400> 63
gaggtgcagc tgctggaatc cggcggaggc ctggtgcagc ctggcggatc tctgagactg 60

tcctgcgccg ccagcggctt taccttctcc aacgcctgga tgtcctgggt ccgacaggcc 120

cctggcaagg gactggaatg ggtgtcctac atctcctcct ccggctccac catctactac 180

gccgactccg tgaagggccg gttcaccatc tcccgggaca actccaagaa caccctgtac 240

ctgcagatga actccctgcg ggccgaggac accgccgtgt actactgcgc ccgagagggc 300

ctgtgggcct tcgattattg gggccagggc accctggtca ccgtcagctc a 351

<210> 64
<211> 339
<212> DNA
<213> Artificial Sequence

<220>
<223> C4.4a binder

<400> 64
cagtccgtgc tgacccagcc cccttctgtg tctggcgccc ctggccagag agtgaccatc 60

tcttgcaccg gctcctccag caacatcggc gctggctacg tggtgcactg gtatcagcag 120

ctgcccggca ccgcccccaa gctgctgatc tacgacaaca acaagcggcc ctccggcgtg 180

cccgacagat tctccggctc caagtccggc acctccgcct ccctggccat ctccggcctg 240

agatctgagg acgaggccga ctactactgc gccgcctacg acgactccct gtccggccct 300

gtgttcggcg gaggcacaaa gttaaccgtg ctgggccag 339

<210> 65
<211> 9
<212> PRT
<213> Artificial Sequence

<220>
<223> C4.4a binder

<400> 65

Phe Ser Asn Ala Trp Met Ser Trp Val
1               5

<210> 66
<211> 20
<212> PRT
<213> Artificial Sequence

<220>
<223> C4.4a binder

<400> 66

Val Ser Tyr Ile Ser Ser Ser Gly Ser Thr Ile Tyr Tyr Ala Asp Ser
1               5                   10                  15

Val Lys Gly Arg
            20

<210> 67
<211> 10
<212> PRT
<213> Artificial Sequence

<220>
<223> C4.4a binder

<400> 67

Ala Arg Glu Gly Leu Trp Ala Phe Asp Tyr
1 5 10

<210> 68
<211> 14
<212> PRT
<213> Artificial Sequence

<220>
<223> C4.4a binder

<400> 68

Thr Gly Ser Ser Ser Asn Ile Gly Ala Gly Tyr Val Val His
1 5 10

<210> 69
<211> 7
<212> PRT
<213> Artificial Sequence

<220>
<223> C4.4a binder

<400> 69

Asp Asn Asn Lys Arg Pro Ser
1 5

<210> 70
<211> 12
<212> PRT
<213> Artificial Sequence

<220>
<223> C4.4a binder

<400> 70

Cys Ala Ala Phe Asp Asp Ser Leu Asn Gly Pro Val
1 5 10

<210> 71
<211> 117
<212> PRT
<213> Artificial Sequence

<220>
<223> C4.4a binder

<400> 71

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Lys Trp Gly Gln Gly Thr Leu
100 105 110

Val Thr Val Ser Ser
115

<210> 72
<211> 113
<212> PRT
<213> Artificial Sequence

<220>
<223> C4.4a binder

<400> 72

Gln Ser Val Leu Thr Gln Pro Pro Ser Ala Ser Gly Thr Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Arg
85 90 95

Leu Asn Gly Pro Val Phe Gly Gly Gly Thr Lys Leu Thr Val Leu Gly
100 105 110

Gln

<210> 73
<211> 351
<212> DNA
<213> Artificial Sequence

<220>
<223> C4.4a binder

<400> 73
gaggtgcagc tgctggaatc cggcggaggc ctggtgcagc ctggcggatc tctgagactg 60

tcctgcgccg cctccggctt taccttctcc aacgcctgga tgtcctgggt ccgacaggct 120

cctggcaagg gcctggaatg ggtgtcctac atctcctcct ccggctccac catctactac 180

gccgactccg tgaagggccg gttcaccatc tcccgggaca actccaagaa caccctgtac 240

ctgcagatga actccctgcg ggccgaggac accgccgtgt actactgcgc ccgagagggc 300

ctgtgggcct tcgataagtg gggccagggc accctggtca ccgtcagctc a 351

<210> 74
<211> 339
<212> DNA
<213> Artificial Sequence

<220>
<223> C4.4a binder

<400> 74
cagtccgtgc tgacccagcc tccttccgcc tctggcaccc ctggccagag agtgaccatc 60

tcctgcaccg gctcctccag caacatcggc gctggctacg tggtgcactg gtatcagcag 120

ctgcccggca ccgcccccaa gctgctgatc tacgacaaca acaagcggcc ctccggcgtg 180

cccgacagat tctccggctc caagtccggc acctccgcct ccctggccat ctccggcctg 240

agatctgagg acgaggccga ctactactgc gccgccttcg acgaccggct gaacggccct 300

gtgttcggcg gaggcacaaa gttaaccgtg ctgggccag 339

<210> 75
<211> 9
<212> PRT
<213> Artificial Sequence

<220>
<223> C4.4a binder

<400> 75

Phe Ser Ser Ala Trp Met Ser Trp Val
1               5

<210>  76
<211>  20
<212>  PRT
<213>  Artificial Sequence

<220>
<223>  C4.4a binder

<400>  76

Val Ser Tyr Ile Ser Ser Ser Gly Ser Thr Ile Tyr Tyr Ala Asp Ser
1               5                   10                  15

Val Lys Gly Arg
            20

<210>  77
<211>  10
<212>  PRT
<213>  Artificial Sequence

<220>
<223>  C4.4a binder

<400>  77

Ala Arg Glu Gly Leu Trp Ala Phe Asp Tyr
1               5                   10

<210>  78
<211>  14
<212>  PRT
<213>  Artificial Sequence

<220>
<223>  C4.4a binder

<400>  78

Thr Gly Ser Ser Ser Asn Ile Gly Ala Gly Tyr Val Val His
1               5                   10

<210>  79
<211>  7
<212>  PRT
<213>  Artificial Sequence

<220>
<223>  C4.4a binder

<400>  79

Asp Asn Asn Lys Arg Pro Ser
1               5

<210> 80
<211> 12
<212> PRT
<213> Artificial Sequence

<220>
<223> C4.4a binder

<400> 80

Cys Ala Ala Tyr Asp Asp Ser Leu Ser Gly Pro Val
1 5 10

<210> 81
<211> 117
<212> PRT
<213> Artificial Sequence

<220>
<223> C4.4a binder

<400> 81

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Tyr Trp Gly Gln Gly Thr Leu
100 105 110

Val Thr Val Ser Ser
115

<210> 82
<211> 113
<212> PRT
<213> Artificial Sequence

<220>

<223> C4.4a binder

<400> 82

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Ser
85 90 95

Leu Ser Gly Pro Val Phe Gly Gly Gly Thr Lys Leu Thr Val Leu Gly
100 105 110

Gln

<210> 83
<211> 351
<212> DNA
<213> Artificial Sequence

<220>
<223> C4.4a binder

<400> 83
gaggtgcagc tgctggaatc cggcggaggc ctggtgcagc ctggcggatc tctgagactg 60

tcctgcgccg ccagcggctt taccttctcc agcgcctgga tgtcctgggt ccgacaggcc 120

cctggcaagg gactggaatg ggtgtcctac atctcctcct ccggctccac catctactac 180

gccgactccg tgaagggccg gttcaccatc tcccgggaca actccaagaa caccctgtac 240

ctgcagatga actccctgcg ggccgaggac accgccgtgt actactgcgc ccgagagggc 300

ctgtgggcct tcgattattg gggccagggc accctggtca ccgtcagctc a 351

<210> 84
<211> 339
<212> DNA
<213> Artificial Sequence

<220>
<223> C4.4a binder

<400> 84
cagtccgtgc tgacccagcc cccttctgtg tctggcgccc ctggccagag agtgaccatc 60

tcttgcaccg gctcctccag caacatcggc gctggctacg tggtgcactg gtatcagcag 120

ctgcccggca ccgcccccaa gctgctgatc tacgacaaca acaagcggcc ctccggcgtg 180

cccgacagat tctccggctc caagtccggc acctccgcct ccctggccat ctccggcctg 240

agatctgagg acgaggccga ctactactgc gccgcctacg acgactccct gtccggccct 300

gtgttcggcg gaggcacaaa gttaaccgtg ctgggccag 339

<210> 85
<211> 9
<212> PRT
<213> Artificial Sequence

<220>
<223> C4.4a binder

<400> 85

Phe Ser Ser Ala Trp Met Ser Trp Val
1 5

<210> 86
<211> 20
<212> PRT
<213> Artificial Sequence

<220>
<223> C4.4a binder

<400> 86

Val Ser Tyr Ile Ser Ser Ser Gly Ser Ser Thr Tyr Tyr Ala Asp Ser
1 5 10 15

Val Lys Gly Arg
20

<210> 87
<211> 10
<212> PRT
<213> Artificial Sequence

<220>
<223> C4.4a binder

<400> 87

Ala Arg Glu Gly Leu Trp Ala Phe Asp Lys
1 5 10

<210> 88
<211> 14
<212> PRT
<213> Artificial Sequence

<220>
<223> C4.4a binder

<400> 88

```
Ser Gly Ser Ser Ser Asn Ile Gly Ala Gly Tyr Val Val His
1               5                   10
```

<210> 89
<211> 7
<212> PRT
<213> Artificial Sequence

<220>
<223> C4.4a binder

<400> 89

```
Asp Asn Asn Gln Arg Pro Ser
1               5
```

<210> 90
<211> 12
<212> PRT
<213> Artificial Sequence

<220>
<223> C4.4a binder

<400> 90

```
Cys Ala Ala Phe Asp Asp Arg Leu Ser Gly Pro Val
1               5                   10
```

<210> 91
<211> 117
<212> PRT
<213> Artificial Sequence

<220>
<223> C4.4a binder

<400> 91

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45
```

```
Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser
        115


<210>  92
<211>  113
<212>  PRT
<213>  Artificial Sequence

<220>
<223>  C4.4a binder

<400>  92

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Arg
                85                  90                  95

Leu Ser Gly Pro Val Phe Gly Gly Gly Thr Lys Leu Thr Val Leu Gly
            100                 105                 110

Gln
```

<210> 93
<211> 351
<212> DNA
<213> Artificial Sequence

<220>
<223> C4.4a binder

<400> 93
gaggtgcagc tgctggaaag cggcggaggc ctggtgcagc ctggcggaag cctgagactg 60

agctgtgccg ccagcggctt caccttcagc agcgcctgga tgagctgggt ccgacaggcc 120

cctggcaagg gcctggaatg ggtgtcctac atcagcagca gcggcagcag cacctactac 180

gccgacagcg tgaagggccg gttcaccatc agccgggaca acagcaagaa caccctgtac 240

ctgcagatga acagcctgcg ggccgaggac accgccgtgt actactgcgc cagagaaggc 300

ctgtgggcct tcgataagtg gggccagggc accctggtca ccgtcagctc a 351

<210> 94
<211> 339
<212> DNA
<213> Artificial Sequence

<220>
<223> C4.4a binder

<400> 94
cagagcgtgc tgacccagcc tcctagcgcc tctggcaccc ctggccagag agtgaccatc 60

agctgcagcg gcagcagcag caacatcgga gccggctacg tggtgcactg gtatcagcag 120

ctgcccggca ccgcccccaa gctgctgatc tacgacaaca accagcggcc cagcggcgtg 180

cccgacagat tttccggcag caagagcggc accagcgcca gcctggccat cagcggcctg 240

agaagcgagg acgaggccga ctactactgc gccgccttcg acgacagact gagcggccct 300

gtgttcggcg gaggcacaaa gttaaccgtg ctgggccag 339

<210> 95
<211> 9
<212> PRT
<213> Artificial Sequence

<220>
<223> C4.4a binder

<400> 95

Phe Ser Asp Tyr Gln Met Thr Trp Ile
1 5

<210> 96
<211> 20
<212> PRT
<213> Artificial Sequence

<220>
<223> C4.4a binder

<400> 96

Val Ser Gly Val Ser Trp Asn Gly Ala Arg Thr His Tyr Ala Asp Ser
1 5 10 15

Val Lys Gly Arg
20

<210> 97
<211> 15
<212> PRT
<213> Artificial Sequence

<220>
<223> C4.4a binder

<400> 97

Ala Lys Gly Asp Tyr Leu Val Tyr Ser Ala Tyr Tyr Phe Asp Ser
1 5 10 15

<210> 98
<211> 13
<212> PRT
<213> Artificial Sequence

<220>
<223> C4.4a binder

<400> 98

Ser Gly Ser Ser Ser Asn Val Gly Ser Asn Pro Val Asn
1 5 10

<210> 99
<211> 7
<212> PRT
<213> Artificial Sequence

<220>
<223> C4.4a binder

<400> 99

Arg Asn Asn Gln Arg Pro Ser
1 5

<210> 100
<211> 12
<212> PRT
<213> Artificial Sequence

<220>
<223> C4.4a binder

<400> 100

Cys Ala Ala Trp Asp Asp Arg Leu Asn Gly Trp Val
1 5 10

<210> 101
<211> 122
<212> PRT
<213> Artificial Sequence

<220>
<223> C4.4a binder

<400> 101

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ala Tyr Tyr Phe Asp Ser Trp
100 105 110

Gly Gln Gly Thr Leu Val Thr Val Ser Ser
115 120

<210> 102
<211> 112
<212> PRT
<213> Artificial Sequence

<220>
<223> C4.4a binder

<400> 102

Glu Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn

20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Arg Leu
85 90 95

Asn Gly Trp Gly Phe Gly Gly Gly Thr Lys Leu Thr Val Leu Gly Gln
100 105 110

<210> 103
<211> 366
<212> DNA
<213> Artificial Sequence

<220>
<223> C4.4a binder

<400> 103
gaggtgcagc tgctggaaag cggcggagga ctggtgcagc ctggaggcag cctgagactg 60

tcttgcgccg ccagcggctt caccttcagc gactaccaga tgacctggat ccgacagacc 120

cctggcaagg gcctggaatg ggtgtccggc atcagctgga acggaggcag cacccactac 180

gccgacagcg tgaagggccg gttcaccatc agccgggaca acagcaagaa caccctgtac 240

ctgcagatga acagcctgcg ggccgaggac accgccgtgt actactgcgc caagggcgac 300

tacctggtgt acagcgccta ctacttcgac agctggggcc agggcaccct ggtcaccgtg 360

tctagc 366

<210> 104
<211> 336
<212> DNA
<213> Artificial Sequence

<220>
<223> C4.4a binder

<400> 104
gagagcgtgc tgacccagcc tcctagcgcc tctggcaccc ctggccagag agtgaccatc 60

agctgctctg gcagcagcag caacatcgga agcaaccccg tgaactggta tcagcagctg 120

cccggcaccg cccccaagct gctgatctac cggaacaacc agcggcctag cggcgtgccc 180

gacagattca gcggcagcaa gagcggcacc agcgccagcc tggccatcag cggcctgaga 240

agcgaggacg aggccgacta ctactgcgcc gcctgggacg acagactgaa cggctggggc 300

ttcggcggag gcaccaagct gaccgtgctg ggacag 336

<210> 105
<211> 9
<212> PRT
<213> Artificial Sequence

<220>
<223> C4.4a binder

<400> 105

Phe Ser Asp Tyr Gln Met Thr Trp Ile
1               5

<210> 106
<211> 20
<212> PRT
<213> Artificial Sequence

<220>
<223> C4.4a binder

<400> 106

Val Ser Gly Ile Ser Trp Asn Gly Gly Ser Thr His Tyr Ala Asp Ser
1               5                   10                  15

Val Lys Gly Arg
            20

<210> 107
<211> 15
<212> PRT
<213> Artificial Sequence

<220>
<223> C4.4a binder

<400> 107

Ala Lys Gly Asp Tyr Leu Val Tyr Ser Ser Tyr Tyr Phe Lys Ser
1               5                   10                  15

<210> 108
<211> 13
<212> PRT
<213> Artificial Sequence

<220>
<223> C4.4a binder

<400> 108

Ser Gly Ser Ser Ser Asn Ile Gly Ser Asn Pro Val Asn

1 5 10

<210> 109
<211> 7
<212> PRT
<213> Artificial Sequence

<220>
<223> C4.4a binder

<400> 109

Arg Asn Asn Gln Arg Pro Ser
1 5

<210> 110
<211> 12
<212> PRT
<213> Artificial Sequence

<220>
<223> C4.4a binder

<400> 110

Cys Ala Ala Trp Asp Asp Arg Leu Ser Gly Trp Ala
1 5 10

<210> 111
<211> 122
<212> PRT
<213> Artificial Sequence

<220>
<223> C4.4a binder

<400> 111

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ser Tyr Tyr Phe Lys Ser Trp
100 105 110

Gly Gln Gly Thr Leu Val Thr Val Ser Ser
115 120

<210> 112
<211> 112
<212> PRT
<213> Artificial Sequence

<220>
<223> C4.4a binder

<400> 112

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Arg Leu
85 90 95

Ser Gly Trp Ala Phe Gly Gly Gly Thr Lys Leu Thr Val Leu Gly Gln
100 105 110

<210> 113
<211> 366
<212> DNA
<213> Artificial Sequence

<220>
<223> C4.4a binder

<400> 113
gaggtgcagc tgctggaatc cggcggaggc ctggtgcagc ctggcggatc tctgagactg 60

tcctgcgccg cctccggctt caccttctcc gactaccaga tgacctggat cagacagacc 120

cccggcaagg gcctggaatg ggtgtccggc atctcctgga acggcggctc cacccactac 180

gccgactctg tgaagggccg gttcaccatc tcccgggaca actccaagaa caccctgtac 240

ctgcagatga actccctgcg ggccgaggac accgccgtgt actactgcgc caagggcgac 300

tacctggtgt actcctccta ctacttcaag tcctggggcc agggcaccct ggtcaccgtc 360

agctca 366

<210> 114
<211> 336
<212> DNA
<213> Artificial Sequence

<220>
<223> C4.4a binder

<400> 114
cagtccgtgc tgacccagcc tccttccgcc tctggcaccc ctggccagag agtgaccatc 60

tcctgctccg gctcctcctc caacatcggc tccaaccccg tgaactggta tcagcagctg 120

cccggcaccg cccccaagct gctgatctac cggaacaacc agcggccctc cggcgtgccc 180

gacagattct ccggctccaa gtccggcacc tccgcctccc tggccatctc cggcctgaga 240

tctgaggacg aggccgacta ctactgcgcc gcctgggacg accggctgtc tggctgggct 300

tttggcggcg gaacaaagtt aaccgtgctg ggccag 336

<210> 115
<211> 9
<212> PRT
<213> Artificial Sequence

<220>
<223> C4.4a binder

<400> 115

Phe Ser Asp Tyr Gln Met Thr Trp Ile
1 5

<210> 116
<211> 20
<212> PRT
<213> Artificial Sequence

<220>
<223> C4.4a binder

<400> 116

Val Ser Gly Ile Ser Trp Asn Gly Gly Ser Thr His Tyr Ala Asp Ser
1 5 10 15

Val Lys Gly Arg
20

<210> 117
<211> 15
<212> PRT
<213> Artificial Sequence

<220>
<223> C4.4a binder

<400> 117

Ala Lys Gly Asp Tyr Leu Val Tyr Lys Ser Tyr Tyr Phe Lys Ser
1 5 10 15

<210> 118
<211> 13
<212> PRT
<213> Artificial Sequence

<220>
<223> C4.4a binder

<400> 118

Ser Gly Ser Ser Ser Asn Ile Gly Ser Asn Pro Val Asn
1 5 10

<210> 119
<211> 7
<212> PRT
<213> Artificial Sequence

<220>
<223> C4.4a binder

<400> 119

Arg Asn Asn Gln Arg Pro Ser
1 5

<210> 120
<211> 12
<212> PRT
<213> Artificial Sequence

<220>
<223> C4.4a binder

<400> 120

Cys Ala Ala Trp Asp Asp Ser Leu Ser Gly Trp Ala
1 5 10

<210> 121
<211> 122
<212> PRT
<213> Artificial Sequence

<220>
<223> C4.4a binder

<400> 121

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Lys Ser Tyr Tyr Phe Lys Ser Trp
100 105 110

Gly Gln Gly Thr Leu Val Thr Val Ser Ser
115 120

<210> 122
<211> 112
<212> PRT
<213> Artificial Sequence

<220>
<223> C4.4a binder

<400> 122

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Ser Leu
85 90 95

Ser Gly Trp Ala Phe Gly Gly Gly Thr Lys Leu Thr Val Leu Gly Gln
100 105 110

<210> 123
<211> 366
<212> DNA
<213> Artificial Sequence

<220>
<223> C4.4a binder

<400> 123
gaggtgcagc tgctggaatc cggcggaggc ctggtgcagc ctggcggatc tctgagactg 60

tcctgcgccg cctccggctt caccttctcc gactaccaga tgacctggat cagacagacc 120

cccggcaagg gcctggaatg ggtgtccggc atctcctgga acggcggctc cacccactac 180

gccgactctg tgaagggccg gttcaccatc tcccgggaca actccaagaa caccctgtac 240

ctgcagatga actccctgcg ggccgaggac accgccgtgt actactgcgc caagggcgac 300

tacctggtgt acaagtccta ctacttcaag tcctggggcc agggcaccct ggtcaccgtc 360

agctca 366

<210> 124
<211> 336
<212> DNA
<213> Artificial Sequence

<220>
<223> C4.4a binder

<400> 124
cagtccgtgc tgacccagcc tccttccgcc tctggcaccc ctggccagag agtgaccatc 60

tcctgctccg gctcctcctc caacatcggc tccaaccccg tgaactggta tcagcagctg 120

cccggcaccg cccccaagct gctgatctac cggaacaacc agcggccctc cggcgtgccc 180

gacagattct ccggctccaa gtccggcacc tccgcctccc tggccatctc cggcctgaga 240

tctgaggacg aggccgacta ctactgcgcc gcctgggacg actccctgtc tggctgggct 300

tttggcggcg gaacaaagtt aaccgtgctg ggccag 336

<210> 125
<211> 9
<212> PRT
<213> Artificial Sequence

<220>
<223> C4.4a binder

<400> 125

Phe Ser Asp Tyr Gln Met Thr Trp Ile
1               5

<210> 126
<211> 20
<212> PRT
<213> Artificial Sequence

<220>
<223> C4.4a binder

<400> 126

Val Ser Gly Ile Ser Trp Asn Gly Gly Ser Thr His Tyr Ala Asp Ser
1               5                   10                  15

Val Lys Gly Arg
            20

<210> 127
<211> 15
<212> PRT
<213> Artificial Sequence

<220>
<223> C4.4a binder

<400> 127

Ala Lys Gly Asp Tyr Leu Val Tyr Ser Ser Tyr Tyr Phe Lys Ser
1               5                   10                  15

<210> 128
<211> 13
<212> PRT
<213> Artificial Sequence

<220>
<223> C4.4a binder

<400> 128

Ser Gly Ser Ser Ser Asn Ile Gly Ser Asn Pro Val Asn
1               5                   10

<210> 129
<211> 7
<212> PRT
<213> Artificial Sequence

<220>
<223> C4.4a binder

<400> 129

```
Arg Asn Asn Gln Arg Pro Ser
1               5


<210>  130
<211>  12
<212>  PRT
<213>  Artificial Sequence

<220>
<223>  C4.4a binder

<400>  130

Cys Ala Ala Trp Asp Asp Arg Leu Ser Gly Trp Gly
1               5                   10


<210>  131
<211>  122
<212>  PRT
<213>  Artificial Sequence

<220>
<223>  C4.4a binder

<400>  131

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Gln Met Thr Trp Ile Arg Gln Thr Pro Gly Lys Gly Leu Glu Trp Val
        35                  40                  45


Ser Gly Ile Ser Trp Asn Gly Gly Ser Thr His Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Gly Asp Tyr Leu Val Tyr Ser Ser Tyr Tyr Phe Lys Ser Trp
            100                 105                 110


Gly Gln Gly Thr Leu Val Thr Val Ser Ser
        115                 120


<210>  132
<211>  112
<212>  PRT
```

<213>  Artificial Sequence

<220>
<223>  C4.4a binder

<400>  132

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110
```

<210>  133
<211>  366
<212>  DNA
<213>  Artificial Sequence

<220>
<223>  C4.4a binder

<400>  133

```
gaggtgcagc tgctggaatc cggcggaggc ctggtgcagc ctggcggatc tctgagactg      60

tcctgcgccg cctccggctt caccttctcc gactaccaga tgacctggat cagacagacc     120

cccggcaagg gcctggaatg ggtgtccggc atctcctgga acggcggctc cacccactac     180

gccgactctg tgaagggccg gttcaccatc tcccgggaca actccaagaa caccctgtac     240

ctgcagatga actccctgcg ggccgaggac accgccgtgt actactgcgc caagggcgac     300

tacctggtgt actcctccta ctacttcaag tcctggggcc agggcaccct ggtcaccgtc     360

agctca                                                                366
```

<210>  134
<211>  336
<212>  DNA
<213>  Artificial Sequence

<220>
<223> C4.4a binder

<400> 134
cagtccgtgc tgacccagcc tccttccgcc tctggcaccc ctggccagag agtgaccatc 60

tcctgctccg gctcctcctc caacatcggc tccaaccccg tgaactggta tcagcagctg 120

cccggcaccg cccccaagct gctgatctac cggaacaacc agcggccctc cggcgtgccc 180

gacagattct ccggctccaa gtccggcacc tccgcctccc tggccatctc cggcctgaga 240

tctgaggacg aggccgacta ctactgcgcc gcctgggacg accggctgtc tggctgggga 300

tttggcggcg gaacaaagtt aaccgtgctg ggccag 336

<210> 135
<211> 9
<212> PRT
<213> Artificial Sequence

<220>
<223> C4.4a binder

<400> 135

Phe Ser Ser Tyr Gln Met Thr Trp Ile
1 5

<210> 136
<211> 20
<212> PRT
<213> Artificial Sequence

<220>
<223> C4.4a binder

<400> 136

Val Ser Gly Ile Ser Trp Asn Gly Gly Ser Thr His Tyr Ala Asp Ser
1 5 10 15

Val Lys Gly Arg
20

<210> 137
<211> 15
<212> PRT
<213> Artificial Sequence

<220>
<223> C4.4a binder

<400> 137

Ala Lys Gly Asp Tyr Leu Val Tyr Lys Ser Tyr Tyr Phe Lys Ser
1 5 10 15

<210> 138
<211> 13
<212> PRT
<213> Artificial Sequence

<220>
<223> C4.4a binder

<400> 138

Ser Gly Ser Ser Ser Asn Ile Gly Ser Asn Pro Val Asn
1               5                   10

<210> 139
<211> 7
<212> PRT
<213> Artificial Sequence

<220>
<223> C4.4a binder

<400> 139

Arg Asn Asn Gln Arg Pro Ser
1               5

<210> 140
<211> 12
<212> PRT
<213> Artificial Sequence

<220>
<223> C4.4a binder

<400> 140

Cys Ala Ala Trp Asp Asp Ser Leu Ser Gly Trp Ala
1               5                   10

<210> 141
<211> 122
<212> PRT
<213> Artificial Sequence

<220>
<223> C4.4a binder

<400> 141

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Ala Pro Gly Lys Gly Leu Glu Trp Val
        35                  40                  45

```
Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser
        115                 120


<210>  142
<211>  112
<212>  PRT
<213>  Artificial Sequence

<220>
<223>  C4.4a binder

<400>  142

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Ala Phe Gly Gly Gly Thr Lys Leu Thr Val Leu Gly Gln
            100                 105                 110


<210>  143
<211>  366
<212>  DNA
```

<213>  Artificial Sequence

<220>
<223>  C4.4a binder

<400>  143
gaggtgcagc tgctggaaag cggcggaggc ctggtgcagc ctggcggaag cctgagactg    60

agctgtgccg ccagcggctt caccttcagc agctaccaga tgacctggat cagacaggcc    120

cctggcaagg gcctggaatg ggtgtccggc atcagctgga acggcggcag cacccactac    180

gccgacagcg tgaagggccg gttcaccatc agccgggaca acagcaagaa caccctgtac    240

ctgcagatga acagcctgcg ggccgaggac accgccgtgt actactgcgc caagggcgac    300

tacctggtgt acaagagcta ctacttcaag agctggggcc agggcacact ggtcaccgtc    360

agctca    366

<210>  144
<211>  336
<212>  DNA
<213>  Artificial Sequence

<220>
<223>  C4.4a binder

<400>  144
cagagcgtgc tgacccagcc tcctagcgcc tctggcaccc ctggccagag agtgaccatc    60

agctgcagcg gcagcagcag caacatcggc agcaaccccg tgaactggta tcagcagctg    120

cccggcaccg cccccaagct gctgatctac cggaacaacc agcggcccag cggcgtgccc    180

gacagatttt ccggcagcaa gagcggcacc agcgccagcc tggccatcag cggcctgaga    240

agcgaggacg aggccgacta ctactgcgcc gcctgggacg atagcctgag cggctgggcc    300

tttggcggcg gaacaaagtt aaccgtgctg ggccag    336

<210>  145
<211>  9
<212>  PRT
<213>  Artificial Sequence

<220>
<223>  C4.4a binder

<400>  145

Phe Ser Asn Ala Trp Met Ser Trp Val
1               5

<210>  146
<211>  20
<212>  PRT
<213>  Artificial Sequence

<220>

<223> C4.4a binder

<400> 146

Val Ser Tyr Ile Ser Ser Ser Gly Ser Thr Ile Tyr Tyr Ala Asp Ser
1 5 10 15

Val Lys Gly Arg
20

<210> 147
<211> 10
<212> PRT
<213> Artificial Sequence

<220>
<223> C4.4a binder

<400> 147

Ala Arg Glu Gly Leu Trp Ala Phe Asp Lys
1 5 10

<210> 148
<211> 14
<212> PRT
<213> Artificial Sequence

<220>
<223> C4.4a binder

<400> 148

Thr Gly Ser Ser Ser Asn Ile Gly Ala Gly Tyr Val Val His
1 5 10

<210> 149
<211> 7
<212> PRT
<213> Artificial Sequence

<220>
<223> C4.4a binder

<400> 149

Asp Asn Asn Lys Arg Pro Ser
1 5

<210> 150
<211> 12
<212> PRT
<213> Artificial Sequence

<220>
<223> C4.4a binder

<400> 150

Cys Ala Ala Tyr Asp Asp Ser Leu Lys Gly Pro Val
1               5                   10

<210> 151
<211> 117
<212> PRT
<213> Artificial Sequence

<220>
<223> C4.4a binder

<400> 151

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Thr Ser
        115

<210> 152
<211> 113
<212> PRT
<213> Artificial Sequence

<220>
<223> C4.4a binder

<400> 152

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Ser
85 90 95

Leu Lys Gly Pro Val Phe Gly Gly Gly Thr Lys Leu Thr Val Leu Gly
100 105 110

Gln

<210> 153
<211> 9
<212> PRT
<213> Artificial Sequence

<220>
<223> C4.4a binder

<400> 153

Phe Ser Asn Ala Trp Met Ser Trp Val
1 5

<210> 154
<211> 20
<212> PRT
<213> Artificial Sequence

<220>
<223> C4.4a binder

<400> 154

Val Ser Tyr Ile Ser Ser Ser Gly Ser Thr Ile Tyr Tyr Ala Asp Ser
1 5 10 15

Val Lys Gly Arg
20

<210> 155
<211> 10
<212> PRT
<213> Artificial Sequence

<220>
<223> C4.4a binder

<400> 155

Ala Arg Glu Gly Leu Trp Ala Phe Asp Lys
1               5                   10

<210> 156
<211> 14
<212> PRT
<213> Artificial Sequence

<220>
<223> C4.4a binder

<400> 156

Thr Gly Ser Ser Ser Asn Ile Gly Ala Gly Tyr Val Val His
1               5                   10

<210> 157
<211> 7
<212> PRT
<213> Artificial Sequence

<220>
<223> C4.4a binder

<400> 157

Asp Asn Asn Lys Arg Pro Ser
1               5

<210> 158
<211> 12
<212> PRT
<213> Artificial Sequence

<220>
<223> C4.4a binder

<400> 158

Cys Ala Ala Phe Asp Asp Ser Leu Asn Gly Pro Val
1               5                   10

<210> 159
<211> 117
<212> PRT
<213> Artificial Sequence

<220>
<223> C4.4a binder

<400> 159

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Lys Trp Gly Gln Gly Thr Leu
100 105 110

Val Thr Val Thr Ser
115

<210> 160
<211> 113
<212> PRT
<213> Artificial Sequence

<220>
<223> C4.4a binder

<400> 160

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Ser
85 90 95

Leu Asn Gly Pro Val Phe Gly Gly Gly Thr Lys Leu Thr Val Leu Gly
100 105 110

Gln

<210> 161
<211> 9
<212> PRT
<213> Artificial Sequence

<220>
<223> C4.4a binder

<400> 161

Phe Ser Asn Ala Trp Met Ser Trp Val
1 5

<210> 162
<211> 20
<212> PRT
<213> Artificial Sequence

<220>
<223> C4.4a binder

<400> 162

Val Ser Tyr Ile Ser Ser Ser Gly Ser Thr Ile Tyr Tyr Ala Asp Ser
1 5 10 15

Val Lys Gly Arg
20

<210> 163
<211> 10
<212> PRT
<213> Artificial Sequence

<220>
<223> C4.4a binder

<400> 163

Ala Arg Glu Gly Leu Trp Ala Phe Asp Lys
1 5 10

<210> 164
<211> 14
<212> PRT
<213> Artificial Sequence

<220>
<223> C4.4a binder

<400> 164

Thr Gly Ser Ser Ser Asn Ile Gly Ala Gly Tyr Val Val His
1 5 10

<210> 165
<211> 7
<212> PRT
<213> Artificial Sequence

<220>
<223> C4.4a binder

<400> 165

Asp Asn Asn Lys Arg Pro Ser
1 5

<210> 166
<211> 12
<212> PRT
<213> Artificial Sequence

<220>
<223> C4.4a binder

<400> 166

Cys Ala Ala Tyr Asp Asp Ser Leu Ser Gly Pro Val
1 5 10

<210> 167
<211> 117
<212> PRT
<213> Artificial Sequence

<220>
<223> C4.4a binder

<400> 167

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Lys Trp Gly Gln Gly Thr Leu
100 105 110

Val Thr Val Thr Ser
115

<210> 168
<211> 113
<212> PRT
<213> Artificial Sequence

<220>
<223> C4.4a binder

<400> 168

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Ser
85 90 95

Leu Asn Gly Pro Val Phe Gly Gly Gly Thr Lys Leu Thr Val Leu Gly
100 105 110

Gln

<210> 169
<211> 9
<212> PRT
<213> Artificial Sequence

<220>
<223> C4.4a binder

<400> 169

Phe Ser Ser Ala Trp Met Ser Trp Val
1 5

<210> 170
<211> 20
<212> PRT
<213> Artificial Sequence

<220>
<223> C4.4a binder

<400> 170

Val Ser Tyr Ile Ser Ser Ser Gly Ser Ser Thr Tyr Tyr Ala Asp Ser
1 5 10 15

Val Lys Gly Arg
20

<210> 171
<211> 10
<212> PRT
<213> Artificial Sequence

<220>
<223> C4.4a binder

<400> 171

Ala Arg Glu Gly Leu Trp Ala Phe Asp Trp
1 5 10

<210> 172
<211> 14
<212> PRT
<213> Artificial Sequence

<220>
<223> C4.4a binder

<400> 172

Ser Gly Ser Ser Ser Asn Ile Gly Ala Gly Tyr Val Val His
1 5 10

<210> 173
<211> 7
<212> PRT
<213> Artificial Sequence

<220>
<223> C4.4a binder

<400> 173

Asp Asn Asn Gln Arg Pro Ser

1 5

<210> 174
<211> 12
<212> PRT
<213> Artificial Sequence

<220>
<223> C4.4a binder

<400> 174

Cys Ala Ala Tyr Asp Asp Ser Leu Ser Gly Pro Val
1 5 10

<210> 175
<211> 117
<212> PRT
<213> Artificial Sequence

<220>
<223> C4.4a binder

<400> 175

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Ala
20 25 30

Trp Met Ser Trp Val Arg Gln Ala Pro Gly Lys Gly Leu Glu Trp Val
35 40 45

Ser Tyr Ile Ser Ser Ser Gly Ser Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Trp Trp Gly Gln Gly Thr Leu
100 105 110

Val Thr Val Thr Ser
115

<210> 176
<211> 113
<212> PRT
<213> Artificial Sequence

<220>
<223> C4.4a binder

<400> 176

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Gln Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Ser
85 90 95

Leu Ser Gly Pro Val Phe Gly Gly Gly Thr Lys Leu Thr Val Leu Gly
100 105 110

Gln

<210> 177
<211> 9
<212> PRT
<213> Artificial Sequence

<220>
<223> C4.4a binder

<400> 177

Phe Ser Ser Ala Trp Met Ser Trp Val
1 5

<210> 178
<211> 20
<212> PRT
<213> Artificial Sequence

<220>
<223> C4.4a binder

<400> 178

Val Ser Tyr Ile Ser Ser Ser Gly Ser Ser Thr Tyr Tyr Ala Asp Ser

1 5 10 15

Val Lys Gly Arg
20

<210> 179
<211> 10
<212> PRT
<213> Artificial Sequence

<220>
<223> C4.4a binder

<400> 179

Ala Arg Glu Gly Leu Trp Ala Phe Asp Asn
1 5 10

<210> 180
<211> 14
<212> PRT
<213> Artificial Sequence

<220>
<223> C4.4a binder

<400> 180

Ser Gly Ser Ser Ser Asn Ile Gly Ala Gly Tyr Val Val His
1 5 10

<210> 181
<211> 7
<212> PRT
<213> Artificial Sequence

<220>
<223> C4.4a binder

<400> 181

Asp Asn Asn Gln Arg Pro Ser
1 5

<210> 182
<211> 12
<212> PRT
<213> Artificial Sequence

<220>
<223> C4.4a binder

<400> 182

Cys Ala Ala Tyr Asp Asp Ser Leu Asn Gly Pro Val
1 5 10

<210> 183
<211> 117
<212> PRT
<213> Artificial Sequence

<220>
<223> C4.4a binder

<400> 183

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Thr Ser
        115
```

<210> 184
<211> 113
<212> PRT
<213> Artificial Sequence

<220>
<223> C4.4a binder

<400> 184

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45
```

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Tyr Asp Asp Ser
                85                  90                  95

Leu Asn Gly Pro Val Phe Gly Gly Gly Thr Lys Leu Thr Val Leu Gly
            100                 105                 110

Gln

<210> 185
<211> 9
<212> PRT
<213> Artificial Sequence

<220>
<223> C4.4a binder

<400> 185

Phe Ser Ser Ala Trp Met Ser Trp Val
1               5

<210> 186
<211> 20
<212> PRT
<213> Artificial Sequence

<220>
<223> C4.4a binder

<400> 186

Val Ser Tyr Ile Ser Ser Ser Gly Ser Ser Thr Tyr Tyr Ala Asp Ser
1               5                   10                  15

Val Lys Gly Arg
            20

<210> 187
<211> 10
<212> PRT
<213> Artificial Sequence

<220>
<223> C4.4a binder

<400> 187

Ala Arg Glu Gly Leu Trp Ala Phe Asp Tyr
1               5                   10

<210>  188
<211>  14
<212>  PRT
<213>  Artificial Sequence

<220>
<223>  C4.4a binder

<400>  188

Ser Gly Ser Ser Ser Asn Ile Gly Ala Gly Tyr Val Val His
1               5                   10

<210>  189
<211>  7
<212>  PRT
<213>  Artificial Sequence

<220>
<223>  C4.4a binder

<400>  189

Asp Asn Asn Gln Arg Pro Ser
1               5

<210>  190
<211>  12
<212>  PRT
<213>  Artificial Sequence

<220>
<223>  C4.4a binder

<400>  190

Cys Ala Ala Trp Asp Asp Arg Leu Asn Gly Pro Val
1               5                   10

<210>  191
<211>  117
<212>  PRT
<213>  Artificial Sequence

<220>
<223>  C4.4a binder

<400>  191

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

```
Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
            100                 105                 110

Val Thr Val Ser Ser
        115


<210>  192
<211>  113
<212>  PRT
<213>  Artificial Sequence

<220>
<223>  C4.4a binder

<400>  192

Glu Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Trp Asp Asp Arg
                85                  90                  95

Leu Asn Gly Pro Val Phe Gly Gly Gly Thr Lys Leu Thr Val Leu Gly
            100                 105                 110
```

Gln

<210> 193
<211> 11
<212> PRT
<213> Artificial Sequence

<220>
<223> C4.4a binder

<400> 193

Phe Thr Phe Ser Asn Ala Trp Met Ser Trp Val
1 5 10

<210> 194
<211> 20
<212> PRT
<213> Artificial Sequence

<220>
<223> C4.4a binder

<400> 194

Val Ser Tyr Ile Ser Ser Ser Gly Ser Thr Ile Tyr Tyr Ala Asp Ser
1 5 10 15

Val Lys Gly Arg
20

<210> 195
<211> 10
<212> PRT
<213> Artificial Sequence

<220>
<223> C4.4a binder

<400> 195

Ala Arg Glu Gly Leu Trp Ala Phe Asp Tyr
1 5 10

<210> 196
<211> 14
<212> PRT
<213> Artificial Sequence

<220>
<223> C4.4a binder

<400> 196

Thr Gly Ser Ser Ser Asn Ile Gly Ala Gly Tyr Val Val His
1 5 10

<210> 197
<211> 7
<212> PRT
<213> Artificial Sequence

<220>
<223> C4.4a binder

<400> 197

Asp Asn Asn Lys Arg Pro Ser
1               5

<210> 198
<211> 12
<212> PRT
<213> Artificial Sequence

<220>
<223> C4.4a binder

<400> 198

Cys Ala Ala Tyr Asp Asp Arg Leu Asn Gly Pro Val
1               5                   10

<210> 199
<211> 117
<212> PRT
<213> Artificial Sequence

<220>
<223> C4.4a binder

<400> 199

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Tyr Trp Gly Gln Gly Thr Leu
100 105 110

Val Thr Val Ser Ser
115

<210> 200
<211> 113
<212> PRT
<213> Artificial Sequence

<220>
<223> C4.4a binder

<400> 200

Glu Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Arg
85 90 95

Leu Asn Gly Pro Val Phe Gly Gly Gly Thr Lys Leu Thr Val Leu Gly
100 105 110

Gln

<210> 201
<211> 11
<212> PRT
<213> Artificial Sequence

<220>
<223> C4.4a binder

<400> 201

Phe Thr Phe Ser Ser Ala Trp Met Ser Trp Val
1 5 10

<210> 202
<211> 20
<212> PRT
<213> Artificial Sequence

<220>
<223> C4.4a binder

<400> 202

Val Ser Tyr Ile Ser Ser Ser Gly Ser Ser Thr Tyr Tyr Ala Asp Ser
1 5 10 15

Val Lys Gly Arg
20

<210> 203
<211> 10
<212> PRT
<213> Artificial Sequence

<220>
<223> C4.4a binder

<400> 203

Ala Arg Glu Gly Leu Trp Ala Phe Asp Gly
1 5 10

<210> 204
<211> 14
<212> PRT
<213> Artificial Sequence

<220>
<223> C4.4a binder

<400> 204

Ser Gly Ser Ser Ser Asn Ile Gly Ala Gly Tyr Val Val His
1 5 10

<210> 205
<211> 7
<212> PRT
<213> Artificial Sequence

<220>
<223> C4.4a binder

<400> 205

Asp Asn Asn Gln Arg Pro Ser
1 5

<210> 206
<211> 12

<212> PRT
<213> Artificial Sequence

<220>
<223> C4.4a binder

<400> 206

Cys Ala Ala Tyr Asp Asp Ser Leu Asn Arg Pro Val
1 5 10

<210> 207
<211> 117
<212> PRT
<213> Artificial Sequence

<220>
<223> C4.4a binder

<400> 207

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Ala
20 25 30

Trp Met Ser Trp Val Arg Gln Ala Pro Gly Lys Gly Leu Glu Trp Val
35 40 45

Ser Tyr Ile Ser Ser Ser Gly Ser Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Gly Trp Gly Gln Gly Thr Leu
100 105 110

Val Thr Val Ser Ser
115

<210> 208
<211> 113
<212> PRT
<213> Artificial Sequence

<220>
<223> C4.4a binder

<400> 208

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Gln Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Tyr Asp Asp Ser
85 90 95

Leu Asn Arg Pro Val Phe Gly Gly Gly Thr Lys Leu Thr Val Leu Gly
100 105 110

Gln

<210> 209
<211> 11
<212> PRT
<213> Artificial Sequence

<220>
<223> C4.4a binder

<400> 209

Phe Thr Phe Ser Asn Ala Trp Met Ser Trp Val
1 5 10

<210> 210
<211> 20
<212> PRT
<213> Artificial Sequence

<220>
<223> C4.4a binder

<400> 210

Val Ser Tyr Ile Ser Ser Ser Gly Ser Thr Ile Tyr Tyr Ala Asp Ser
1 5 10 15

Val Lys Gly Arg
20

<210> 211
<211> 10
<212> PRT
<213> Artificial Sequence

<220>
<223> C4.4a binder

<400> 211

Ala Arg Glu Gly Leu Trp Ala Phe Asp Tyr
1 5 10

<210> 212
<211> 14
<212> PRT
<213> Artificial Sequence

<220>
<223> C4.4a binder

<400> 212

Thr Gly Ser Ser Ser Asn Ile Gly Ala Gly Tyr Val Val His
1 5 10

<210> 213
<211> 7
<212> PRT
<213> Artificial Sequence

<220>
<223> C4.4a binder

<400> 213

Asp Asn Asn Lys Arg Pro Ser
1 5

<210> 214
<211> 12
<212> PRT
<213> Artificial Sequence

<220>
<223> C4.4a binder

<400> 214

Cys Ala Ala Phe Asp Asp Ser Leu Asn Gly Pro Val
1 5 10

<210> 215
<211> 117
<212> PRT
<213> Artificial Sequence

<220>
<223> C4.4a binder

<400> 215

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ser Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Tyr Trp Gly Gln Gly Thr Leu
100 105 110

Val Thr Val Ser Ser
115

<210> 216
<211> 113
<212> PRT
<213> Artificial Sequence

<220>
<223> C4.4a binder

<400> 216

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Ser
85 90 95

Leu Asn Gly Pro Val Phe Gly Gly Gly Thr Lys Leu Thr Val Leu Gly
100 105 110

Gln

<210> 217
<211> 9
<212> PRT
<213> Artificial Sequence

<220>
<223> C4.4a binder

<400> 217

Phe Ser Ser Ala Trp Met Ser Trp Val
1 5

<210> 218
<211> 20
<212> PRT
<213> Artificial Sequence

<220>
<223> C4.4a binder

<400> 218

Val Ser Tyr Ile Ser Ser Ser Gly Ser Ser Thr Tyr Tyr Ala Asp Ser
1 5 10 15

Val Lys Gly Arg
20

<210> 219
<211> 10
<212> PRT
<213> Artificial Sequence

<220>
<223> C4.4a binder

<400> 219

Ala Arg Glu Gly Leu Trp Ala Phe Asp Lys
1 5 10

<210> 220

<211> 14
<212> PRT
<213> Artificial Sequence

<220>
<223> C4.4a binder

<400> 220

Thr Gly Ser Ser Ser Asn Ile Gly Ala Gly Tyr Val Val His
1 5 10

<210> 221
<211> 7
<212> PRT
<213> Artificial Sequence

<220>
<223> C4.4a binder

<400> 221

Asp Asn Asn Lys Arg Pro Ser
1 5

<210> 222
<211> 12
<212> PRT
<213> Artificial Sequence

<220>
<223> C4.4a binder

<400> 222

Cys Ala Ala Phe Asp Asp Ser Leu Asn Gly Pro Val
1 5 10

<210> 223
<211> 117
<212> PRT
<213> Artificial Sequence

<220>
<223> C4.4a binder

<400> 223

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Ala
20 25 30

Trp Met Ser Trp Val Arg Gln Ala Pro Gly Lys Gly Leu Glu Trp Val
35 40 45

Ser Tyr Ile Ser Ser Ser Gly Ser Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Glu Gly Leu Trp Ala Phe Asp Lys Trp Gly Gln Gly Thr Leu
100 105 110

Val Thr Val Thr Ser
115

<210> 224
<211> 113
<212> PRT
<213> Artificial Sequence

<220>
<223> C4.4a binder

<400> 224

Gln Ser Val Leu Thr Gln Pro Pro Ser Ala Ser Gly Thr Pro Gly Gln
1 5 10 15

Arg Val Thr Ile Ser Cys Thr Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Lys Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Ser
85 90 95

Leu Asn Gly Pro Val Phe Gly Gly Gly Thr Lys Leu Thr Val Leu Gly
100 105 110

Gln

<210> 225

<211> 11
<212> PRT
<213> Artificial Sequence

<220>
<223> C4.4a binder

<400> 225

Phe Thr Phe Ser Ser Ala Trp Met Ser Trp Val
1 5 10

<210> 226
<211> 20
<212> PRT
<213> Artificial Sequence

<220>
<223> C4.4a binder

<400> 226

Val Ser Tyr Ile Ser Ser Ser Gly Ser Ser Thr Tyr Tyr Ala Asp Ser
1 5 10 15

Val Lys Gly Arg
20

<210> 227
<211> 10
<212> PRT
<213> Artificial Sequence

<220>
<223> C4.4a binder

<400> 227

Ala Arg Glu Gly Leu Trp Ala Phe Asp Lys
1 5 10

<210> 228
<211> 14
<212> PRT
<213> Artificial Sequence

<220>
<223> C4.4a binder

<400> 228

Ser Gly Ser Ser Ser Asn Ile Gly Ala Gly Tyr Val Val His
1 5 10

<210> 229
<211> 7
<212> PRT
<213> Artificial Sequence

<220>
<223>  C4.4a binder

<400>  229

Asp Asn Asn Gln Arg Pro Ser
1               5

<210>  230
<211>  12
<212>  PRT
<213>  Artificial Sequence

<220>
<223>  C4.4a binder

<400>  230

Cys Ala Ala Phe Asp Asp Ser Leu Asn Gly Pro Val
1               5                   10

<210>  231
<211>  117
<212>  PRT
<213>  Artificial Sequence

<220>
<223>  C4.4a binder

<400>  231

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser

115

<210> 232
<211> 113
<212> PRT
<213> Artificial Sequence

<220>
<223> C4.4a binder

<400> 232

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
            20                  25                  30

Tyr Val Val His Trp Tyr Gln Gln Leu Pro Gly Thr Ala Pro Lys Leu
        35                  40                  45

Leu Ile Tyr Asp Asn Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Ala Ala Phe Asp Asp Ser
                85                  90                  95

Leu Asn Gly Pro Val Phe Gly Gly Gly Thr Lys Leu Thr Val Leu Gly
            100                 105                 110

Gln
```

<210> 233
<211> 11
<212> PRT
<213> Artificial Sequence

<220>
<223> C4.4a binder

<400> 233

```
Phe Thr Phe Ser Ser Ala Trp Met Ser Trp Val
1               5                   10
```

<210> 234
<211> 20
<212> PRT
<213> Artificial Sequence

<220>
<223>  C4.4a binder

<400>  234

Val Ser Tyr Ile Ser Ser Ser Gly Ser Ser Thr Tyr Tyr Ala Asp Ser
1               5                   10                  15

Val Lys Gly Arg
            20

<210>  235
<211>  10
<212>  PRT
<213>  Artificial Sequence

<220>
<223>  C4.4a binder

<400>  235

Ala Arg Glu Gly Leu Trp Ala Phe Asp Lys
1               5                   10

<210>  236
<211>  14
<212>  PRT
<213>  Artificial Sequence

<220>
<223>  C4.4a binder

<400>  236

Ser Gly Ser Ser Ser Asn Ile Gly Ala Gly Tyr Val Val His
1               5                   10

<210>  237
<211>  7
<212>  PRT
<213>  Artificial Sequence

<220>
<223>  C4.4a binder

<400>  237

Asp Asn Asn Gln Arg Pro Ser
1               5

<210>  238
<211>  12
<212>  PRT
<213>  Artificial Sequence

<220>
<223>  C4.4a binder

<400> 238

Cys Ala Ala Phe Asp Asp Arg Leu Ser Gly Pro Val
1               5                   10

<210> 239
<211> 117
<212> PRT
<213> Artificial Sequence

<220>
<223> C4.4a binder

<400> 239

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ala
            20                  25                  30

Trp Met Ser Trp Val Arg Gln Ala Pro Gly Lys Gly Leu Glu Trp Val
        35                  40                  45

Ser Tyr Ile Ser Ser Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Leu Trp Ala Phe Asp Lys Trp Gly Gln Gly Thr Leu
            100                 105                 110

Val Thr Val Ser Ser
        115

<210> 240
<211> 113
<212> PRT
<213> Artificial Sequence

<220>
<223> C4.4a binder

<400> 240

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ala Gly
20 25 30

Tyr Val Val His Trp Tyr Gln Gln Leu Pro Gly Thr Ala Pro Lys Leu
35 40 45

Leu Ile Tyr Asp Asn Asn Gln Arg Pro Ser Gly Val Pro Asp Arg Phe
50 55 60

Ser Gly Ser Lys Ser Gly Thr Ser Ala Ser Leu Ala Ile Ser Gly Leu
65 70 75 80

Arg Ser Glu Asp Glu Ala Asp Tyr Tyr Cys Ala Ala Phe Asp Asp Arg
85 90 95

Leu Ser Gly Pro Val Phe Gly Gly Gly Thr Lys Leu Thr Val Leu Gly
100 105 110

Gln

<210> 241
<211> 9
<212> PRT
<213> Artificial Sequence

<220>
<223> C4.4a binder

<400> 241

Phe Ser Asp Tyr Gln Met Thr Trp Ile
1 5

<210> 242
<211> 20
<212> PRT
<213> Artificial Sequence

<220>
<223> C4.4a binder

<400> 242

Val Ser Gly Val Ser Trp Asn Gly Ala Arg Thr His Tyr Ala Asp Ser
1 5 10 15

Val Lys Gly Arg
20

<210> 243
<211> 15
<212> PRT

<213>  Artificial Sequence

<220>
<223>  C4.4a binder

<400>  243

Ala Lys Gly Asp Tyr Leu Val Tyr Ser Ser Tyr Tyr Phe Lys Ser
1               5                   10                  15

<210>  244
<211>  13
<212>  PRT
<213>  Artificial Sequence

<220>
<223>  C4.4a binder

<400>  244

Ser Gly Ser Ser Ser Asn Val Gly Ser Asn Pro Val Asn
1               5                   10

<210>  245
<211>  7
<212>  PRT
<213>  Artificial Sequence

<220>
<223>  C4.4a binder

<400>  245

Arg Asn Asn Gln Arg Pro Ser
1               5

<210>  246
<211>  13
<212>  PRT
<213>  Artificial Sequence

<220>
<223>  C4.4a binder

<400>  246

Cys Ala Ala Trp Asp Asp Arg Leu Asn Gly Trp Thr Gly
1               5                   10

<210>  247
<211>  122
<212>  PRT
<213>  Artificial Sequence

<220>
<223>  C4.4a binder

<400>  247

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Val Ser Trp Asn Gly Ala Arg Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Ser Ser Tyr Tyr Phe Lys Ser Trp
            100                 105                 110

Gly Gln Gly Thr Leu Val Thr Val Thr Ser
        115                 120

<210> 248
<211> 112
<212> PRT
<213> Artificial Sequence

<220>
<223> C4.4a binder

<400> 248

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Val Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu

85 90 95

Asn Gly Trp Gly Phe Gly Gly Gly Thr Lys Leu Thr Val Leu Gly Gln
100 105 110

<210> 249
<211> 11
<212> PRT
<213> Artificial Sequence

<220>
<223> C4.4a binder

<400> 249

Phe Thr Phe Ser Ser Tyr Gln Met Thr Trp Ile
1 5 10

<210> 250
<211> 20
<212> PRT
<213> Artificial Sequence

<220>
<223> C4.4a binder

<400> 250

Val Ser Gly Ile Ser Trp Asn Gly Gly Ser Thr His Tyr Ala Asp Ser
1 5 10 15

Val Lys Gly Arg
20

<210> 251
<211> 15
<212> PRT
<213> Artificial Sequence

<220>
<223> C4.4a binder

<400> 251

Ala Lys Gly Asp Tyr Leu Val Tyr Ser Ala Tyr Tyr Phe Asp Ser
1 5 10 15

<210> 252
<211> 13
<212> PRT
<213> Artificial Sequence

<220>
<223> C4.4a binder

<400> 252

Ser Gly Ser Ser Ser Asn Ile Gly Ser Asn Pro Val Asn
1               5                   10

<210>  253
<211>  7
<212>  PRT
<213>  Artificial Sequence

<220>
<223>  C4.4a binder

<400>  253

Arg Asn Asn Gln Arg Pro Ser
1               5

<210>  254
<211>  12
<212>  PRT
<213>  Artificial Sequence

<220>
<223>  C4.4a binder

<400>  254

Cys Ala Ala Trp Asp Asp Arg Leu Asn Gly Trp Gly
1               5                   10

<210>  255
<211>  122
<212>  PRT
<213>  Artificial Sequence

<220>
<223>  C4.4a binder

<400>  255

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85 90 95

Ala Lys Gly Asp Tyr Leu Val Tyr Ser Ala Tyr Tyr Phe Asp Ser Trp
100 105 110

Gly Gln Gly Thr Leu Val Thr Val Ser Ser
115 120

<210> 256
<211> 112
<212> PRT
<213> Artificial Sequence

<220>
<223> C4.4a binder

<400> 256

Glu Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Arg Leu
85 90 95

Asn Gly Trp Gly Phe Gly Gly Gly Thr Lys Leu Thr Val Leu Gly Gln
100 105 110

<210> 257
<211> 11
<212> PRT
<213> Artificial Sequence

<220>
<223> C4.4a binder

<400> 257

Phe Thr Phe Ser Asp Tyr Gln Met Thr Trp Ile
1 5 10

<210> 258
<211> 20
<212> PRT
<213> Artificial Sequence

<220>
<223> C4.4a binder

<400> 258

Val Ser Gly Ile Ser Trp Asn Gly Gly Ser Thr His Tyr Ala Asp Ser
1 5 10 15

Val Lys Gly Arg
20

<210> 259
<211> 15
<212> PRT
<213> Artificial Sequence

<220>
<223> C4.4a binder

<400> 259

Ala Lys Gly Asp Tyr Leu Val Tyr Ser Ser Tyr Tyr Phe Lys Tyr
1 5 10 15

<210> 260
<211> 13
<212> PRT
<213> Artificial Sequence

<220>
<223> C4.4a binder

<400> 260

Ser Gly Ser Ser Ser Asn Ile Gly Ser Asn Pro Val Asn
1 5 10

<210> 261
<211> 7
<212> PRT
<213> Artificial Sequence

<220>
<223> C4.4a binder

<400> 261

Arg Asn Asn Gln Arg Pro Ser
1 5

<210> 262
<211> 12
<212> PRT

<213> Artificial Sequence

<220>
<223> C4.4a binder

<400> 262

Cys Ala Ala Trp Asp Asp Arg Leu Asn Gly Trp Ala
1 5 10

<210> 263
<211> 122
<212> PRT
<213> Artificial Sequence

<220>
<223> C4.4a binder

<400> 263

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ser Tyr Tyr Phe Lys Tyr Trp
100 105 110

Gly Gln Gly Thr Leu Val Thr Val Ser Ser
115 120

<210> 264
<211> 112
<212> PRT
<213> Artificial Sequence

<220>
<223> C4.4a binder

<400> 264

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Asn Gly Trp Ala Phe Gly Gly Gly Thr Lys Leu Thr Val Leu Gly Gln
            100                 105                 110

<210> 265
<211> 11
<212> PRT
<213> Artificial Sequence

<220>
<223> C4.4a binder

<400> 265

Phe Thr Phe Ser Asp Tyr Gln Met Thr Trp Ile
1               5                   10

<210> 266
<211> 20
<212> PRT
<213> Artificial Sequence

<220>
<223> C4.4a binder

<400> 266

Val Ser Gly Ile Ser Trp Asn Gly Gly Ser Thr His Tyr Ala Asp Ser
1               5                   10                  15

Val Lys Gly Arg
            20

<210> 267
<211> 15
<212> PRT

<213> Artificial Sequence

<220>
<223> C4.4a binder

<400> 267

Ala Lys Gly Asp Tyr Leu Val Tyr Ser Ser Tyr Tyr Phe Lys Ser
1 5 10 15

<210> 268
<211> 13
<212> PRT
<213> Artificial Sequence

<220>
<223> C4.4a binder

<400> 268

Ser Gly Ser Ser Ser Asn Ile Gly Ser Asn Pro Val Asn
1 5 10

<210> 269
<211> 7
<212> PRT
<213> Artificial Sequence

<220>
<223> C4.4a binder

<400> 269

Arg Asn Asn Gln Arg Pro Ser
1 5

<210> 270
<211> 12
<212> PRT
<213> Artificial Sequence

<220>
<223> C4.4a binder

<400> 270

Cys Ala Ala Trp Asp Asp Ser Leu Asn Gly Trp Gly
1 5 10

<210> 271
<211> 122
<212> PRT
<213> Artificial Sequence

<220>
<223> C4.4a binder

<400> 271

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ser Tyr Tyr Phe Lys Ser Trp
100 105 110

Gly Gln Gly Thr Leu Val Thr Val Ser Ser
115 120

<210> 272
<211> 112
<212> PRT
<213> Artificial Sequence

<220>
<223> C4.4a binder

<400> 272

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Ser Leu

85 90 95

Asn Gly Trp Gly Phe Gly Gly Gly Thr Lys Leu Thr Val Leu Gly Gln
100 105 110

<210> 273
<211> 11
<212> PRT
<213> Artificial Sequence

<220>
<223> C4.4a binder

<400> 273

Phe Thr Phe Ser Asp Tyr Gln Met Thr Trp Ile
1 5 10

<210> 274
<211> 20
<212> PRT
<213> Artificial Sequence

<220>
<223> C4.4a binder

<400> 274

Val Ser Gly Ile Ser Trp Asn Gly Gly Ser Thr His Tyr Ala Asp Ser
1 5 10 15

Val Lys Gly Arg
20

<210> 275
<211> 15
<212> PRT
<213> Artificial Sequence

<220>
<223> C4.4a binder

<400> 275

Ala Lys Gly Asp Tyr Leu Val Tyr Lys Ser Tyr Tyr Phe Lys Ser
1 5 10 15

<210> 276
<211> 13
<212> PRT
<213> Artificial Sequence

<220>
<223> C4.4a binder

<400> 276

Ser Gly Ser Ser Ser Asn Ile Gly Ser Asn Pro Val Asn
1               5                   10

<210>  277
<211>  7
<212>  PRT
<213>  Artificial Sequence

<220>
<223>  C4.4a binder

<400>  277

Arg Asn Asn Gln Arg Pro Ser
1               5

<210>  278
<211>  12
<212>  PRT
<213>  Artificial Sequence

<220>
<223>  C4.4a binder

<400>  278

Cys Ala Ala Trp Asp Asp Arg Leu Ser Gly Trp Gly
1               5                   10

<210>  279
<211>  122
<212>  PRT
<213>  Artificial Sequence

<220>
<223>  C4.4a binder

<400>  279

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85 90 95

Ala Lys Gly Asp Tyr Leu Val Tyr Lys Ser Tyr Tyr Phe Lys Ser Trp
100 105 110

Gly Gln Gly Thr Leu Val Thr Val Ser Ser
115 120

<210> 280
<211> 112
<212> PRT
<213> Artificial Sequence

<220>
<223> C4.4a binder

<400> 280

Gln Ser Val Leu Thr Gln Pro Pro Ser Ala Ser Gly Thr Pro Gly Gln
1 5 10 15

Arg Val Thr Ile Ser Cys Ser Gly Ser Ser Ser Asn Ile Gly Ser Asn
20 25 30

Pro Val Asn Trp Tyr Gln Gln Leu Pro Gly Thr Ala Pro Lys Leu Leu
35 40 45

Ile Tyr Arg Asn Asn Gln Arg Pro Ser Gly Val Pro Asp Arg Phe Ser
50 55 60

Gly Ser Lys Ser Gly Thr Ser Ala Ser Leu Ala Ile Ser Gly Leu Arg
65 70 75 80

Ser Glu Asp Glu Ala Asp Tyr Tyr Cys Ala Ala Trp Asp Asp Arg Leu
85 90 95

Ser Gly Trp Gly Phe Gly Gly Gly Thr Lys Leu Thr Val Leu Gly Gln
100 105 110

<210> 281
<211> 11
<212> PRT
<213> Artificial Sequence

<220>
<223> C4.4a binder

<400> 281

Phe Thr Phe Ser Asp Tyr Gln Met Thr Trp Ile
1 5 10

<210> 282
<211> 20
<212> PRT
<213> Artificial Sequence

<220>
<223> C4.4a binder

<400> 282

Val Ser Gly Ile Ser Trp Asn Gly Gly Ser Thr His Tyr Ala Asp Ser
1               5                   10                  15

Val Lys Gly Arg
            20

<210> 283
<211> 15
<212> PRT
<213> Artificial Sequence

<220>
<223> C4.4a binder

<400> 283

Ala Lys Gly Asp Tyr Leu Val Tyr Ser Ser Tyr Tyr Phe Lys Ser
1               5                   10                  15

<210> 284
<211> 13
<212> PRT
<213> Artificial Sequence

<220>
<223> C4.4a binder

<400> 284

Ser Gly Ser Ser Ser Asn Ile Gly Ser Asn Pro Val Asn
1               5                   10

<210> 285
<211> 7
<212> PRT
<213> Artificial Sequence

<220>
<223> C4.4a binder

<400> 285

Arg Asn Asn Gln Arg Pro Ser
1               5

<210> 286
<211> 12
<212> PRT

<213> Artificial Sequence

<220>
<223> C4.4a binder

<400> 286

Cys Ala Ala Trp Asp Asp Arg Leu Ser Gly Trp Gly
1 5 10

<210> 287
<211> 122
<212> PRT
<213> Artificial Sequence

<220>
<223> C4.4a binder

<400> 287

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

Gln Met Thr Trp Ile Arg Gln Thr Pro Gly Lys Gly Leu Glu Trp Val
35 40 45

Ser Gly Ile Ser Trp Asn Gly Gly Ser Thr His Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Asp Tyr Leu Val Tyr Ser Ser Tyr Tyr Phe Lys Ser Trp
100 105 110

Gly Gln Gly Thr Leu Val Thr Val Ser Ser
115 120

<210> 288
<211> 112
<212> PRT
<213> Artificial Sequence

<220>
<223> C4.4a binder

<400> 288

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Arg Leu
                85                  90                  95

Ser Gly Trp Gly Phe Gly Gly Gly Thr Lys Leu Thr Val Leu Gly Gln
            100                 105                 110

<210> 289
<211> 11
<212> PRT
<213> Artificial Sequence

<220>
<223> C4.4a binder

<400> 289

Phe Thr Phe Ser Asp Tyr Gln Met Thr Trp Ile
1               5                   10

<210> 290
<211> 20
<212> PRT
<213> Artificial Sequence

<220>
<223> C4.4a binder

<400> 290

Val Ser Gly Ile Ser Trp Asn Gly Gly Ser Thr His Tyr Ala Asp Ser
1               5                   10                  15

Val Lys Gly Arg
            20

<210> 291
<211> 15
<212> PRT

<213> Artificial Sequence

<220>
<223> C4.4a binder

<400> 291

Ala Lys Gly Asp Tyr Leu Val Tyr Lys Ser Tyr Tyr Phe Lys Ser
1               5                   10                  15

<210> 292
<211> 13
<212> PRT
<213> Artificial Sequence

<220>
<223> C4.4a binder

<400> 292

Ser Gly Ser Ser Ser Asn Ile Gly Ser Asn Pro Val Asn
1               5                   10

<210> 293
<211> 7
<212> PRT
<213> Artificial Sequence

<220>
<223> C4.4a binder

<400> 293

Arg Asn Asn Gln Arg Pro Ser
1               5

<210> 294
<211> 12
<212> PRT
<213> Artificial Sequence

<220>
<223> C4.4a binder

<400> 294

Cys Ala Ala Trp Asp Asp Ser Leu Asn Gly Trp Gly
1               5                   10

<210> 295
<211> 122
<212> PRT
<213> Artificial Sequence

<220>
<223> C4.4a binder

<400> 295

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gln Met Thr Trp Ile Arg Gln Thr Pro Gly Lys Gly Leu Glu Trp Val
        35                  40                  45

Ser Gly Ile Ser Trp Asn Gly Gly Ser Thr His Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Asp Tyr Leu Val Tyr Lys Ser Tyr Tyr Phe Lys Ser Trp
            100                 105                 110

Gly Gln Gly Thr Leu Val Thr Val Ser Ser
        115                 120


<210>  296
<211>  112
<212>  PRT
<213>  Artificial Sequence

<220>
<223>  C4.4a binder

<400>  296

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Pro Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
```

85 90 95

Asn Gly Trp Gly Phe Gly Gly Gly Thr Lys Leu Thr Val Leu Gly Gln
100 105 110

<210> 297
<211> 9
<212> PRT
<213> Artificial Sequence

<220>
<223> consensus sequence for M20 D02 S-A derived CDR H1

<220>
<221> VARIANT
<222> (3)..(3)
<223> X = D or S

<400> 297

Phe Ser Xaa Tyr Gln Met Thr Trp Ile
1 5

<210> 298
<211> 20
<212> PRT
<213> Artificial Sequence

<220>
<223> consensus sequence for M20 D02 S-A derived CDRH2

<220>
<221> VARIANT
<222> (4)..(4)
<223> X = V or I

<220>
<221> VARIANT
<222> (9)..(9)
<223> X = A or G

<220>
<221> VARIANT
<222> (10)..(10)
<223> X = R or S

<400> 298

Val Ser Gly Xaa Ser Trp Asn Gly Xaa Xaa Thr His Tyr Ala Asp Ser
1 5 10 15

Val Lys Gly Arg
20

<210> 299
<211> 15

<212>  PRT
<213>  Artificial Sequence

<220>
<223>  consensus for M20 D02 S-A derived CDRH3

<220>
<221>  VARIANT
<222>  (9)..(9)
<223>  X = S, K, or R

<220>
<221>  VARIANT
<222>  (10)..(10)
<223>  X =  A, S or R

<220>
<221>  VARIANT
<222>  (14)..(14)
<223>  X =  D, K, E or R

<220>
<221>  VARIANT
<222>  (15)..(15)
<223>  X =  S or Y

<400>  299

```
Ala Lys Gly Asp Tyr Leu Val Tyr Xaa Xaa Tyr Tyr Phe Xaa Xaa
1               5                   10                  15
```

<210>  300
<211>  13
<212>  PRT
<213>  Artificial Sequence

<220>
<223>  consensus seqeunce derived from M20 D02 S-A CDRL1

<220>
<221>  VARIANT
<222>  (7)..(7)
<223>  X = V or I

<400>  300

```
Ser Gly Ser Ser Ser Asn Xaa Gly Ser Asn Pro Val Asn
1               5                   10
```

<210>  301
<211>  12
<212>  PRT
<213>  Artificial Sequence

<220>
<223>  consensus sequence derived from M20 D02 S-A derived CDR L3

<220>

<221> VARIANT
<222> (3)..(3)
<223> X = A, Q or R

<220>
<221> VARIANT
<222> (5)..(5)
<223> X = D or G

<220>
<221> VARIANT
<222> (7)..(7)
<223> X = R or S

<220>
<221> VARIANT
<222> (9)..(9)
<223> X = N, W or S

<220>
<221> VARIANT
<222> (12)..(12)
<223> X = V, A or G

<400> 301

Cys Ala Xaa Trp Xaa Asp Xaa Leu Xaa Gly Trp Xaa
1 5 10

<210> 302
<211> 9
<212> PRT
<213> Artificial Sequence

<220>
<223> consensus sequence derived from M31 B01 CDR H1

<220>
<221> VARIANT
<222> (3)..(3)
<223> X = N or S

<220>
<221> VARIANT
<222> (4)..(4)
<223> X = A or Y

<400> 302

Phe Ser Xaa Xaa Trp Met Ser Trp Val
1 5

<210> 303
<211> 20
<212> PRT
<213> Artificial Sequence

<220>
<223> consensus sequence derived from M31 B01 CDR H2

<220>
<221> VARIANT
<222> (10)..(10)
<223> X = T or S

<220>
<221> VARIANT
<222> (11)..(11)
<223> X = I or T

<400> 303

Val Ser Tyr Ile Ser Ser Ser Gly Ser Xaa Xaa Tyr Tyr Ala Asp Ser
1 5 10 15

Val Lys Gly Arg
20

<210> 304
<211> 10
<212> PRT
<213> Artificial Sequence

<220>
<223> consensus sequence derived from M31 B01 CDR H3

<220>
<221> VARIANT
<222> (10)..(10)
<223> X = Y, K, G, W or N

<400> 304

Ala Arg Glu Gly Leu Trp Ala Phe Asp Xaa
1 5 10

<210> 305
<211> 14
<212> PRT
<213> Artificial Sequence

<220>
<223> consensus sequence derived from M31 B01 CDR L1

<220>
<221> VARIANT
<222> (1)..(1)
<223> X = T or S

<400> 305

Xaa Gly Ser Ser Ser Asn Ile Gly Ala Gly Tyr Val Val His
1 5 10

<210> 306
<211> 7

<212> PRT
<213> Artificial Sequence

<220>
<223> consensus sequence derived from M31 B01 CDR L2

<220>
<221> VARIANT
<222> (4)..(4)
<223> X = K or Q

<400> 306

Asp Asn Asn Xaa Arg Pro Ser
1 5

<210> 307
<211> 12
<212> PRT
<213> Artificial Sequence

<220>
<223> consensus sequence derived from M31 B01 CDR L3

<220>
<221> VARIANT
<222> (4)..(4)
<223> X = W, E, F or Y

<220>
<221> VARIANT
<222> (7)..(7)
<223> X = R, S or M

<220>
<221> VARIANT
<222> (9)..(9)
<223> X = N, K or S

<220>
<221> VARIANT
<222> (10)..(10)
<223> X = G or R

<220>
<221> VARIANT
<222> (11)..(11)
<223> X = P or A

<400> 307

Cys Ala Ala Xaa Asp Asp Xaa Leu Xaa Xaa Xaa Val
1 5 10

<210> 308
<211> 351
<212> DNA
<213> Artificial Sequence

<220>
<223> C4.4a binder

<400> 308
gaggtgcagc tgctggagag cggggggggg ctggtgcagc cggggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcagggg accctggtga ccgtgaccag c 351

<210> 309
<211> 339
<212> DNA
<213> Artificial Sequence

<220>
<223> C4.4a binder

<400> 309
cagagcgtgc tgacccagcc gccgagcgcg agcgggaccc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gaaagggccg 300

gtgtttgggg gggggaccaa actgaccgtg ctggggcag 339

<210> 310
<211> 351
<212> DNA
<213> Artificial Sequence

<220>
<223> C4.4a binder

<400> 310
gaggtgcagc tgctggagag cggggggggg ctggtgcagc cggggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcagggg accctggtga ccgtgaccag c 351

<210> 311
<211> 339

<212> DNA
<213> Artificial Sequence

<220>
<223> C4.4a binder

<400> 311
cagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 312
<211> 351
<212> DNA
<213> Artificial Sequence

<220>
<223> C4.4a binder

<400> 312
gaggtgcagc tgctggagag cgggggggggg ctggtgcagc cggggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcaggggg accctggtga ccgtgaccag c 351

<210> 313
<211> 339
<212> DNA
<213> Artificial Sequence

<220>
<223> C4.4a binder

<400> 313
cagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 314
<211> 351
<212> DNA
<213> Artificial Sequence

<220>
<223> C4.4a binder

<400> 314
gaggtgcagc tgctggagag cggggggggg ctggtgcagc cggggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattggtg gggcaggggg accctggtga ccgtgaccag c 351

<210> 315
<211> 339
<212> DNA
<213> Artificial Sequence

<220>
<223> C4.4a binder

<400> 315
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gagcgggccg 300

gtgtttgggg gggggaccaa actgaccgtg ctggggcag 339

<210> 316
<211> 351
<212> DNA
<213> Artificial Sequence

<220>
<223> C4.4a binder

<400> 316
gaggtgcagc tgctggagag cggggggggg ctggtgcagc cggggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattattg ggggcagggg accctggtga ccgtgaccag c 351

<210> 317
<211> 339
<212> DNA
<213> Artificial Sequence

<220>
<223> C4.4a binder

<400> 317
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gaacgggccg 300

gtgtttgggg gggggaccaa actgaccgtg ctggggcag 339

<210> 318
<211> 351
<212> DNA
<213> Artificial Sequence

<220>
<223> C4.4a binder

<400> 318
gaggtgcagc tgctggagag cggggggggg ctggtgcagc cggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattattg ggggcagggg accctggtga ccgtgagcag c 351

<210> 319
<211> 339
<212> DNA
<213> Artificial Sequence

<220>
<223> C4.4a binder

<400> 319
gagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtggg atgatcgcct gaacgggccg 300

gtgtttgggg gggggaccaa actgaccgtg ctggggcag 339

<210> 320
<211> 351
<212> DNA
<213> Artificial Sequence

<220>
<223> C4.4a binder

<400> 320
gaggtgcagc tgctggagag cggggggggg ctggtgcagc cggggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattattg gggcaggggg accctggtga ccgtgagcag c 351

<210> 321
<211> 339
<212> DNA
<213> Artificial Sequence

<220>
<223> C4.4a binder

<400> 321
gagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtatg atgatcgcct gaacgggccg 300

gtgtttgggg gggggaccaa actgaccgtg ctggggcag 339

<210> 322
<211> 351
<212> DNA
<213> Artificial Sequence

<220>
<223> C4.4a binder

<400> 322
gaggtgcagc tgctggagag cggggggggg ctggtgcagc cggggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgatgggtg gggcaggggg accctggtga ccgtgagcag c 351

<210> 323
<211> 339
<212> DNA
<213> Artificial Sequence

<220>
<223> C4.4a binder

<400> 323
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtatg atgatagcct gaaccgcccg 300

gtgtttgggg gggggaccaa actgaccgtg ctggggcag 339

<210> 324
<211> 351
<212> DNA
<213> Artificial Sequence

<220>
<223> C4.4a binder

<400> 324
gaggtgcagc tgctggagag cggggggggg ctggtgcagc cgggggggag cctgcgcctg 60

agctgcgcgg cgagcgggtt tacctttagc aacgcgtgga tgagctgggt gcgccaggcg 120

ccggggaaag ggctggagtg ggtgagctat attagcagca gcgggagcac catttattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgattattg gggcaggggg accctggtga ccgtgagcag c 351

<210> 325
<211> 339
<212> DNA
<213> Artificial Sequence

<220>
<223> C4.4a binder

<400> 325

cagagcgtgc tgacccagcc gccgagcgtg agcggggcgc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 326
<211> 351
<212> DNA
<213> Artificial Sequence

<220>
<223> C4.4a binder

<400> 326
gaggtgcagc tgctggagag cgggggggggg ctggtgcagc cgggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg ggggcagggg accctggtga ccgtgaccag c 351

<210> 327
<211> 339
<212> DNA
<213> Artificial Sequence

<220>
<223> C4.4a binder

<400> 327
cagagcgtgc tgacccagcc gccgagcgcg agcgggaccc cggggcagcg cgtgaccatt 60

agctgcaccg ggagcagcag caacattggg gcggggtatg tggtgcattg gtatcagcag 120

ctgccgggga ccgcgccgaa actgctgatt tatgataaca acaaacgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacgggccg 300

gtgtttgggg gggggaccaa actgaccgtg ctggggcag 339

<210> 328
<211> 351
<212> DNA
<213> Artificial Sequence

<220>

<223> C4.4a binder

<400> 328
gaggtgcagc tgctggagag cggggggggg ctggtgcagc cgggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcagggg accctggtga ccgtgagcag c 351

<210> 329
<211> 339
<212> DNA
<213> Artificial Sequence

<220>
<223> C4.4a

<400> 329
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtttg atgatagcct gaacggccg 300

gtgtttgggg gggggaccaa actgaccgtg ctggggcag 339

<210> 330
<211> 351
<212> DNA
<213> Artificial Sequence

<220>
<223> C4.4a binder

<400> 330
gaggtgcagc tgctggagag cggggggggg ctggtgcagc cgggggggag cctgcgcctg 60

agctgcgcgg cgagcgggtt tacctttagc agcgcgtgga tgagctgggt gcgccaggcg 120

ccggggaaag ggctggagtg ggtgagctat attagcagca gcgggagcag cacctattat 180

gcggatagcg tgaaagggcg ctttaccatt agccgcgata acagcaaaaa caccctgtat 240

ctgcagatga acagcctgcg cgcggaggat accgcggtgt attattgcgc gcgcgagggg 300

ctgtgggcgt ttgataaatg gggcagggg accctggtga ccgtgagcag c 351

<210> 331
<211> 339
<212> DNA

<213> Artificial Sequence

<220>
<223> C4.4a binder

<400> 331
cagagcgtgc tgacccagcc gccgagcgcg agcgggaccc cggggcagcg cgtgaccatt 60

agctgcagcg ggagcagcag caacattggg gcggggtatg tggtgcattg gtatcagcag 120

ctgccgggga ccgcgccgaa actgctgatt tatgataaca accagcgccc gagcggggtg 180

ccggatcgct ttagcgggag caaaagcggg accagcgcga gcctggcgat tagcgggctg 240

cgcagcgagg atgaggcgga ttattattgc gcggcgtttg atgatcgcct gagcgggccg 300

gtgtttgggg gggggaccaa actgaccgtg ctggggcag 339

<210> 332
<211> 366
<212> DNA
<213> Artificial Sequence

<220>
<223> C4.4a binder

<400> 332
gaggtgcagc tgctggagag cggggggggg ctggtgcagc cggggggggag cctgcgcctg 60

agctgcgcgg cgagcgggtt tacctttagc gattatcaga tgacctggat tcgccagacc 120

ccggggaaag ggctggagtg ggtgagcggg gtgagctgga acggggcgcg cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt atagcagcta ttattttaaa agctgggggc aggggaccct ggtgaccgtg 360

accagc 366

<210> 333
<211> 336
<212> DNA
<213> Artificial Sequence

<220>
<223> C4.4a binder

<400> 333
cagagcgtgc tgacccagcc gccgagcgcg agcgggaccc cggggcagcg cgtgaccatt 60

agctgcagcg ggagcagcag caacgtgggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atcgcctgaa cgggtggggg 300

tttgggggg ggaccaaact gaccgtgctg ggcag 336

<210> 334
<211> 363
<212> DNA
<213> Artificial Sequence

<220>
<223> C4.4a binder

<400> 334
gagcagctgc tggagagcgg ggggggctg gtgcagccgg gggggagcct gcgcctgagc 60

tgcgcggcga gcgggtttac ctttagcagc tatcagatga cctggattcg ccagaccccg 120

gggaaagggc tggagtgggt gagcgggatt agctggaacg gggggagcac ccattatgcg 180

gatagcgtga aagggcgctt taccattagc cgcgataaca gcaaaaacac cctgtatctg 240

cagatgaaca gcctgcgcgc ggaggatacc gcggtgtatt attgcgcgaa aggggattat 300

ctggtgtata gcgcgtatta ttttgatagc tgggggcagg ggaccctggt gaccgtgagc 360

agc 363

<210> 335
<211> 333
<212> DNA
<213> Artificial Sequence

<220>
<223> C4.4a binder

<400> 335
caggtgctga cccagccgcc gagcgcgagc gggaccccgg ggcagcgcgt gaccattagc 60

tgcagcggga gcagcagcaa cattgggagc aacccggtga actggtatca gcagctgccg 120

gggaccgcgc cgaaactgct gatttatcgc aacaaccagc gcccgagcgg ggtgccggat 180

cgctttagcg ggagcaaaag cgggaccagc gcgagcctgg cgattagcgg gctgcgcagc 240

gaggatgagg cggattatta ttgcgcggcg tgggatgatc gcctgaacgg gtgggggttt 300

ggggggggga ccaaactgac cgtgctgggg cag 333

<210> 336
<211> 366
<212> DNA
<213> Artificial Sequence

<220>
<223> C4.4a binder

<400> 336
gaggtgcagc tgctggagag cgggggggg ctggtgcagc cggggggag cctgcgcctg 60

agctgcgcgg cgagcgggtt tacctttagc gattatcaga tgacctggat tcgccagacc 120

ccggggaaag ggctggagtg ggtgagcggg attagctgga acggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt atagcagcta ttattttaaa tattgggggc aggggaccct ggtgaccgtg 360

agcagc 366

<210> 337
<211> 336
<212> DNA
<213> Artificial Sequence

<220>
<223> C4.4a binder

<400> 337
cagagcgtgc tgacccagcc gccgagcgcg agcgggaccc cggggcagcg cgtgaccatt 60

agctgcagcg ggagcagcag caacattggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atcgcctgaa cgggtgggcg 300

tttggggggg ggaccaaact gaccgtgctg ggcag 336

<210> 338
<211> 366
<212> DNA
<213> Artificial Sequence

<220>
<223> C4.4a binder

<400> 338
gaggtgcagc tgctggagag cggggggggg ctggtgcagc cgggggggag cctgcgcctg 60

agctgcgcgg cgagcgggtt tacctttagc gattatcaga tgacctggat tcgccagacc 120

ccggggaaag ggctggagtg ggtgagcggg attagctgga acgggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt atagcagcta ttattttaaa agctgggggc aggggaccct ggtgaccgtg 360

agcagc 366

<210> 339
<211> 336
<212> DNA
<213> Artificial Sequence

<220>
<223> C4.4a binder

<400> 339
cagagcgtgc tgacccagcc gccgagcgcg agcgggaccc cggggcagcg cgtgaccatt 60

agctgcagcg ggagcagcag caacattggg agcaaccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atagcctgaa cgggtggggg 300

tttggggggg ggaccaaact gaccgtgctg gggcag 336

<210> 340
<211> 366
<212> DNA
<213> Artificial Sequence

<220>
<223> C4.4a binder

<400> 340
gaggtgcagc tgctggagag cggggggggg ctggtgcagc cgggggggag cctgcgcctg 60

agctgcgcgg cgagcgggtt tacctttagc gattatcaga tgacctggat tcgccagacc 120

ccggggaaag ggctggagtg ggtgagcggg attagctgga acgggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt ataaaagcta ttattttaaa agctgggggc aggggaccct ggtgaccgtg 360

agcagc 366

<210> 341
<211> 336
<212> DNA
<213> Artificial Sequence

<220>
<223> C4.4a binder

<400> 341
cagagcgtgc tgacccagcc gccgagcgcg agcgggaccc cggggcagcg cgtgaccatt 60

agctgcagcg ggagcagcag caacattggg agcaaccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atcgcctgag cgggtggggg 300

tttggggggg ggaccaaact gaccgtgctg gggcag 336

<210> 342
<211> 366
<212> DNA

<213> Artificial Sequence

<220>
<223> C4.4a binder

<400> 342
gaggtgcagc tgctggagag cggggggggg ctggtgcagc cggggggggag cctgcgcctg 60

agctgcgcgg cgagcgggtt tacctttagc gattatcaga tgacctggat tcgccagacc 120

ccggggaaag ggctggagtg ggtgagcggg attagctgga acgggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt atagcagcta ttattttaaa agctgggggc aggggaccct ggtgaccgtg 360

agcagc 366

<210> 343
<211> 336
<212> DNA
<213> Artificial Sequence

<220>
<223> C4.4a binder

<400> 343
cagagcgtgc tgacccagcc gccgagcgcg agcgggaccc cggggcagcg cgtgaccatt 60

agctgcagcg ggagcagcag caacattggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atcgcctgag cgggtggggg 300

tttggggggg ggaccaaact gaccgtgctg gggcag 336

<210> 344
<211> 366
<212> DNA
<213> Artificial Sequence

<220>
<223> C4.4a binder

<400> 344
gaggtgcagc tgctggagag cggggggggg ctggtgcagc cggggggggag cctgcgcctg 60

agctgcgcgg cgagcgggtt tacctttagc gattatcaga tgacctggat tcgccagacc 120

ccggggaaag ggctggagtg ggtgagcggg attagctgga acgggggggag cacccattat 180

gcggatagcg tgaaagggcg ctttaccatt agccgcgata acagcaaaaa caccctgtat 240

ctgcagatga acagcctgcg cgcggaggat accgcggtgt attattgcgc gaaaggggat 300

tatctggtgt ataaaagcta ttattttaaa agctgggggc aggggaccct ggtgaccgtg 360

agcagc 366

<210> 345
<211> 336
<212> DNA
<213> Artificial Sequence

<220>
<223> C4.4a binder

<400> 345
cagagcgtgc tgacccagcc gccgagcgcg agcgggaccc cggggcagcg cgtgaccatt 60

agctgcagcg ggagcagcag caacattggg agcaacccgg tgaactggta tcagcagctg 120

ccggggaccg cgccgaaact gctgatttat cgcaacaacc agcgcccgag cggggtgccg 180

gatcgcttta gcgggagcaa aagcgggacc agcgcgagcc tggcgattag cgggctgcgc 240

agcgaggatg aggcggatta ttattgcgcg gcgtgggatg atagcctgaa cgggtggggg 300

tttggggggg ggaccaaact gaccgtgctg gggcag 336

## Description

The present invention provides recombinant antigen-binding regions and antibodies and functional fragments containing such antigen-binding regions that are specific for the membrane-anchored, 29 kDa polypeptide named C4.4a , which is over expressed in tumors.
Furthermore, it has a high abundance in metastases of these cancer types. The antibodies, accordingly, can be used to treat these and other disorders and conditions. Antibodies of the invention also can be used in the diagnostics field, as well as for further investigating the role of C4.4a in the progression of disorders associated with cancer. The invention also provides nucleic acid sequences encoding the foregoing antibodies, vectors containing the same, pharmaceutical compositions and kits with instructions for use.

## BACKGROUND OF THE INVENTION

Antibody-based therapy is proving very effective in the treatment of various cancers, including solid tumors. For example, HERCEPTIN® has been used successfully to treat breast cancer and RITUXAN® is effective in B-cell related cancer types. Central to the development of a successful antibody-based therapy is isolation of antibodies against cell-surface proteins found to be preferentially expressed on tumor cells. The C4.4a (gene name: LYPD3) polypeptide is a glycophosphatidylinositol (GPI)-anchored, highly glycosylated cell surface protein. Rat C4.4a was first described as a metastasis-associated, cell surface protein in metastasizing rat pancreatic tumor cells (Rösel M. et al., Oncogene 1998,17(15):1989-2002). Human C4.4a was cloned form a placental cDNA library (Würfel, J. et. al. Gene 2001,262:35-41). C4.4a displays structural homology to the uPAR receptor and contains two LY6 domains, exhibiting the typical three finger protein fold (Jacobsen B. & Ploug M., Current Medicinal Chemistry 2008, 15:2559-2573). The protein is highly glycosylated and contains 6 predicted N-glycosylation sites and several O-glycosylation sites. Furthermore C4.4a contains in total 9 disulfide bridges located in the two Ly6 domains (Hansen L. et al., Biochem J. 2004, 380:845-857). C4.4a shows a strong expression in tumor cells like lung cancer, colorectal cancer, breast cancer, Cervix cancer, pancreatic cancer, renal cancer, Head and Neck cancer and melanomas. Northern blot analysis demonstrated C4.4a expression in ~ 50 % of primary lung tumors and ~75 % of lung tumor metastases, while expression in non-diseased lung tissue was undetectable (Würfel J. et. al., Gene 2001, 262:35-41). In non-small cell lung cancer C4.4a can be used as a prognostic marker. Here clinical data clearly show that high C4.4a expression correlates with poor prognosis (Hansen L. et al., Lung Cancer 2007, 58:260-266). In melanoma detailed expression analysis revealed that C4.4a is not expressed in melanocytes and nevi but is expressed in ~ 60 % of primary malignant melanomas and in 100 % of lymph node and skin metastases (Seiter S. et al., J Invest Dermatol. 2001, 116(2):344-347). Furthermore up regulation of C4.4a gene expression was found in breast cancer tissue compared to matched adjacent normal breast tissue (Fletcher G.C., Br. J. Cancer 2003, 88(4):579-585), in various breast cancer cell lines and in urothelial cancer compared to normal urothelium (Smith B. A. et al., Cancer Res 2001, 61(4):1678-1685). C4.4a expression was demonstrated by FACS with a polyclonal antibody in various tumor cell lines of colorectal cancer, pancreatic cancer, breast cancer and prostate cancer. In IHC studies of colorectal cancer, pancreatic cancer and breast cancer samples variable glycosylation of C4.4a on human tumor cell lines interferes with binding of these antibodies. Therefore, C4.4a has to be at least partially deglycosylated to allow for binding of these polyclonal antibodies. In colorectal cancer patients C4.4a expression is highly prevalent and C4.4a is shed from the cell surface, making it a prognostic serum tumor marker. Expression of C4.4a at invasive front is a novel prognostic marker for disease recurrence of colorectal cancer (K. Konishi et al., Cancer Science 2010) Diagnostic antibodies against soluble serum C4.4a have not been described (Paret C. et al., British Journal of Cancer 2007, 97:1146-1156). In normal tissue C4.4a expression is limited to skin keratinocytes, esophagus endothelial cells and placental cells (Würfel J. et. al., Gene 2001, 262:35-41), making it an ideal target for tumor therapy. WO01/23553 suggests the use of a C4.4a inhibitor (e.g. an anti-C4.4a antibody) which decreases or inhibits C4.4a expression or activity for the treatment of cancer.

The exact function of C4.a is unknown; however it is up regulated in migrating keratinocytes in wound healing (Hansen L. et al., Biochem J. 2004, 380:845-857). In light of metastasis association and structural homology to uPAR it is proposed that this molecule is involved in tumor cell invasion probably through interaction with the extra cellular matrix (Rösel M. et al., Oncogene 1998, 17(15):1989-2002; Paret C. et al., British Journal of Cancer 2007, 97:1146-1156). Potential ligands are Laminin1 and 5, Galectin 3 (Paret C., Int. J. Cancer 2005, 115:724-733) as well as agr2 and agr3 (Fletcher GC., Brit. J. Cancer 2003, 88:579-585).

The predictive value of xenograft murine cancer models for clinical outcome of immunotoxin cancer therapy is often limited by a lack of cross-reactivity of the therapeutic antibodies with their murine orthologues, which leads to reduced unspecific binding to normal tissue. On the other hand, neutralizing anti-mouse Fv antibodies which are formed in patients being treated with murine or chimeric antibodies may result in either dose-limiting toxicity or diminished therapeutic potency. Thus, to fully exploit the potential of specific C4.4a expression in cancer therapy, targeting antibodies are required which combine the advantages of high affinity C4.4a binding with a fully human or humanized antibody format, and with murine cross-reactivity.

A further necessary feature of novel antibodies is high affinity binding to different cancer cell lines expressing C4.4a on their surface. C4.4a is differently glycosylated on tumor cells (Paret C. et al., British Journal of Cancer 2007 97:1146-1156). Thus, effective anti-C4.4a antibodies must bind to an epitope presented by tumor cells from different patients, independently of individual variance including, but not restricted to, variances in glycosylation patterns, which leads to the expression of different forms of C4.4a.

Provided herein are antibodies, antigen-binding antibody fragments thereof, or variants thereof, that bind to C4.4a with high affinity, internalize efficiently, and that are preferably cross-reactive to C4.4a from another species. Also provided are antibody-based therapies for cancer, in particular for C4.4a expressing tumors, such as cancers of the breast, respiratory tract, brain, reproductive organs, digestive tract, urinary tract, eye, liver, skin, head and neck, thyroid, parathyroid, and their distant metastases and also including lymphomas, sarcomas and leukemias. These Therapies are using antibodies, antigen-binding antibody fragments thereof, or variants thereof, that facilitate delivery of therapeutically active agents to cancer cells.

## SUMMARY OF THE INVENTION

It is an object of the invention to provide antibodies, or antigen-binding antibody fragments thereof, or variants thereof, that are highly selective for the C4.4a polypeptide either GPI-anchored onto the cell surface or soluble, by removal of the GPI-portion, in patients' serum and which may be employed in methods for detection of C4.4a expression, which is associated with disease states such as cancer of the lung, colon, breast, cervix, pancreas, kidney, Head and Neck or melanomas, and in the treatment of such disease states. Toward these ends, it is an object of the invention to provide isolated human, humanized or chimeric antibodies, or antigen binding antibody fragments thereof, that specifically bind to a C4.4a epitope which is present in different forms of the mature human C4.4a polypeptide of 278 amino acids (SEQ ID 1), which is presented by C4.4a expressing cancer cell lines, and/or which is bound by these antibodies with high affinities. As used herein, different 'forms' of C4.4a include, but are not restricted to, different glycoforms, different isoforms or C4.4a polypeptides which undergo different translational and posttranslational modifications. It is another object of the invention to provide antibodies, or antigen-binding antibody fragments thereof, or variants thereof that are safe for human administration.

It is another object of the invention to provide antibodies, or antigen-binding antibody fragments thereof, or variants thereof, which bind to human C4.4a and are cross-reactive to C4.4a of another species. Preferably said other species is a rodent, such as for example mouse or rat. Most preferably the antibodies, or antigen-binding antibody fragments thereof, or variants thereof bind to human C4.4a and are cross-reactive to murine C4.4a.

It is another object of the invention to provide antibodies, or antigen-binding antibody fragments thereof, or variants thereof, which bind to a broad range of different C4.4a-expressing cell lines. It is another object of the invention to provide antibodies or variants thereof, which bind to different C4.4a-expressing cancer cells or tumor cells and elicit immune effector activity (e.g. ADCC or CDC) against C4.4a-expressing cancer cells, by using one or more antibodies or variants thereof, of the invention.

It is another object of the invention to provide antibodies, or antigen-binding antibody fragments thereof, or variants thereof, which are internalized efficiently following binding to a C4.4a expressing cell. An antibody of the invention might be co-administered with known medicaments, and in some instances the antibody might itself be modified. For example, an antibody could be conjugated to a cytotoxic agent, immunotoxin, toxophore or radioisotope to potentially further increase efficacy.

It is another object of the invention to provide antibodies which constitute a tool for diagnosis of malignant or dysplastic conditions in which C4.4a expression is elevated compared to normal tissue or where C4.4a is shed from the cell surface and becoming detectable in serum. Provided are anti-C4.4a antibodies conjugated to a detectable marker. Preferred markers are a radiolabel, an enzyme, a chromophore or a fluorescer.

The invention is also related to polynucleotides encoding the antibodies of the invention, or antigen-binding fragments thereof, cells expressing the antibodies of the invention, or antigen-binding fragments thereof, methods for producing the antibodies of the invention, or antigen-binding fragments thereof, methods for inhibiting the growth of dysplastic cells using the antibodies of the invention, or antigen-binding fragments thereof, and methods for treating and detecting cancer using the antibodies of the invention, or antigen-binding fragments thereof.

The invention provides antibodies that are distinguished from existing C4.4a antibodies (Paret C. et al., British Journal of Cancer 2007 97:1146-1156) in that they a) bind to native, cell surface expressed and fully glycosylated C4.4a, preferably to domain S1 of native, cell surface expressed and fully glycosylated C4.4a, b) are cross-reactive to murine C4.4a and c) internalize efficiently into C4.4a-expressing cells,. These and other objects of the invention are more fully described herein.

In one aspect, the invention provides an isolated antibody or antigen-binding fragment thereof that contains an antigen-binding region that binds specifically to native, cell surface expressed and fully glycosylated C4.4a, preferably binds specifically to domain S1 (amino acids 1-85 of C4.4a; SEQ ID NO: 1) of native, cell surface expressed and fully glycosylated C4.4a polypeptide. In another embodiment the antibodies or antigen-binding fragments are internalized into a C4.4a expressing cell upon binding of the antibody or antigen-binding fragment to the aforementioned cell. In a further preferred embodiment the antibodies or antigen-binding fragments compete in binding to C4.4a with the antibodies M31-B01 or M20-D02 S-A. In a further preferred embodiment the antibodies or antigen-binding fragments compete in binding to human C4.4a with the antibodies M31-B01 or M20-D02 S-A. In a further preferred embodiment the antibodies or antigen-binding fragments compete in binding to human and rodent C4.4a with the antibodies M31-B01 or M20-D02 S-A, a further preferred embodiment is wherein the rodent C4.4a is mouse C4.4a.

In a more preferred embodiment the antibody of the invention or antigen-binding fragment thereof comprises a heavy chain antigen-binding region that comprises SEQ ID NO:5 (H-CDR1), SEQ ID NO:9 (H-CDR2) and SEQ ID NO: 13 (H-CDR3) and comprises a light chain antigen-binding region that comprises SEQ ID NO:17 (L-CDR1), SEQ ID NO:21 (L-CDR2) and SEQ ID NO:25 (L-CDR3).

In more preferred embodiment the antibody of the invention or antigen-binding fragment thereof comprises a heavy chain antigen-binding region that comprises SEQ ID NO:6 (H-CDR1), SEQ ID NO:10 (H-CDR2) and SEQ ID NO:14 (H-CDR3) and comprises a light chain antigen-binding region that comprises SEQ ID NO:18 (L-CDR1), SEQ ID NO:22 (L-CDR2) and SEQ ID NO:26 (L-CDR3).

In a more preferred embodiment the antibody of the invention or antigen-binding fragment thereof comprises a heavy chain antigen-binding region that comprises SEQ ID NO:7 (H-CDR1), SEQ ID NO:11 (H-CDR2) and SEQ ID NO:15 (H-CDR3) and comprises a light chain antigen-binding region that comprises SEQ ID NO:19 (L-CDR1), SEQ ID NO:23 (L-CDR2) and SEQ ID NO:27 (L-CDR3).

In a more preferred embodiment the antibody of the invention or antigen-binding fragment thereof comprises a heavy chain antigen-binding region that comprises SEQ ID NO:8 (H-CDR1), SEQ ID NO:12 (H-CDR2) and SEQ ID NO:16 (H-CDR3) and comprises a light chain antigen-binding region that comprises SEQ ID NO:20 (L-CDR1), SEQ ID NO:24 (L-CDR2) and SEQ ID NO:28 (L-CDR3).

In a more preferred embodiment the antibody of the invention or antigen-binding fragment thereof comprises a heavy chain antigen-binding region that comprises SEQ ID NO:45 (H-CDR1), SEQ ID NO:46 (H-CDR2) and SEQ ID NO:47 (H-CDR3) and comprises a light chain antigen-binding region that comprises SEQ ID NO:48 (L-CDR1), SEQ ID NO:49 (L-CDR2) and SEQ ID NO:50 (L-CDR3).

In a more preferred embodiment the antibody of the invention or antigen-binding fragment thereof comprises a heavy chain antigen-binding region that comprises SEQ ID NO:55 (H-CDR1), SEQ ID NO:56 (H-CDR2) and SEQ ID NO:57 (H-CDR3) and comprises a light chain antigen-binding region that comprises SEQ ID NO:58 (L-CDR1), SEQ ID NO:59 (L-CDR2) and SEQ ID NO:60 (L-CDR3).

In a more preferred embodiment the antibody of the invention or antigen-binding fragment thereof comprises a heavy chain antigen-binding region that comprises SEQ ID NO:65 (H-CDR1), SEQ ID NO:66 (H-CDR2) and SEQ ID NO:67 (H-CDR3) and comprises a light chain antigen-binding region that comprises SEQ ID NO:68 (L-CDR1), SEQ ID NO:69 (L-CDR2) and SEQ ID NO:70 (L-CDR3).

In a further more preferred embodiment the antibody of the invention or antigen-binding fragment thereof comprises a heavy chain antigen-binding region that comprises SEQ ID NO:75 (H-CDR1), SEQ ID NO:76 (H-CDR2) and SEQ ID NO:77 (H-CDR3) and comprises a light chain antigen-binding region that comprises SEQ ID NO:78 (L-CDR1), SEQ ID NO:79 (L-CDR2) and SEQ ID NO:80 (L-CDR3).

In a more preferred embodiment the antibody of the invention or antigen-binding fragment thereof comprises a heavy chain antigen-binding region that comprises SEQ ID NO:85 (H-CDR1), SEQ ID NO:86 (H-CDR2) and SEQ ID NO:87 (H-CDR3) and comprises a light chain antigen-binding region that comprises SEQ ID NO:88 (L-CDR1), SEQ ID NO:89 (L-CDR2) and SEQ ID NO:90 (L-CDR3).

In a more preferred embodiment the antibody of the invention or antigen-binding fragment thereof comprises a heavy chain antigen-binding region that comprises SEQ ID NO:95 (H-CDR1), SEQ ID NO:96 (H-CDR2) and SEQ ID NO:97 (H-CDR3) and comprises a light chain antigen-binding region that comprises SEQ ID NO:98 (L-CDR1), SEQ ID NO:99 (L-CDR2) and SEQ ID NO:100 (L-CDR3).

In a more preferred embodiment the antibody of the invention or antigen-binding fragment thereof comprises a heavy chain antigen-binding region that comprises SEQ ID NO:105 (H-CDR1), SEQ ID NO:106 (H-CDR2) and SEQ ID NO:107 (H-CDR3) and comprises a light chain antigen-binding region that comprises SEQ ID NO:108 (L-CDR1), SEQ ID NO:109 (L-CDR2) and SEQ ID NO:110 (L-CDR3).

In a more preferred embodiment the antibody of the invention or antigen-binding fragment thereof comprises a heavy chain antigen-binding region that comprises SEQ ID NO:115 (H-CDR1), SEQ ID NO:116 (H-CDR2) and SEQ ID NO: 117 (H-CDR3) and comprises a light chain antigen-binding region that comprises SEQ ID NO:118 (L-CDR1), SEQ ID NO:119 (L-CDR2) and SEQ ID NO:120 (L-CDR3).

In a more preferred embodiment the antibody of the invention or antigen-binding fragment thereof comprises a heavy chain antigen-binding region that comprises SEQ ID NO:125 (H-CDR1), SEQ ID NO:126 (H-CDR2) and SEQ ID NO: 127 (H-CDR3) and comprises a light chain antigen-binding region that comprises SEQ ID NO:128 (L-CDR1), SEQ ID NO:129 (L-CDR2) and SEQ ID NO:130 (L-CDR3).

In a more preferred embodiment the antibody of the invention or antigen-binding fragment thereof comprises a heavy chain antigen-binding region that comprises SEQ ID NO:135 (H-CDR1), SEQ ID NO:136 (H-CDR2) and SEQ ID NO:137 (H-CDR3) and comprises a light chain antigen-binding region that comprises SEQ ID NO:138 (L-CDR1), SEQ ID NO:139 (L-CDR2) and SEQ ID NO:140(L-CDR3).

An antibody of the invention may be an IgG (e.g., $IgG_1$ $IgG_2$, $IgG_3IgG_4$), while an antibody fragment may be a Fab, Fab', $F(ab')_2$ or scFv, for example. An inventive antibody fragment, accordingly, may be, or may contain, an antigen-binding region that behaves in one or more ways as described herein.

The invention also is related to isolated nucleic acid sequences, each of which can encode an aforementioned antibody or antigen-binding fragment thereof that is specific for an epitope of C4.4a. Nucleic acids of the invention are suitable for recombinant production of antibodies or antigen-binding antibody fragments. Thus, the invention also relates to vectors and host cells containing a nucleic acid sequence of the invention.

Compositions of the invention may be used for therapeutic or prophylactic applications. The invention, therefore, includes a pharmaceutical composition comprising an inventive antibody (or antigen-binding fragment thereof) and a pharmaceutically acceptable carrier or excipient therefore. In a related aspect, the invention provides a method for treating a disorder or condition associated with the undesired presence of C4.4a expressing cells. In a preferred embodiment the aforementioned disorder is cancer. Such method contains the steps of administering to a subject in need thereof an effective amount of the pharmaceutical composition that contains an inventive antibody as described or contemplated herein.

The invention also provides instructions for using an antibody library to isolate one or more members of such library that binds specifically to C4.4a.

## DESCRIPTION OF THE FIGURES

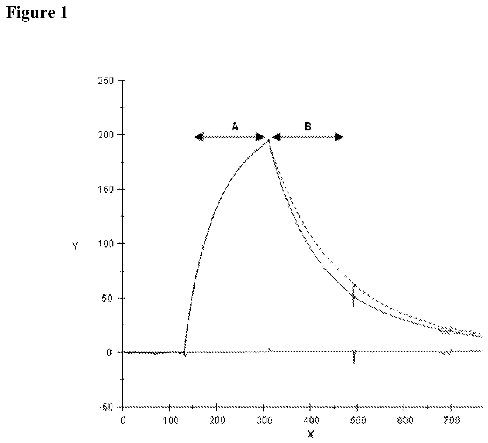

Figure 1 shows the result of an Epitope grouping experiment performed using sandwich surface plasmon resonance analysis. Y is resonance units X is time in seconds. One of the antibodies of the invention was coated to the chip. A indicates the time C4.4a was added. B indicates the time the other antibody of the invention was added. Both antibodies were unable to bind simultaneously to C4.4a indicating an at least overlapping epitope.

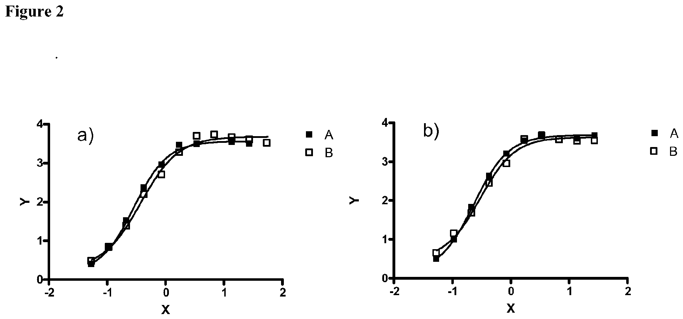

Figure 2 provides data on binding of anti C4.4a antibodies of the invention to recombinant human (a) and mouse (b) C4.4a in human IgG1 format. $EC_{50}$ values were determined by ELISA as described in Example 4. M31-B01 (A) is binding with an $EC_{50}$ of 0.24 and 0.29 nM to human and murine C4.4a respectively. M20 B02 S-A (B) is binding with an $EC_{50}$ of 0.3 and 0.38 nM to human and murine C4.4a respectively. X is log nM ; Y is Extinction at 360 nm.

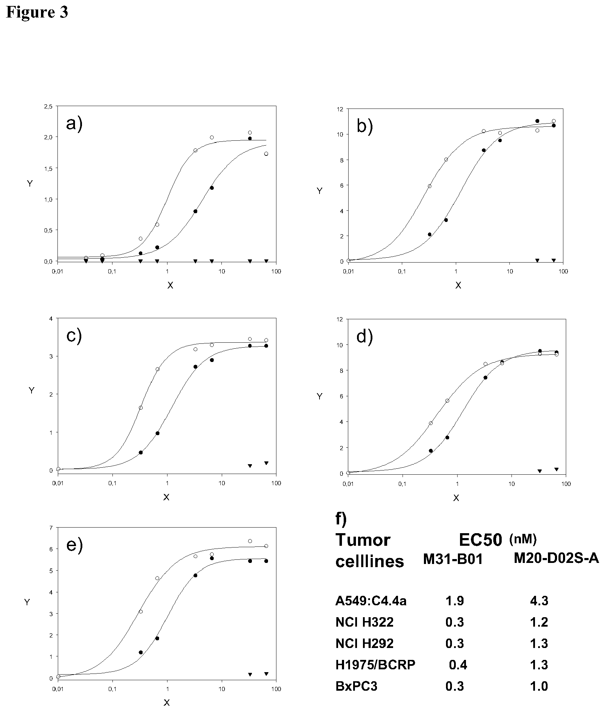

Figure 3 provides data on specific binding of antibodies of the invention to different tumor cell lines, either transfected with hC4.4a like A549:hC4.4a (a) or natively C4.4a expressing tumor cell lines as, NCI H322 (b) NCI H292 (c), H1975/BCRP (d) or BxPC3 (e). $EC_{50}$ values for binding of M31-B01 (open circles) and M20-D02 S-A (closed circles) are summarized in (f). The IgG1 isotype control (closed triangles) did not show any binding to the cells. All data was generated by FACS titration. X is concentration (log nM) and Y is geomean fluorescence ($x10^3$).

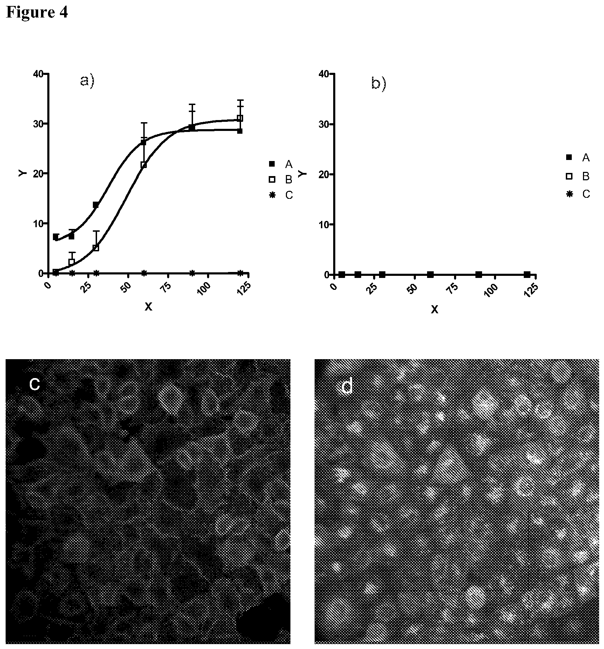

Figure 4(a) provides data on specific internalization of Fluorophor labeled-anti-C4.4a antibodies into A549:hC4.4a cells. Figure 4 (b) provides data on non transfected A549 cells as negative control. A is M31-B01 (closed squares); B is M20-D02 S-A (open squares); C is an isotype control for hIgG1 (stars); X is time in minutes and Y is the granule count/cell [$*10^3$]. Both C4.4a antibodies are internalized specifically, efficiently and rapidly into C4.4a bearing tumor cells.

Fig 4 (c) and (d) provide data on internalization of M31-B01 into A549:hC4.4a cells. After 5 minutes (c) only a weak staining of the cell surface can be observed. After 90 min (d) intensively coloured endosomes containing the internalized M31-B01 antibody, which is carrying the pH dependent fluorescence dye, are clearly visible and indicate effective internalization.

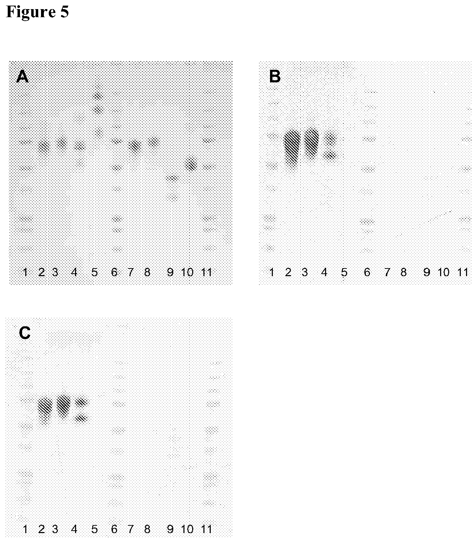

Figure 5 provides data on the epitope M31-B01 and M20-D02 S-A are binding to as determined by western blotting. A) shows a Coomassie 250 stained SDS-PAGE of different C4.4a species (lanes 2+7 hC4.4a , lanes 3+8 mC4.4a, lanes 4+9 hC4.4a domain S1-Fc-his6, lanes 5+10 hC4.4a domain S2-Fc-his6; lanes 1, 6 and 11 contain molecular weight markers; lanes 2-5 contain non reduced samples; lanes 6-10 contain reduced samples) B) and C) show the respective western blots with the identical sample load as in A). B) was incubated with M31-B01 and C) was incubated with M20-D02 S-A as detection antibody. Both antibodies show specific reduction dependent staining indicating a conformational epitope. However both bind to human and mouse full length C4.4a and to human C4.4a domain S1 but not to human C4.4a domain S2.

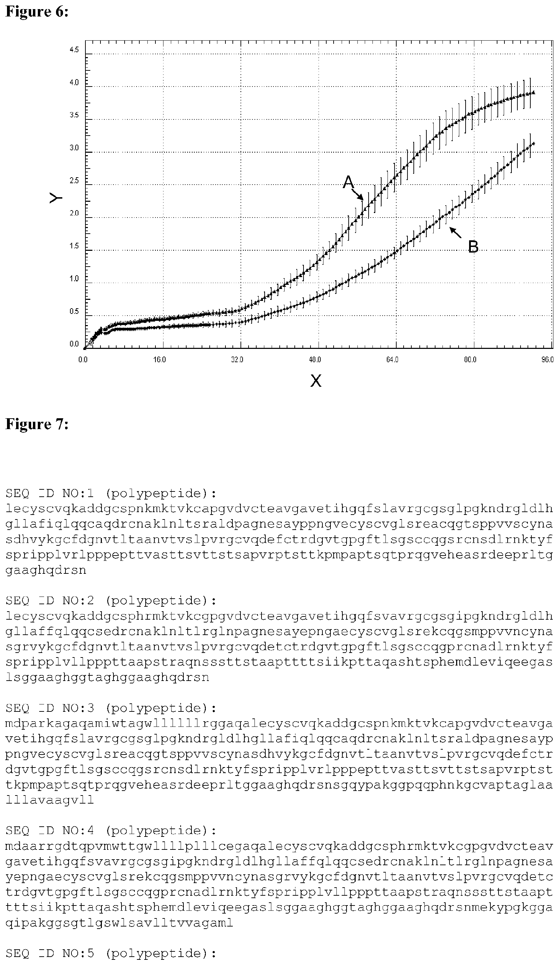

Figure 6 provides data on the inhibition of tumor cell proliferation in vitro by an antibody of the invention (B01-3) determined by measurement with an xCELLigence analyzer. A549:hC4.4a cells were either co-incubated with 100 nM of an non-binding hIgG1 isotype control antibody (A) or 100 nM of B01-3 (B) in single wells of an E-plate for the time indicated under conditions described in example 15. X is time in hours, Y is the relative rate of cell proliferation. A549:hC4.4a cells incubated with B01-3 show decreased cell proliferation relative to the control IgG1.

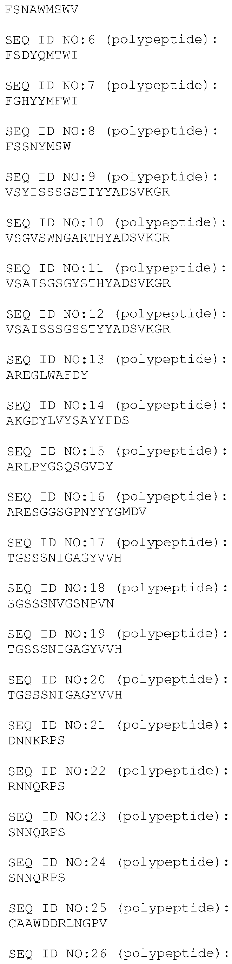

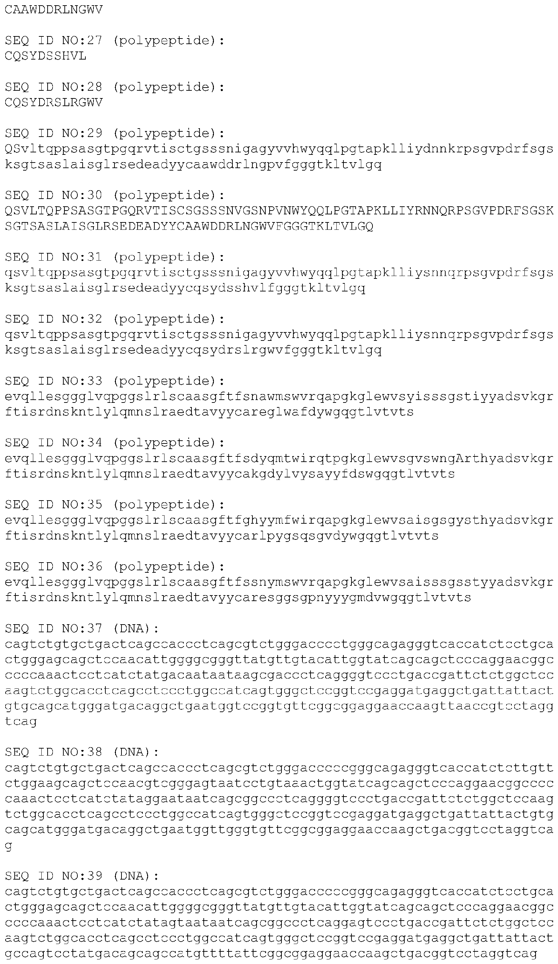

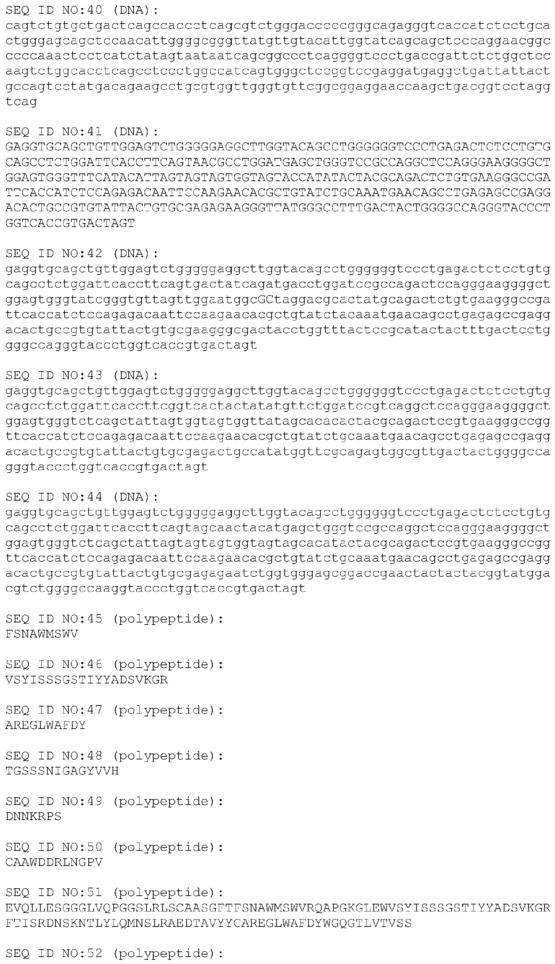

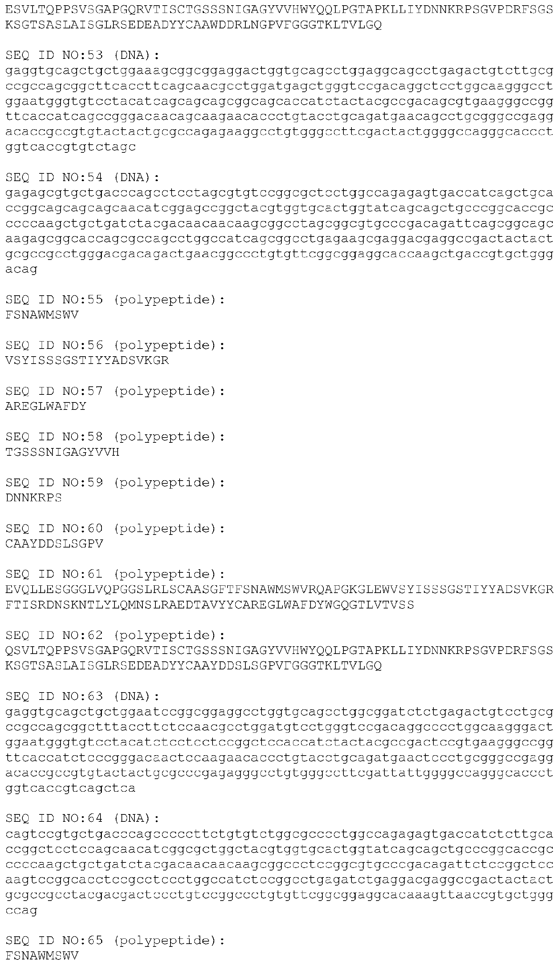

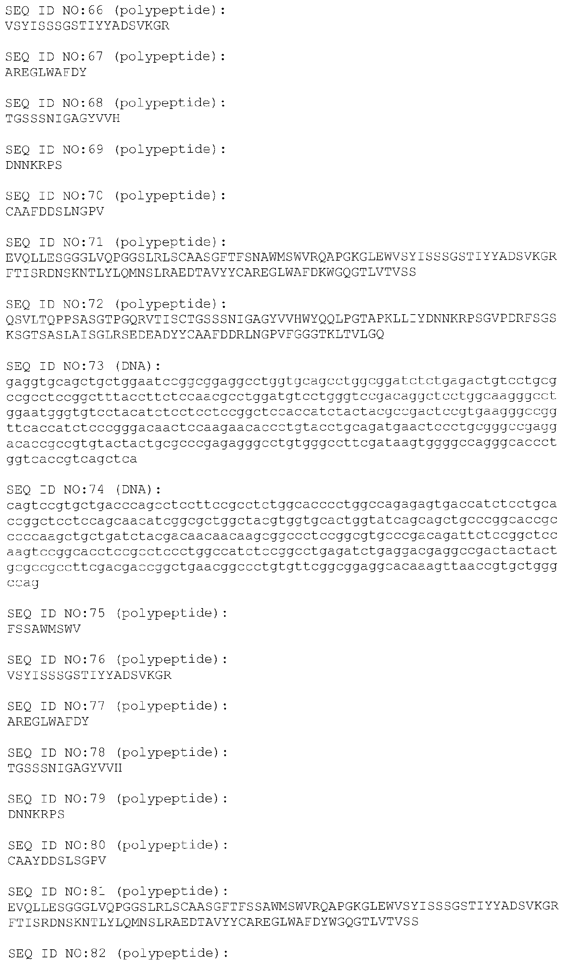

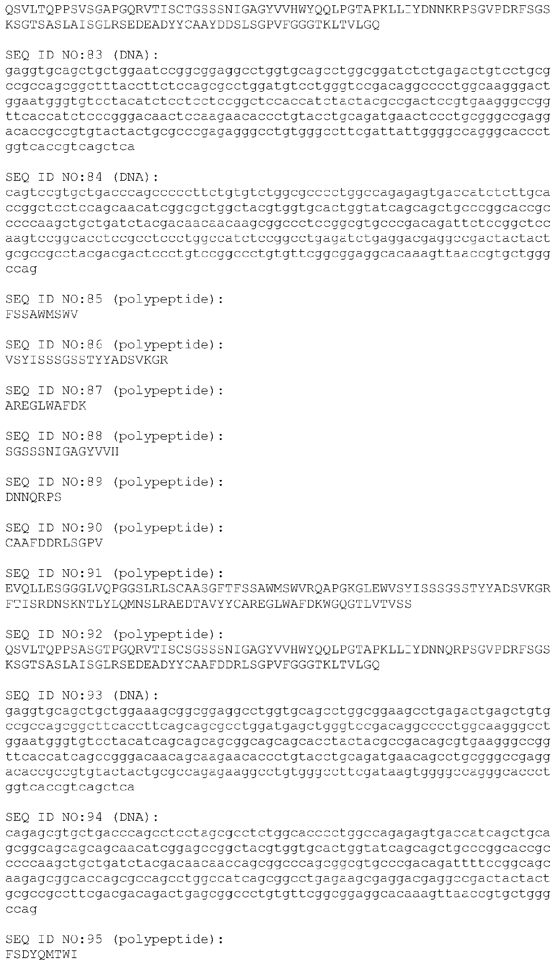

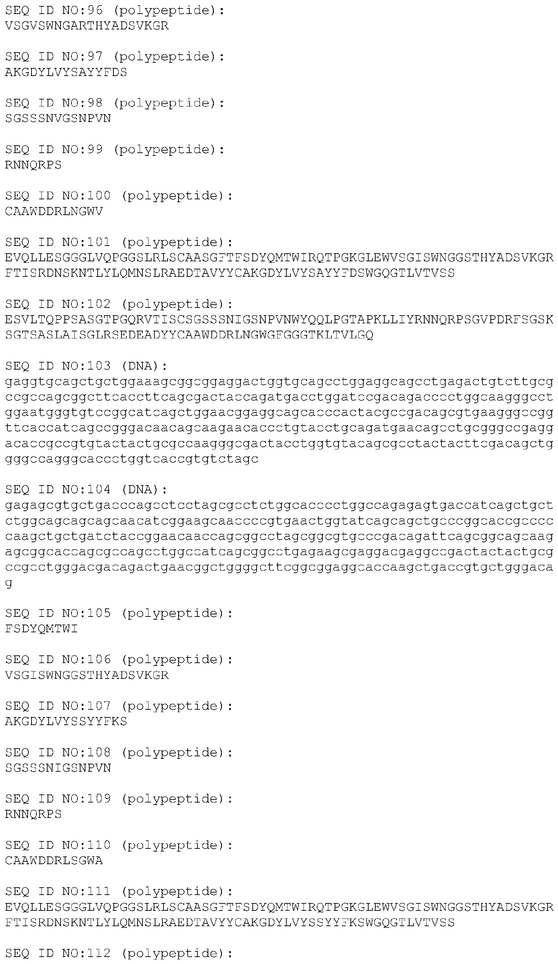

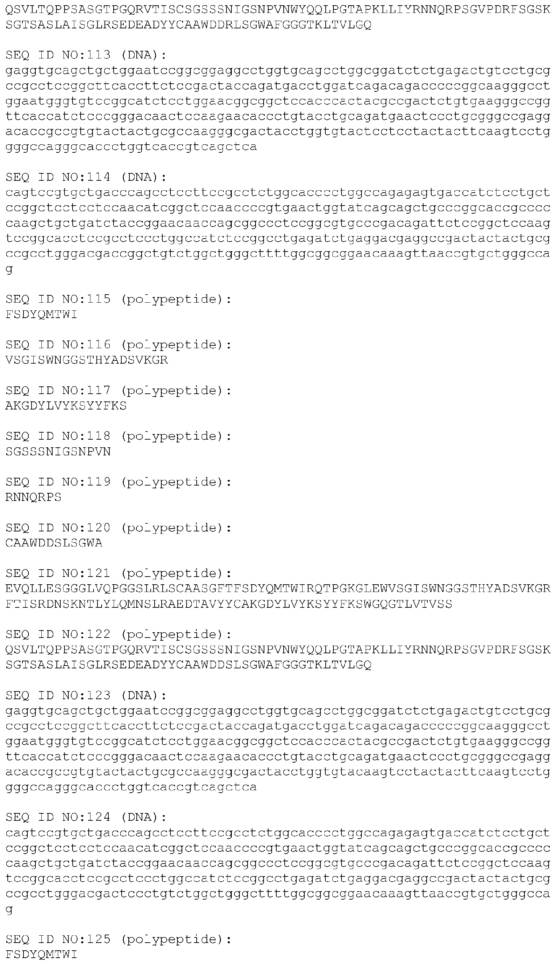

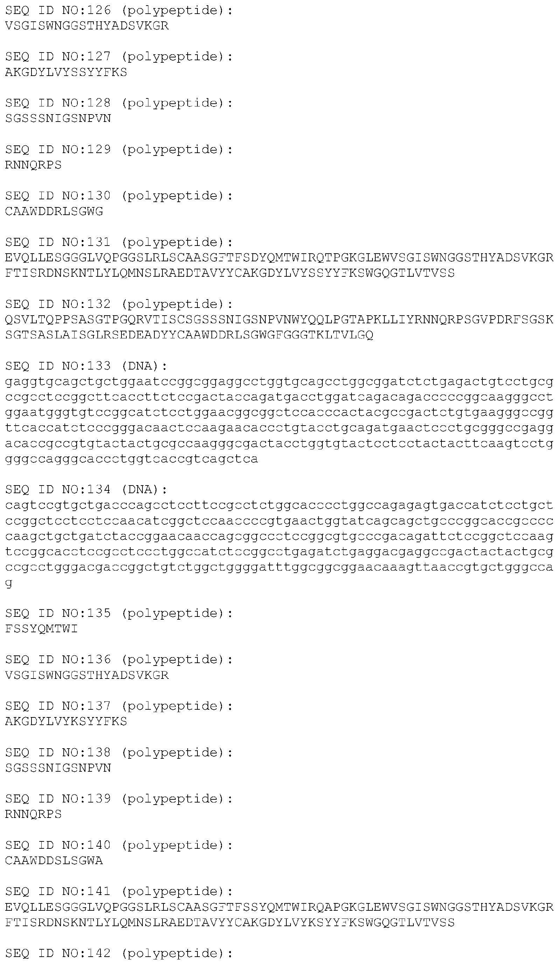

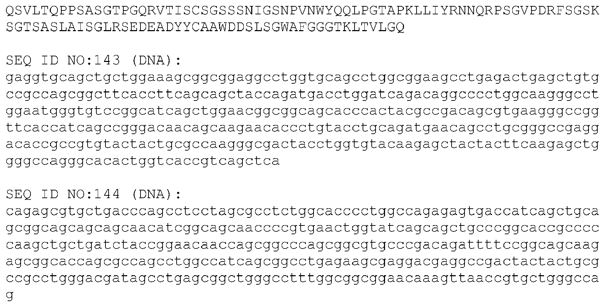

Figure 7 shows sequences of the invention.

## DETAILED DESCRIPTION OF THE INVENTION

The present invention is based on the discovery of novel antibodies that are specific to or have a high affinity for C4.4a and can deliver a therapeutic benefit to a subject. The antibodies of the invention, which may be human, humanized or chimeric, can be used in many contexts, which are more fully described herein.

### Definitions

A "human" antibody or antigen-binding fragment thereof is hereby defined as one that is not chimeric (*e.g.*, not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, *in silico* from synthetic sequences that are based on the analysis of known human antibody sequences. *In silico* design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (*e.g.*., such library being based on antibodies taken from a human natural source). Examples of human antibodies include antibodies as described in Söderlind et al., Nature Biotech. 2000, 18:853-856.

A "humanized antibody" or humanized antigen-binding fragment thereof is defined herein as one that is (i) derived from a non-human source (*e.g.,* a transgenic mouse which bears a heterologous immune system), which antibody is based on a human germline sequence; (ii) where amino acids of the framework regions of a non human antibody are partially exchanged to human amino acid sequences by genetic engineering or (iii) CDR-grafted, wherein the CDRs of the variable domain are from a non-human origin, while one or more frameworks of the variable domain are of human origin and the constant domain (if any) is of human origin.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

As used herein, an antibody "binds specifically to", is "specific to/for" or "specifically recognizes" an antigen of interest, e.g. a tumor-associated polypeptide antigen target (here, C4.4a), is one that binds the antigen with sufficient affinity such that the antibody is useful as a therapeutic agent in targeting a cell or tissue expressing the antigen, and does not significantly cross-react with other proteins or does not significantly cross-react with proteins other than orthologs and variants (e.g. mutant forms, splice variants, or proteolytically truncated forms) of the aforementioned antigen target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent $K_D$ for the antigen of less than about $10^{-4}$ M, alternatively less than about $10^{-5}$ M, alternatively less than about $10^{-6}$ M, alternatively less than about $10^{-7}$ M, alternatively less than about $10^{-8}$ M, alternatively less than about $10^{-9}$ M, alternatively less than about $10^{-10}$ M, alternatively less than about $10^{-11}$ M, alter-

natively less than about $10^{-12}$ M, or less. An antibody "binds specifically to," is "specific to/for" or "specifically recognizes" an antigen if such antibody is able to discriminate between such antigen and one or more reference antigen(s). In its most general form, "specific binding". "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to Western blots, ELISA-, RIA-, ECL-, IRMA-tests and peptide scans. For example, a standard ELISA assay can be carried out. The scoring may be carried out by standard color development (e.g. secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" refers to the strength of the sum total of noncovalent interactions between a single binding site of a molecule and its binding partner. Unless indicated otherwise, as used herein, "binding affinity" refers to intrinsic binding affinity which reflects a 1 : 1 interaction between members of a binding pair (e.g. an antibody and an antigen). The dissociation constant "$K_D$" is commonly used to describe the affinity between a molecule (such as an antibody) and its binding partner (such as an antigen) i.e. how tightly a ligand binds to a particular protein. Ligand-protein affinities are influenced by non-covalent intermolecular interactions between the two molecules Affinity can be measured by common methods known in the art, including those described herein. In one embodiment, the "$K_D$" or "$K_D$ value" according to this invention is measured by using surface plasmon resonance assays using a Biacore T100 instrument (GE Healthcare Biacore, Inc.) according to Example 3. In brief, antibodies were immobilized onto a CM5 sensor chip through an indirect capturing reagent, anti-human IgG Fc. Reagents from the "Human Antibody Capture Kit" (BR-1008-39, GE Healthcare Biacore, Inc.) were used as described by the manufacturer. Approximately 5000 resonance units (RU) monoclonal mouse anti-human IgG (Fc) antibody were immobilized per cell. Anti C4.4 antibodies were injected to reach a capturing level of approximately 200 to 600 RU. Various concentrations of human or murine C4.4a were injected over immobilized anti-C4.4a antibodies. Sensograms were generated after in-line reference cell correction followed by buffer sample subtraction. The dissociation equilibrium constant ($K_D$) was calculated based on the ratio of association ($k_{on}$) and dissociation rated ($k_{off}$) constants, obtained by fitting sensograms with a first order 1:1 binding model using Biacore Evaluation Software. Other suitable devices are BIACORE(R)-2000, a BIACORE (R)-3000 (BIAcore, Inc., Piscataway, NJ), or ProteOn XPR36 instrument (Bio-Rad Laboratories, Inc.).

The term "antibody", as used herein, is intended to refer to immunglobulin molecules, preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g. three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs. arranged from amino terminus to carboxy-terminus e.g. in the following order: FR1, CDR1, FR2, CDR2, FR3, CDR3, FR4.

As used herein, the term "Complementarity Determining Regions (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immulological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop. Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA, and IgA2. The heavy-chain constant domains that correspond to the different classes of antibodies are called [alpha], [delta], [epsilon], [gamma], and [mu], respectively. The subunit structures and three-dimensional configurations of different classes of immunglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region.

An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320). A preferred class of immunoglobulins for use in the present invention is IgG.
"Functional fragments" or "antigen-binding antibody fragments" of the invention include Fab, Fab', $F(ab')_2$, and Fv fragments; diabodies; single domain antibodies (DAbs), linear antibodies; single-chain antibody molecules (scFv); and multispecific, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multispecific" or "multi-functional" antibody is understood to have each of its binding sites identical. The $F(ab')_2$ or Fab may be engineered to minimize or completely remove the intermolecular disulphide interactions that occur between the $C_{H1}$ and $C_L$ domains.

An antibody of the invention may be derived from a recombinant antibody library that is based on amino acid sequences that have been isolated from the antibodies of a large number of healthy volunteers. Using the n-CoDeR® technology the fully human CDRs are recombined into new antibody molecules. The unique recombination process allows the library to contain a wider variety of antibodies than could have been created naturally by the human immune system.

As used herein, different 'forms' of antigen, e.g. C4.4a are hereby defined as different protein molecules resulting from different translational and posttranslational modifications, such as, but not limited to, differences in splicing of the primary C4.4a transcript, differences in glycosylation, and differences in posttranslational proteolytic cleavage.
As used herein, the term 'epitope' includes any protein determinant capable of specific binding to an immunoglobulin or T-cell receptors. Epitopic determinants usually consist of chemically active surface groupings of molecules such as amino acids or sugar side chains, or combinations thereof and usually have specific three dimensional structural characteristics, as well as specific charge characteristics. Two antibodies are said to 'bind the same epitope' if one antibody is shown to compete with the second antibody in a competitive binding assay, by any of the methods well known to those of skill in the art.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or nonproteinaceous solutes. In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or nonreducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

"Antibody-dependent cell-mediated cytotoxicity" or "ADCC" refers to a form of cytotoxicity in which secreted Ig bound onto Fc gamma receptors (FcγRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 B1 and WO 1999/51642.

The term immunoconjugate (interchangeably referred to as "antibody-drug conjugate," or "ADC") refers to an antibody conjugated to one or more cytotoxic agents, such as a chemotherapeutic agent, a drug, a growth inhibitory agent, a toxin (e.g., a protein toxin, a enzymatically active toxin of bacterial, fungal, plant, or animal origin, or fragments thereof), or a radioactive isotope (i.e., a radioconjugate). Immunoconjugates have been used for the local delivery of cytotoxic agents, i.e., drugs that kill or inhibit the growth or proliferation of cells, in the treatment of cancer (e.g. Liu et al., Proc Natl. Acad. Sci. (1996), 93, 8618-8623)). Immunoconjugates allow for the targeted delivery of a drug moiety to a tumor, and intracellular accumulation therein, where systemic administration of unconjugated drugs may result in unacceptable levels of toxicity to normal cells and/or tissues. Toxins used in antibody-toxin conjugates include bacterial toxins such as diphtheria toxin, plant toxins such as ricin, small molecule toxins such as geldanamycin. The toxins may exert their cytotoxic effects by mechanisms including tubulin binding, DNA binding, or topoisomerase inhibition.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are ungapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

## Antibodies of the Invention

The present invention relates to methods to inhibit growth of C4.4a-positive cancer cells and the progression of neoplastic disease by providing anti-C4.4a antibodies. Provided are antibodies, antigen-binding antibody fragments thereof, and variants of the antibodies and fragments, that specifically bind to the 29 kDa, human C4.4a polypeptide, (SEQ ID NO: 1 or fragments thereof). In a preferred embodiment, the antibodies, antigen-binding fragments or variants thereof bind specifically to the extracellular domain S1 of the C4.4a polypeptide. The C4.4a polypeptide is named ‘C4.4a’ herein.

In another embodiment the antibodies or antigen-binding fragments thereof are internalized into a C4.4a expressing cell upon binding of the antibody or antigen-binding fragment thereof to the aforementioned cell. In a further preferred embodiment the antibodies or antigen-binding fragments thereof compete in binding to C4.4a with the antibodies M31-B01 or M20-D02 S-A. In a further preferred embodiment the antibodies or antigen-binding fragments thereof compete in binding to human C4.4a with the antibodies M31-B01 or M20-D02 S-A. In a further preferred embodiment the antibodies or antigen-binding fragments thereof compete in binding to human and rodent C4.4a with the antibodies M31-B01 or M20-D02 S-A, a further preferred embodiment is wherein the rodent C4.4a is mouse C4.4a.

In a further preferred embodiment the antibodies or antigen-binding fragments thereof comprise heavy or light chain CDR sequences which are at least 50%, 55%, 60% 70%, 80%, 90%, or 95% identical to at least one, preferably corresponding, CDR sequence as depicted in table 7, or which comprise variable heavy or light chain sequences which are at least 50%, 60%, 70%, 80%, 90%, 92% or 95% identical to a VH or VL sequence depicted in table 7, respectively.

In a further preferred embodiment the antibodies or antigen-binding fragments thereof comprise heavy and/or light chain CDR sequences which are at least 50%, 55%, 60% 70%, 80%, 90%, or 95% identical to at least one, preferably corresponding, CDR sequence of the antibodies M31-B01 or M20-D02 S-A, respectively.

In a further preferred embodiment the antibodies or antigen-binding fragments thereof comprise heavy and/or light chain CDR sequences which are at least 50%, 55%, 60% 70%, 80%, 90%, or 95% identical to the, preferably corresponding, heavy and/or light chain CDR sequences of the antibodies M31-B01 or M20-D02 S-A, respectively.

In a further preferred embodiment the antibodies or antigen-binding fragments thereof comprise heavy chain CDR2 and -3 sequences which are at least 50%, 55%, 60% 70%, 80%, 90%, or 95% identical to the heavy chain CDR2 and -3 sequences and light chain CDR1 and -3 sequences which are at least 50%, 55%, 60% 70%, 80%, 90%, or 95% identical to the light chain CDR1 and -3 sequences of the antibodies M31-B01. In a further preferred embodiment the antibodies or antigen-binding fragments thereof comprise heavy chain CDR2 and -3 sequences which are at least 50%, 55%, 60% 70%, 80%, 90%, or 95% identical to the heavy chain CDR2 and -3 sequences and light chain CDR1 and -3 sequences which are at least 50%, 55%, 60% 70%, 80%, 90%, or 95% identical to the light chain CDR1 and -3 sequences of the antibodies M20-D02 S-A.

In a further preferred embodiment the antibodies or antigen-binding fragments thereof comprise a variable heavy chain sequence which is at least 50%, 60%, 70%, 80%, 90%, 92% or 95% identical to a VH sequence disclosed in table 7 or table 4, preferably of the antibodies M31-B01 or M20-D02 S-A,. In a further preferred embodiment the antibodies or antigen-binding fragments thereof comprise a variable light chain sequence which is at least 50%, 60%, 70%, 80%, 90%, 92% or 95% identical to a VL sequence disclosed in table 7 or table 3, preferably of the antibodies M31-B01 or M20-D02 S-A.

In a further preferred embodiment the antibodies or antigen-binding fragments thereof comprise variable heavy and light chain sequences that are at least 50%, 60%, 70%, 80%, 90%, 92% or 95% identical to the VH and VL sequence of the antibodies M31-B01 or M20-D02 S-A, respectively.

In a further preferred embodiment the antibodies or antigen-binding fragments thereof comprise heavy and light chain CDR sequences which conform to the M31-B01 or M20-D02 S-A derived, preferably corresponding, CDR consensus sequences as depicted in table 15. A further preferred embodiment are antibodies or antigen-binding fragments thereof comprising heavy chain CDR sequences conforming to the corresponding heavy chain CDR sequences as represented by the consensus sequences SEQ ID NO: 297 (CDR H1), SEQ ID NO: 298 (CDR H2) and SEQ ID NO: 299 (CDR H3), and light chain CDR sequences conforming to the corresponding light chain CDR sequences as represented by the consensus sequences SEQ ID NO: 300 (CDR L1), SEQ ID NO: 22 (CDR L2) and SEQ ID NO: 301 (CDR L3), or comprising heavy chain CDR sequences conforming to the corresponding heavy chain CDR sequences as represented by the consensus sequences SEQ ID NO: 302 (CDR H1), SEQ ID NO: 303 (CDR H2) and SEQ ID NO: 304 (CDR H3), and light chain CDR sequences conforming to the corresponding light chain CDR sequences as represented by the consensus sequences SEQ ID NO: 305 (CDR L1), SEQ ID NO: 306 (CDR L2) and SEQ ID NO: 307 (CDR L3).

In a further preferred embodiment the antibodies or antigen-binding antibody fragments comprise at least one, preferably corresponding, heavy and/or light chain CDR sequence as disclosed in table 7 or table 3 and 4, or preferably of an antibody as depicted in table 7 or table 3 and 4. In a further preferred embodiment the antibodies or antigen-binding antibody fragments comprise at least one, two, three, four, five or six, preferably corresponding, heavy and light chain CDR sequences as disclosed in table 7 or table 3 and 4, or preferably of an antibody as depicted in table 7 or table 3 and 4. In a further preferred embodiment the antibodies or antigen-binding antibody fragments comprise the heavy or light chain CDR1, CDR2 or CDR3 sequences of an antibody as depicted in table 7 or table 3 and 4, the heavy or light chain CDR1 and CDR2 sequences of an antibody as depicted in table 7 or table 3 and 4, the heavy or light chain CDR1 and CDR3 sequences of an antibody as depicted in table 7 or table 3 and 4, the heavy or light chain CDR2 and CDR3 sequences of an antibody as depicted in table or table 3 and 4, the heavy or light chain CDR1, CDR2 and CDR3 sequences of an antibody as depicted in table or table 3 and 4. In a further preferred embodiment the antibodies or antigen-binding antibody fragments comprise the heavy chain CDR sequences CDR1 and CDR2 and the light chain CDR sequences CDR1, CDR2, CDR3 of an antibody as depicted in table 7 or table 3 and 4. In a further preferred embodiment the antibodies or antigen-binding antibody fragments comprise the heavy and light chain CDR1, CDR2 or CDR3 sequences of an antibody as depicted in table 7 or table 3 and 4, the heavy and light chain CDR1 and CDR2 sequences of an antibody as depicted in table or table 3 and 4, the heavy and light chain CDR1 and CDR3 sequences of an antibody as depicted in table 7 or table 3 and 4, the heavy and light chain CDR2 and CDR3 sequences of an antibody as depicted in table 7 or table 3 and 4, the heavy and light chain CDR1, CDR2 and CDR3 sequences of an antibody as depicted in table 7 or table 3 and 4. In a further preferred embodiment the antibodies or antigen-binding antibody fragments comprise the heavy and light chain CDR sequences of an antibody as depicted in table 7 or table 3 and 4.

In a further embodiment the antibodies or antigen-binding antibody fragments comprise a VH and/or VL sequence disclosed in table 7 or table 3 and 4,. In a further preferred embodiment the antibodies or antigen-binding antibody fragments comprise the VH and VL sequence of an antibody depicted in table 7 or table 3 and 4.

In a preferred embodiment the antibodies or antigen-binding antibody fragments of the invention are monoclonal. In a further preferred embodiment the antibodies or antigen-binding antibody fragments of the invention are human, humanized or chimeric.

Variants of the antibodies or antigen-binding antibody fragments contemplated in the invention are molecules in which the binding activity of the antibody or antigen-binding antibody fragment for C4.4a is maintained. In a preferred embodiment the variants compete in binding to C4.4a with an antibody depicted in table 7, preferably with antibody M31-B01 or M20-D02 S-A.

Throughout this document, reference is made to the following preferred antibodies of the invention: "M31-B01", "M20-D02 S-A", "M60-G03" and "M36-H02", "B01-3", "B01-5", "B01-7", "B01-10", "B01-12", "D02-4", "D02-6", "D02-7", "D02-11" and "D02-13".

M31-B01 represents an antibody comprising a variable heavy chain region corresponding to SEQ ID NO: 41 (DNA)/SEQ ID NO: 33 (protein) and a variable light chain region corresponding to SEQ ID NO: 37 (DNA)/SEQ ID NO: 29 (protein).

M20-D02 S-A represents an antibody comprising a variable heavy chain region corresponding to SEQ ID NO: 42 (DNA)/SEQ ID NO: 34 (protein) and a variable light chain region corresponding to SEQ ID NO: 38 (DNA)/SEQ ID NO: 30 (protein).

M60-DG03 represents an antibody comprising a variable heavy chain region corresponding to SEQ ID NO: 43 (DNA)/SEQ ID NO: 35 (protein) and a variable light chain region corresponding to SEQ ID NO: 39 (DNA)/SEQ ID NO: 31 (protein).

M36-H02 represents an antibody comprising a variable heavy chain region corresponding to SEQ ID NO: 44 (DNA)/SEQ ID NO: 36 (protein) and a variable light chain region corresponding to SEQ ID NO: 40 (DNA)/SEQ ID NO: 32 (protein).

B01-3 represents an antibody comprising a variable heavy chain region corresponding to SEQ ID NO: 53 (DNA)/SEQ ID NO: 51 (protein) and a variable light chain region corresponding to SEQ ID NO: 54 (DNA)/SEQ ID NO:

52 (protein).

B01-5 represents an antibody comprising a variable heavy chain region corresponding to SEQ ID NO: 63 (DNA)/SEQ ID NO: 61 (protein) and a variable light chain region corresponding to SEQ ID NO: 64 (DNA)/SEQ ID NO: 62 (protein).

B01-7 represents an antibody comprising a variable heavy chain region corresponding to SEQ ID NO: 73 (DNA)/SEQ ID NO: 71 (protein) and a variable light chain region corresponding to SEQ ID NO: 74 (DNA)/SEQ ID NO: 72 (protein).

B01-10 represents an antibody comprising a variable heavy chain region corresponding to SEQ ID NO: 83 (DNA)/SEQ ID NO: 81 (protein) and a variable light chain region corresponding to SEQ ID NO: 84 (DNA)/SEQ ID NO: 82 (protein).

B01-12 represents an antibody comprising a variable heavy chain region corresponding to SEQ ID NO: 93 (DNA)/SEQ ID NO: 91 (protein) and a variable light chain region corresponding to SEQ ID NO: 94 (DNA)/SEQ ID NO: 92 (protein).

D02-4 represents an antibody comprising a variable heavy chain region corresponding to SEQ ID NO: 103 (DNA)/SEQ ID NO: 101 (protein) and a variable light chain region corresponding to SEQ ID NO: 104 (DNA)/SEQ ID NO: 102 (protein).

D02-6 represents an antibody comprising a variable heavy chain region corresponding to SEQ ID NO: 113 (DNA)/SEQ ID NO: 111 (protein) and a variable light chain region corresponding to SEQ ID NO: 114 (DNA)/SEQ ID NO: 112 (protein).

D02-7 represents an antibody comprising a variable heavy chain region corresponding to SEQ ID NO: 123 (DNA)/SEQ ID NO: 121 (protein) and a variable light chain region corresponding to SEQ ID NO: 124 (DNA)/SEQ ID NO: 122 (protein).

D02-11 represents an antibody comprising a variable heavy chain region corresponding to SEQ ID NO: 133 (DNA)/SEQ ID NO: 131 (protein) and a variable light chain region corresponding to SEQ ID NO: 134 (DNA)/SEQ ID NO: 132 (protein).

D02-13 represents an antibody comprising a variable heavy chain region corresponding to SEQ ID NO: 143 (DNA)/SEQ ID NO: 141 (protein) and a variable light chain region corresponding to SEQ ID NO: 144 (DNA)/SEQ ID NO: 142 (protein).

In another aspect, the invention provides antibodies or antigen-binding fragments having an antigen-binding region that bind specifically to and/or has a high affinity for one or more regions of C4.4a, whose amino acid sequence is depicted by SEQ ID NO: 1. An antibody or antigen-binding fragment is said to have a "high affinity" for an antigen if the affinity measurement is less than 250 nM (monovalent affinity of the antibody or antigen-binding fragment). An inventive antibody or antigen-binding region preferably can bind to human C4.4a with an affinity of less than 250 nM, preferably less than 100 nM, more preferably less than 25 nM and even more preferable with less than 11 nM determined as monovalent affinity to human C4.4a. For instance, the affinity of an antibody of the invention against C4.4a may be about 220.0 nM or 1 nM (monovalent affinity of the antibody or antigen-binding fragment).

Table 1 provides a summary of dissociation constants of representative antibodies of the invention, as determined by surface plasmon resonance (Biacore) on directly immobilized human or murine C4.4a.

Table 1: Monovalent dissociation constants determined for anti-C4.4a IgG1 by surface plasmon resonance

| | Human C4.4a | Mouse C4.4a |
|---|---|---|
| Antibody (IgG1) | $K_D$ [M] | $K_D$ [M] |
| M31-B01 | *$7.0 \times 10^{-8}$* | *$1.3 \times 10^{-7}$* |
| M20-D02 S-A | *$2.2 \times 10^{-7}$* | *$1.8 \times 10^{-7}$* |
| B01-3 | *$6.0 \times 10^{-8}$* | *$1.2 \times 10^{-7}$* |
| B01-5 | *$4 \times 10^{-9}$* | *$1 \times 10^{-8}$* |
| B01-7 | *$7 \times 10^{-9}$* | *$9 \times 10^{-9}$* |
| B01-10 | *$4 \times 10^{-9}$* | *$1 \times 10^{-9}$* |
| B01-12 | *$1 \times 10^{-9}$* | *$2 \times 10^{-9}$* |
| D02-4 | *$2.9 \times 10^{-8}$* | *$5.6 \times 10^{-8}$* |
| D02-6 | *$6 \times 10^{-9}$* | *$2.1 \times 10^{-8}$* |
| D02-7 | *$9 \times 10^{-9}$* | *$2.2 \times 10^{-8}$* |

(continued)

| | Human C4.4a | Mouse C4.4a |
|---|---|---|
| D02-11 | *$1 \times 10^{-8}$* | *$2.4 \times 10^{-8}$* |
| D02-13 | *$2 \times 10^{-9}$* | *$4.2 \times 10^{-8}$* |

The IgG1 format was used for the cell-based affinity determination by fluorescence-activated cell sorting (FACS) combined with Scatchard analysis. figure 3 f) denotes the binding strength of representative IgG antibodies on transfected C4.4a-expressing A549 tumor cells and endogenously C4.4a expressing tumor cells. Table 8 provides a summary of the binding strength ($EC_{50}$) of representative IgG antibodies on transfected murine CHO-S:mC4.4a cells and endogenously C4.4a expressing NCI H292 tumor cells.

Table 8: $EC_{50}$ values determined for anti-C4.4a IgG1 by FACS

| | Mouse C4.4a:CHO | NCI H292 |
|---|---|---|
| Antibody (IgG1) | $EC_{50}$ [M] | $EC_{50}$ [M] |
| M31-B01 | 1.9 x *$10^{-9}$* | *$3 \times 10^{-10}$* |
| M20-D02 S-A | *$1.6 \times 10^{-9}$* | *$1.3 \times 10^{-9}$* |
| B01-3 | *$3.6 \times 10^{-10}$* | *$6 \times 10^{-11}$* |
| B01-5 | *$1.2 \times 10^{-9}$* | *$5 \times 10^{-11}$* |
| B01-7 | *$5.7 \times 10^{-10}$* | *$6 \times 10^{-11}$* |
| B01-10 | *$4.3 \times 10^{-10}$* | *$1 \times 10^{-10}$* |
| B01-12 | *$1.4 \times 10^{-9}$* | *Nd* |
| D02-6 | *$1 \times 10^{-9}$* | *$2 \times 10^{-11}$* |
| D02-7 | *$7.8 \times 10^{-10}$* | *$3 \times 10^{-11}$* |
| D02-11 | *$1.7 \times 10^{-9}$* | *$1 \times 10^{-11}$* |
| D02-13 | *$2 \times 10^{-9}$* | *$1.2 \times 10^{-10}$* |
| nd= not determined | | |

An IgG1 is said to have a "high affinity" for an antigen if the affinity measurement measured by FACS is less than 100 nM (apparent affinity of IgG). An inventive bivalent antibody or antigen-binding fragment preferably can bind to human C4.4a with an affinity of less than 100 nM, more preferably less than 50 nM, and still more preferably less than 10 nM. Further preferred are bivalent antibodies that bind to C4.4a with an affinity of less than 5 nM, and more preferably less than 1 nM determined as aparent affinity of an IgG to human C4.4a. For instance, the apparent affinity of an antibody of the invention against C4.4a may be about 4.3 nM or 0.03 nM on different tumor cell lines as determined by FACS analysis as depicted in figure 3 f.

An antibody or antigen-binding fragment of the invention internalizes "efficiently" when its time of half maximal internalization (t ½) into C4.4a expressing tumor cells is shorter than 180 min or more preferably shorter than 120 min and still more preferably shorter than 90 min. Further preferred are antibodies or antigen-binding fragments with half maximal internalization times (t ½) of 60 minutes or less as determined by the protocol described in example 6, further preferred are less than 50 minutes, or less than 35 minutes. Table 9 provides a summary of internalization times of representative antibodies of the invention, as determined by the protocol described in example 6. Internalizable antibodies or antigen-binding fragments of the invention are suitable as targeting moiety of an antibody-drug conjugate (ADC). An antibody or antigen-binding fragment is suitable in an in vitro or in vivo method to deliver a compound, preferably a cytotoxic agent, into a C4.4a expressing cell.

Table 9:

| | interalization |
|---|---|
| Antibody (IgG1) | t ½ [min] |
| M31-B01 | 49 |
| M20-D02 S-A | 60 |
| B01-3 | *55* |
| B01-7 | *33* |
| B01-10 | *30* |
| D02-6 | *39* |
| D02-7 | *33* |
| D02-11 | *22* |

In some embodiments, the antibody, antigen-binding fragment thereof, or derivative thereof or nucleic acid encoding the same is isolated. An isolated biological component (such as a nucleic acid molecule or protein such as an antibody) is one that has been substantially separated or purified away from other biological components in the cell of the organism in which the component naturally occurs, e.g., other chromosomal and extra-chromosomal DNA and RNA, proteins and organelles. Nucleic acids and proteins that have been "isolated" include nucleic acids and proteins purified by standard purification methods Sambrook et al., 1989 (Sambrook, J., Fritsch, E. F. and Maniatis, T. (1989) Molecular Cloning: A laboratory manual, Cold Spring Harbor Laboratory Press, Cold Spring Harbor, USA) and Robert K. Scopes eat al 1994 Protein Purification, - Principles and Practice, Springer Science and Business Media LLC. The term also embraces nucleic acids and proteins prepared by recombinant expression in a host cell as well as chemically synthesized nucleic acids.

**Antibody Generation**

A fully human N-CoDeR antibody phage display library was used to isolate high affinity, C4.4a-specific, human monoclonal antibodies by a combination of whole cell and protein panning and through the development of specific tools. These tools and methods include a human C4.4a-expressing recombinant cell-line, a murine C4.4a expressing cell line, recombinant human and murine C4.4a and the development of panning procedures and screening assays capable of identifying antibodies that preferentially bind to C4.4a displayed on the cell surface and that are cross-reactive to murine C4.4a.

Antibodies to the cancer cell-surface marker C4.4a were developed by a combination of three non-conventional approaches in phage-display technology (PDT). First, recombinant cell lines expressing the membrane-bound form of human and mouse C4.4a were constructed by stable transfection of CHO-S cells and A549 tumor cells with a plasmid encoding the full length GPI-anchored form of the human or mouse protein (SEQ ID NO:3) and (SEQ ID NO:4) respectively, to give the human CHO-S:hC4.4a, the murine CHO-S:mC4.4a and the human A549:hC4.4a cell lines, respectively. Second, cell-surface selections were performed with the latter recombinant cell lines and the breast cancer cell line MCF-7. Pre-adsorption with CHO-S cells or non transfected A549 cells was included to avoid the selection of Fab fragments binding to epitopes of the parental cells. Additional selections were performed with recombinant, soluble, purified human C4.4a, with recombinant, soluble, purified murine C4.4a. Third, screening methods were developed which allowed for successive screening of the phage outputs obtained in panning on whole A549:hC4.4a cells as well as CHO-S:hC4.4a cells. The combination of these specific methods allowed the isolation of the unique antibodies "M31-B01", "M20-D02 S-A", "M60-G03", and, "M36-H02".

These unique antibodies were further characterized by their binding affinity in ELISA's, by BIAcore binding to soluble C4.4a, by their ability to recognize different epitopes on soluble C4.4a and by their ability to cross react with murine C4.4a assessed by BIAcore and FACS, and their ability to be internalized in a cell based assay. The internalization assays quantitatively measured the internalization of fluorescently labeled anti-C4.4a antibodies in a time resolved manner.

**Peptide Variants**

Antibodies or antigen-binding fragments of the invention are not limited to the specific peptide sequences provided herein. Rather, the invention also embodies variants of these polypeptides. With reference to the instant disclosure and conventionally available technologies and references, the skilled worker will be able to prepare, test and utilize functional variants of the antibodies disclosed herein, while appreciating that variants having the ability to bind to C4.4a fall within the scope of the present invention.

A variant can include, for example, an antibody that has at least one altered complementary determining region (CDR) (hyper-variable) and/or framework (FR) (variable) domain/position, vis-a-vis a peptide sequence disclosed herein. To better illustrate this concept, a brief description of antibody structure follows.

An antibody is composed of two peptide chains, each containing one (light chain) or three (heavy chain) constant domains and a variable region (VL, VH), the latter of which is in each case made up of four FR regions and three interspaced CDRs. The antigen-binding site is formed by one or more CDRs, yet the FR regions provide the structural framework for the CDRs and, hence, play an important role in antigen binding. By altering one or more amino acid residues in a CDR or FR region, the skilled worker routinely can generate mutated or diversified antibody sequences, which can be screened against the antigen, for new or improved properties, for example.

Tables 3 (VL) and 4 (VH) delineate the CDR and FR regions for certain antibodies of the invention and compare amino acids at a given position to each other and to corresponding consensus sequences.

## *Table 3: VL Sequences*

```
VL sequences

M20-D02 S-A  (1) QSVLTQPPSASGTPGQRVTISCSGSSSNVGS-NPVNWYQQLPGTAPKLLIYRNNQRPSGV
M31-B01      (1) QSVLTQPPSASGTPGQRVTISCTGSSSNIGAGYVVHWYQQLPGTAPKLLIYDNNKRPSGV
B01-3        (1) ESVLTQPPSVSGAPGQRVTISCTGSSSNIGAGYVVHWYQQLPGTAPKLLIYDNNKRPSGV
B01-5        (1) QSVLTQPPSVSGAPGQRVTISCTGSSSNIGAGYVVHWYQQLPGTAPKLLIYDNNKRPSGV
B01-7        (1) QSVLTQPPSASGTPGQRVTISCTGSSSNIGAGYVVHWYQQLPGTAPKLLIYDNNKRPSGV
B01-10       (1) QSVLTQPPSVSGAPGQRVTISCTGSSSNIGAGYVVHWYQQLPGTAPKLLIYDNNKRPSGV
B01-12       (1) QSVLTQPPSASGTPGQRVTISCSGSSSNIGAGYVVHWYQQLPGTAPKLLIYDNNQRPSGV
D02-4        (1) ESVLTQPPSASGTPGQRVTISCSGSSSNIGS-NPVNWYQQLPGTAPKLLIYRNNQRPSGV
D02-6        (1) QSVLTQPPSASGTPGQRVTISCSGSSSNIGS-NPVNWYQQLPGTAPKLLIYRNNQRPSGV
D02-7        (1) QSVLTQPPSASGTPGQRVTISCSGSSSNIGS-NPVNWYQQLPGTAPKLLIYRNNQRPSGV
D02-11       (1) QSVLTQPPSASGTPGQRVTISCSGSSSNIGS-NPVNWYQQLPGTAPKLLIYRNNQRPSGV
D02-13       (1) QSVLTQPPSASGTPGQRVTISCSGSSSNIGS-NPVNWYQQLPGTAPKLLIYRNNQRPSGV
consensus    (1) QSVLTQPPSASGTPGQRVTISCXXXXXXXXXXXXXXWYQQLPGTAPKLLIYXXXXXXXGV
                                       ----LCDR1-----                -LCDR2-

M20-D02 S-A (60) PDRFSGSKSGTSASLAISGLRSEDEADYYCAAWDDRLNGWVFGGGTKLTVLGQ
M31-B01     (61) PDRFSGSKSGTSASLAISGLRSEDEADYYCAAWDDRLNGPVFGGGTKLTVLGQ
B01-3       (61) PDRFSGSKSGTSASLAISGLRSEDEADYYCAAWDDRLNGPVFGGGTKLTVLGQ
B01-5       (61) PDRFSGSKSGTSASLAISGLRSEDEADYYCAAYDDSLSGPVFGGGTKLTVLGQ
B01-7       (61) PDRFSGSKSGTSASLAISGLRSEDEADYYCAAFDDRLNGPVFGGGTKLTVLGQ
B01-10      (61) PDRFSGSKSGTSASLAISGLRSEDEADYYCAAYDDSLSGPVFGGGTKLTVLGQ
B01-12      (61) PDRFSGSKSGTSASLAISGLRSEDEADYYCAAFDDRLSGPVFGGGTKLTVLGQ
D02-4       (60) PDRFSGSKSGTSASLAISGLRSEDEADYYCAAWDDRLNGWGFGGGTKLTVLGQ
D02-6       (60) PDRFSGSKSGTSASLAISGLRSEDEADYYCAAWDDRLSGWAFGGGTKLTVLGQ
D02-7       (60) PDRFSGSKSGTSASLAISGLRSEDEADYYCAAWDDSLSGWAFGGGTKLTVLGQ
D02-11      (60) PDRFSGSKSGTSASLAISGLRSEDEADYYCAAWDDRLSGWGFGGGTKLTVLGQ
D02-13      (60) PDRFSGSKSGTSASLAISGLRSEDEADYYCAAWDDSLSGWAFGGGTKLTVLGQ
consensus   (58) PDRFSGSKSGTSASLAISGLRSEDEADYYXXXXXXXXXXXXFGGGTKLTVLGQ
                                              ---LCDR3----
```

## *Table : 4: VH Sequences*

```
VH sequences

M20-D02 S-A  (1) EVQLLESGGGLVQPGGSLRLSCAASGFTFSDYQMTWIRQTPGKGLEWVSGVSWNGARTH
M31 B01      (1) EVQLLESGGGLVQPGGSLRLSCAASGFTFSNAWMSWVRQAPGKGLEWVSYISSSGSTIY
B01-3        (1) EVQLLESGGGLVQPGGSLRLSCAASGFTFSNAWMSWVRQAPGKGLEWVSYISSSGSTIY
B01-5        (1) EVQLLESGGGLVQPGGSLRLSCAASGFTFSNAWMSWVRQAPGKGLEWVSYISSSGSTIY
B01-7        (1) EVQLLESGGGLVQPGGSLRLSCAASGFTFSNAWMSWVRQAPGKGLEWVSYISSSGSTIY
B01-10       (1) EVQLLESGGGLVQPGGSLRLSCAASGFTFSSAWMSWVRQAPGKGLEWVSYISSSGSTIY
B01-12       (1) EVQLLESGGGLVQPGGSLRLSCAASGFTFSSAWMSWVRQAPGKGLEWVSYISSSGSSTY
D02-4        (1) EVQLLESGGGLVQPGGSLRLSCAASGFTFSDYQMTWIRQTPGKGLEWVSGISWNGGSTH
D02-6        (1) EVQLLESGGGLVQPGGSLRLSCAASGFTFSDYQMTWIRQTPGKGLEWVSGISWNGGSTH
D02-7        (1) EVQLLESGGGLVQPGGSLRLSCAASGFTFSDYQMTWIRQTPGKGLEWVSGISWNGGSTH
D02-11       (1) EVQLLESGGGLVQPGGSLRLSCAASGFTFSDYQMTWIRQTPGKGLEWVSGISWNGGSTH
D02-13       (1) EVQLLESGGGLVQPGGSLRLSCAASGFTFSSYQMTWIRQAPGKGLEWVSGISWNGGSTH
consensus    (1) EVQLLESGGGLVQPGGSLRLSCAASGFTXXXXXXXXVRQAPGKGLEWXXXXXXXXXXXX
                                             --HCDR1-           ------HCDR2-

M20-D02 S-A (60) YADSVKGRFTISRDNSKNTLYLQMNSLRAEDTAVYYCAKGDYLVYSAYYFDSWGQGTLVTVTS
M31 B01     (60) YADSVKGRFTISRDNSKNTLYLQMNSLRAEDTAVYYCAREG-----LWAFDYWGQGTLVTVTS
B01-3       (60) YADSVKGRFTISRDNSKNTLYLQMNSLRAEDTAVYYCAREG-----LWAFDYWGQGTLVTVSS
B01-5       (60) YADSVKGRFTISRDNSKNTLYLQMNSLRAEDTAVYYCAREG-----LWAFDYWGQGTLVTVSS
B01-7       (60) YADSVKGRFTISRDNSKNTLYLQMNSLRAEDTAVYYCAREG-----LWAFDKWGQGTLVTVSS
B01-10      (60) YADSVKGRFTISRDNSKNTLYLQMNSLRAEDTAVYYCAREG-----LWAFDYWGQGTLVTVSS
B01-12      (60) YADSVKGRFTISRDNSKNTLYLQMNSLRAEDTAVYYCAREG-----LWAFDKWGQGTLVTVSS
D02-4       (60) YADSVKGRFTISRDNSKNTLYLQMNSLRAEDTAVYYCAKGDYLVYSAYYFDSWGQGTLVTVSS
D02-6       (60) YADSVKGRFTISRDNSKNTLYLQMNSLRAEDTAVYYCAKGDYLVYSSYYFKSWGQGTLVTVSS
D02-7       (60) YADSVKGRFTISRDNSKNTLYLQMNSLRAEDTAVYYCAKGDYLVYKSYYFKSWGQGTLVTVSS
D02-11      (60) YADSVKGRFTISRDNSKNTLYLQMNSLRAEDTAVYYCAKGDYLVYSSYYFKSWGQGTLVTVSS
D02-13      (60) YADSVKGRFTISRDNSKNTLYLQMNSLRAEDTAVYYCAKGDYLVYKSYYFKSWGQGTLVTVSS
consensus   (60) XXXXXXXXFTISRDNSKNTLYLQMNSLRAEDTAVYYCXXXXXXXXXXXXXXXWGQGTLVTVTS
                 --------                                 -----HCDR3-----
```

A further preferred embodiment of the invention is an antibody or antigen binding fragment thereof in which the CDR sequences are selected as shown in table 7.

A further preferred embodiment of the invention is an antibody or antigen-binding fragment in which the VH and VL sequences are selected as shown in table 7. The skilled worker can use the data in Tables 3, 4 and 7 to design peptide variants that are within the scope of the present invention. It is preferred that variants are constructed by changing amino acids within one or more CDR regions; a variant might also have one or more altered framework regions. Alterations also may be made in the framework regions. For example, a peptide FR domain might be altered where there is a deviation in a residue compared to a germline sequence.

With reference to a comparison of the novel antibodies to the corresponding consensus sequences, which are listed in Tables 3 and 4 candidate residues that can be changed include e.g. residue 42 of the variable heavy chain of M20-D02 S-A compared to VHIII of Gene DP47. Alternatively, the skilled worker could make the same analysis by comparing the amino acid sequences disclosed herein to known sequences of the same class of such antibodies, using, for example, the procedure described by Knappik A., et al., JMB 2000, 296:57-86.

Furthermore, variants may be obtained by using one antibody as starting point for optimization by diversifying one or more amino acid residues in the antibody, preferably amino acid residues in one or more CDRs, and by screening the resulting collection of antibody variants for variants with improved properties. Particularly preferred is diversification of one or more amino acid residues in CDR3 of VL and/or VH.. Diversification can be done by synthesizing a collection of DNA molecules using trinucleotide mutagenesis (TRIM) technology (Virnekäs B. et al., Nucl. Acids Res. 1994, 22: 5600.). Antibodies or antigen-binding fragments thereof include molecules with modifications/variations including but not limited to e.g. modifications leading to altered half-life (e.g. modification of the Fc part or attachment of further molecules such as PEG) or altered ADCC or CDC activity.

### Conservative Amino Acid Variants

Polypeptide variants may be made that conserve the overall molecular structure of an antibody peptide sequence described herein. Given the properties of the individual amino acids, some rational substitutions will be recognized by the skilled worker. Amino acid substitutions, *i.e.,* "conservative substitutions," may be made, for instance, on the basis of similarity in polarity, charge, solubility, hydrophobicity, hydrophilicity, and/or the amphipathic nature of the residues involved.

For example, (a) nonpolar (hydrophobic) amino acids include alanine, leucine, isoleucine, valine, proline, phenylalanine, tryptophane, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt $\alpha$-helices. Similarly, certain amino acids, such as alanine, cysteine, leucine, methionine, glutamic acid, glutamine, histidine and lysine are more commonly found in $\alpha$-helices, while valine, isoleucine, phenylalanine, tyrosine, tryptophan and threonine are more commonly found in $\beta$-pleated sheets. Glycine, serine, aspartic acid, asparagine, and proline are commonly found in turns. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants. In one particular example, amino acid position 3 in SEQ ID NOS: 33 -36 can be changed from a Q to an E.

As used herein, "sequence identity" between two polypeptide sequences, indicates the percentage of amino acids that are identical between the sequences. "Sequence homology" indicates the percentage of amino acids that either is identical or that represent conservative amino acid substitutions.

**DNA molecules of the invention**

The present invention also relates to the DNA molecules that encode an antibody of the invention or antigen-binding fragment thereof. These sequences include, but are not limited to, those DNA molecules set forth in SEQ IDs 37-44, 53, 54, 63, 64, 73, 74, 83, 84, 93, 94, 103, 104, 113, 114, 123, 124, 133, 134, 143, 144 and 308 - 345.

DNA molecules of the invention are not limited to the sequences disclosed herein, but also include variants thereof. DNA variants within the invention may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 *supra* and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

Structural similarity between two polynucleotide sequences can be expressed as a function of "stringency" of the conditions under which the two sequences will hybridize with one another. As used herein, the term "stringency" refers to the extent that the conditions disfavor hybridization. Stringent conditions strongly disfavor hybridization, and only the most structurally related molecules will hybridize to one another under such conditions. Conversely, non-stringent conditions favor hybridization of molecules displaying a lesser degree of structural relatedness. Hybridization stringency, therefore, directly correlates with the structural relationships of two nucleic acid sequences. The following relationships are useful in correlating hybridization and relatedness (where $T_m$ is the melting temperature of a nucleic acid duplex):

a.

$$T_m = 69.3 + 0.41(G+C)\%$$

b. The $T_m$ of a duplex DNA decreases by 1°C with every increase of 1% in the number of mismatched base pairs.

c.

$$(T_m)_{\mu 2} - (T_m)_{\mu 1} = 18.5 \log_{10}\mu 2/\mu 1$$

where $\mu 1$ and $\mu 2$ are the ionic strengths of two solutions.

Hybridization stringency is a function of many factors, including overall DNA concentration, ionic strength, temperature, probe size and the presence of agents which disrupt hydrogen bonding. Factors promoting hybridization include high DNA concentrations, high ionic strengths, low temperatures, longer probe size and the absence of agents that disrupt hydrogen bonding. Hybridization typically is performed in two phases: the "binding" phase and the "washing" phase.

First, in the binding phase, the probe is bound to the target under conditions favoring hybridization. Stringency is usually controlled at this stage by altering the temperature. For high stringency, the temperature is usually between 65°C and 70°C, unless short (< 20 nt) oligonucleotide probes are used. A representative hybridization solution comprises 6X SSC, 0.5% SDS, 5X Denhardt's solution and 100 μg of nonspecific carrier DNA. See Ausubel *et al.,* section 2.9, supplement 27 (1994). Of course, many different, yet functionally equivalent, buffer conditions are known. Where the degree of relatedness is lower, a lower temperature may be chosen. Low stringency binding temperatures are between about 25°C and 40°C. Medium stringency is between at least about 40°C to less than about 65°C. High stringency is at least about 65°C.

Second, the excess probe is removed by washing. It is at this phase that more stringent conditions usually are applied. Hence, it is this "washing" stage that is most important in determining relatedness via hybridization. Washing solutions typically contain lower salt concentrations. One exemplary medium stringency solution contains 2X SSC and 0.1% SDS. A high stringency wash solution contains the equivalent (in ionic strength) of less than about 0.2X SSC, with a preferred stringent solution containing about O.1X SSC. The temperatures associated with various stringencies are the same as discussed above for "binding." The washing solution also typically is replaced a number of times during washing. For example, typical high stringency washing conditions comprise washing twice for 30 minutes at 55° C. and three times for 15 minutes at 60° C.

An embodiment of the invention is an isolated nucleic acid sequence that encodes (i) the antibody or antigen-binding fragment of the invention, the CDR sequences as depicted in table 7, or (ii) the variable light and heavy chain sequences as depicted in table 7, or (iii) which comprises a nucleic acid sequence that encodes an antibody or antigen-binding fragment of the invention, the CDR sequences as depicted in table 7, or the variable light and heavy chain sequences as depicted in table 7.

In one particular example of a variant of the invention, nucleic acid position 7 in SEQ ID NOS: 37-40, or position 16 in SEQ ID NOS : 41-44 can be substituted from a C to a G, thereby changing the codon from CAC to GAG.

**Functionally Equivalent Variants**

Yet another class of DNA variants within the scope of the invention may be described with reference to the product they encode. These functionally equivalent polynucleotides are characterized by the fact that they encode the same peptide sequences found in SEQ ID NOS: 5-36, 45-50, 55-60, 65-70, 75-80, 85-90, 95-100, 105-110, 115-120, 125-130, 135-140, due to the degeneracy of the genetic code.

It is recognized that variants of DNA molecules provided herein can be constructed in several different ways. For example, they may be constructed as completely synthetic DNAs. Methods of efficiently synthesizing oligonucleotides in the range of 20 to about 150 nucleotides are widely available. *See* Ausubel *et al.,* section 2.11, Supplement 21 (1993). Overlapping oligonucleotides may be synthesized and assembled in a fashion first reported by Khorana et al., J. Mol. Biol. 72:209-217 (1971); *see also* Ausubel *et al., supra,* Section 8.2. Synthetic DNAs preferably are designed with convenient restriction sites engineered at the 5' and 3' ends of the gene to facilitate cloning into an appropriate vector.

As indicated, a method of generating variants is to start with one of the DNAs disclosed herein and then to conduct site-directed mutagenesis. *See* Ausubel et al., *supra,* chapter 8, Supplement 37 (1997). In a typical method, a target DNA is cloned into a single-stranded DNA bacteriophage vehicle. Single-stranded DNA is isolated and hybridized with an oligonucleotide containing the desired nucleotide alteration(s). The complementary strand is synthesized and the double stranded phage is introduced into a host. Some of the resulting progeny will contain the desired mutant, which can be confirmed using DNA sequencing. In addition, various methods are available that increase the probability that the progeny phage will be the desired mutant. These methods are well known to those in the field and kits are commercially available for generating such mutants.

**Recombinant DNA constructs and expression**

The present invention further provides recombinant DNA constructs comprising one or more of the nucleotide sequences of the present invention. The recombinant constructs of the present invention are used in connection with a vector, such as a plasmid, phagemid, phage or viral vector, into which a DNA molecule encoding an antibody of the invention or antigen-binding fragment thereof is inserted.

An antibody, antigen binding portion, or derivative thereof provided herein can be prepared by recombinant expression of nucleic acid sequences encoding light and heavy chains or portions thereof in a host cell. To express an antibody, antigen binding portion, or derivative thereof recombinantly, a host cell can be transfected with one or more recombinant expression vectors carrying DNA fragments encoding the light and/or heavy chains or portions thereof such that the light and heavy chains are expressed in the host cell. Standard recombinant DNA methodologies are used prepare and/or obtain nucleic acids encoding the heavy and light chains, incorporate these nucleic acids into recombinant expression vectors and introduce the vectors into host cells, such as those described in Sambrook, Fritsch and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in U.S. Pat. No. 4,816,397 by Boss et al.

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding portions or derivatives thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g. bacteria, examples for eukaryotic host cells are yeast, insect or mammalian cells. In some embodiments, the DNAs encoding the heavy and light chains are inserted into separate vectors. In other embodiments, the DNA encoding the heavy and light chains are inserted into the same vector. It is understood that the design of the expression vector, including the selection of regulatory sequences is affected by factors such as the choice of the host cell, the level of expression of protein desired and whether expression is constitutive or inducible.

## Bacterial Expression

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include *E. coli, Bacillus subtilis, Salmonella typhimurium* and various species within the genera Pseudomonas, Streptomyces, and Staphylococcus.

Bacterial vectors may be, for example, bacteriophage-, plasmid- or phagemid-based. These vectors can contain a selectable marker and bacterial origin of replication derived from commercially available plasmids typically containing elements of the well known cloning vector pBR322 (ATCC 37017). Following transformation of a suitable host strain and growth of the host strain to an appropriate cell density, the selected promoter is de-repressed/induced by appropriate means (*e.g.*, temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable. Antibodies of the present invention or antigen-binding fragment thereof include naturally purified products, products of chemical synthetic procedures, and products produced by recombinant techniques from a prokaryotic host, including, for example, *E. coli, Bacillus subtilis, Salmonella typhimurium* and various species within the genera Pseudomonas, Streptomyces, and Staphylococcus, preferably, from *E. coli* cells.

## Mammalian Expression & Purification

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, see e.g., U.S. 5,168,062 by Stinski, U.S. 4,510,245 by Bell et al. and U.S. 4,968,615 by Schaffner et al. The recombinant expression vectors can also include origins of replication and selectable markers (see e.g., U.S. 4,399,216, 4,634,665 and U.S. 5,179,017, by Axel et al.). Suitable selectable markers include genes that confer resistance to drugs such as G418, hygromycin or methotrexate, on a host cell into which the vector has been introduced. For example, the dihydrofolate reductase (DHFR) gene confers resistance to methotrexate and the neo gene confers resistance to G418.

Transfection of the expression vector into a host cell can be carried out using standard techniques such as electroporation, calcium-phosphate precipitation, and DEAE-dextran, lipofection or polycation-mediated transfection.

Suitable mammalian host cells for expressing the antibodies, antigen binding portions, or derivatives thereof provided herein include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621, NSO myeloma cells, COS cells and SP2 cells. In some embodiments, the expression vector is designed such that the expressed protein is secreted into the culture medium in which the host cells are grown. Transient transfection/epression of antibodies can for example be achieved following the protocols by Durocher et al (2002) Nucl.Acids Res. Vol 30 e9. Stable transfection/expression of antibodies can for example be achieved following the protocols of the UCOE system (T. Benton et al. (2002) Cytotechnology 38: 43-46).

The antibodies, antigen binding portions, or derivatives thereof can be recovered from the culture medium using standard protein purification methods.

Antibodies of the invention or an antigen-binding fragment thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phosphocellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the present invention or antigen-binding fragment thereof include naturally purified products, products of chemical synthetic procedures, and products produced by recombinant techniques from a eukaryotic host, including, for example, yeast, higher plant, insect and mammalian cells, preferably from mammalian cells. Depending upon the host employed in a recombinant production procedure, the antibody of the present invention can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20.

**Therapeutic Methods**

Therapeutic methods involve administering to a subject in need of treatment a therapeutically effective amount of an antibody or antigen-binding fragment thereof contemplated by the invention. A "therapeutically effective" amount hereby is defined as the amount of an antibody or antigen-binding fragment that is of sufficient quantity to deplete C4.4a-positive cells in a treated area of a subject - either as a single dose or according to a multiple dose regimen, alone or in combination with other agents, which leads to the alleviation of an adverse condition, yet which amount is toxicologically tolerable. The subject may be a human or non-human animal (*e.g.*, rabbit, rat, mouse, dog, monkey or other lower-order primate).

An antibody of the invention or antigen-binding fragment thereof might be co-administered with known medicaments, and in some instances the antibody might itself be modified. For example, an antibody could be conjugated to a cytotoxic agent or radioisotope to potentially further increase efficacy.

Antibodies of the present invention may be administered as the sole pharmaceutical agent or in combination with one or more additional therapeutic agents where the combination causes no unacceptable adverse effects. This combination therapy includes administration of a single pharmaceutical dosage formulation which contains an antibody of the invention and one or more additional therapeutic agents, as well as administration of an antibody of the invention and each additional therapeutic agent in its own separate pharmaceutical dosage formulation. For example, an antibody of the invention and a therapeutic agent may be administered to the patient together in a single oral dosage composition such as a tablet or capsule, or each agent may be administered in separate dosage formulations.

Where separate dosage formulations are used, an antibody of the invention and one or more additional therapeutic agents may be administered at essentially the same time *(e.g.,* concurrently) or at separately staggered times (*e.g.,* sequentially).

In particular, an antibodies of the present invention may be used in fixed or separate combination with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a nonlimiting list of examples of secondary agents that may be used in combination with the antibodies of the present invention:

Alkylating agents include, but are not limited to, nitrogen mustard N-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, bendamustin, and mitolactol; platinum-coordinated alkylating compounds include, but are not limited to, cisplatin, carboplatin, eptaplatin, lobaplatin, nedaplatin, oxaliplatin, and satraplatin;

Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;

Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant, Trelstar, toremifene, raloxifene, lasofoxifene, letrozole, anti-androgens such as bicalutamide, flutamide, mifepristone, nilutamide, Casodex, and anti-progesterones and combinations thereof;

Plant-derived anti-tumor substances include, e.g., those selected from mitotic inhibitors, for example epothilones such as sagopilone, ixabepilone and epothilone B, vinblastine, vinflunine, docetaxel, and paclitaxel;

Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;

Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge;

Biological response modifiers are agents that modify defense mechanisms of living organisms or biological responses such as survival, growth or differentiation of tissue cells to direct them to have anti-tumor activity; such agents include, e.g., krestin, lentinan, sizofiran, picibanil, ProMune, and ubenimex;

Anti-angiogenic compounds include, but are not limited to, acitretin, aflibercept, angiostatin, aplidine, asentar, axitinib, bevacizumab, brivanib alaninat, cilengtide, combretastatin, endostatin, fenretinide, halofuginone, pazopanib, ranibizumab, rebimastat, recentin, regorafenib, removab, revlimid, sorafenib, squalamine, sunitinib, telatinib, thalidomide, ukrain, vatalanib, and vitaxin;

Antibodies include, but are not limited to, trastuzumab, cetuximab, bevacizumab, rituximab, ticilimumab, ipilimumab, lumiliximab, catumaxomab, atacicept, oregovomab, and alemtuzumab;

VEGF inhibitors such as, e.g., sorafenib, regorafenib, bevacizumab, sunitinib, recentin, axitinib, aflibercept, telatinib, brivanib alaninate, vatalanib, pazopanib, and ranibizumab;

EGFR (HER1) inhibitors such as, e.g., cetuximab, panitumumab, vectibix, gefitinib, erlotinib, and Zactima;

HER2 inhibitors such as, e.g., lapatinib, tratuzumab, and pertuzumab;

mTOR inhibitors such as, e.g., temsirolimus, sirolimus/Rapamycin, and everolimus;

c-Met inhibitors;

PI3K and AKT inhibitors;

CDK inhibitors such as roscovitine and flavopiridol;

Spindle assembly checkpoints inhibitors and targeted anti-mitotic agents such as PLK inhibitors, Aurora inhibitors (e.g. Hesperadin), checkpoint kinase inhibitors, and KSP inhibitors;

HDAC inhibitors such as, e.g., panobinostat, vorinostat, MS275, belinostat, and LBH589;

HSP90 and HSP70 inhibitors;

Proteasome inhibitors such as bortezomib and carfilzomib;

Serine/threonine kinase inhibitors including MEK inhibitors and Raf inhibitors such as sorafenib;

Farnesyl transferase inhibitors such as, e.g., tipifarnib;

Tyrosine kinase inhibitors including, e.g., dasatinib, nilotibib, regorafenib, bosutinib, sorafenib, bevacizumab, sunitinib, cediranib, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors;

Vitamin D receptor agonists;

Bcl-2 protein inhibitors such as obatoclax, oblimersen sodium, and gossypol;

Cluster of differentiation 20 receptor antagonists such as, e.g., rituximab;

Ribonucleotide reductase inhibitors such as, e.g., gemcitabine;

Tumor necrosis apoptosis inducing ligand receptor 1 agonists such as, e.g., mapatumumab;
5-Hydroxytryptamine receptor antagonists such as, e.g., rEV598, xaliprode, palonosetron hydrochloride, granisetron, Zindol, and AB-1001;
Integrin inhibitors including alpha5-betal integrin inhibitors such as, e.g., E7820, JSM 6425, volociximab, and endostatin;
Androgen receptor antagonists including, e.g., nandrolone decanoate, fluoxymesterone, Android, Prost-aid, andromustine, bicalutamide, flutamide, apocyproterone, apo-flutamide, chlormadinone acetate, Androcur, Tabi, cyproterone acetate, and nilutamide;
Aromatase inhibitors such as, e.g., anastrozole, letrozole, testolactone, exemestane, aminoglutethimide, and formestane;
Matrix metalloproteinase inhibitors;
Other anti-cancer agents including, e.g., alitretinoin, ampligen, atrasentan bexarotene, bortezomib, bosentan, calcitriol, exisulind, fotemustine, ibandronic acid, miltefosine, mitoxantrone, l-asparaginase, procarbazine, dacarbazine, hydroxycarbamide, pegaspargase, pentostatin, tazaroten, velcade, gallium nitrate, canfosfamide, darinaparsin, and tretinoin.

In a preferred embodiment, the antibodies of the present invention may be used in combination with chemotherapy (i.e. cytotoxic agents), anti-hormones and/or targeted therapies such as other kinase inhibitors (for example, EGFR inhibitors), mTOR inhibitors and angiogenesis inhibitors.

The compounds of the present invention may also be employed in cancer treatment in conjunction with radiation therapy and/or surgical intervention.

An antibody of the invention or antigen-binding fragment thereof might in some instances itself be modified. For example, an antibody could be conjugated to any of but not limited to the compounds mentioned above or any radioisotope to potentially further increase efficacy. Furthermore, the antibodies of the invention may be utilized, as such or in compositions, in research and diagnostics, or as analytical reference standards, and the like, which are well known in the art.

The inventive antibodies or antigen-binding fragments thereof can be used as a therapeutic or a diagnostic tool in a variety of situations where C4.4a is undesirably expressed or found, e.g. cell proliferative disorders such as cancer. Disorders and conditions particularly suitable for treatment with an antibody of the inventions are solid tumors, such as cancers of the breast, respiratory tract, brain, reproductive organs, digestive tract, urinary tract, eye, liver, skin, head and neck, thyroid, parathyroid, and their distant metastases. Those disorders also include lymphomas, sarcomas and leukemias.

Examples of breast cancer include, but are not limited to invasive ductal carcinoma, invasive lobular carcinoma, ductal carcinoma *in situ,* and lobular *carcinoma in situ.*

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal and vulvar cancer, as well as sarcoma of the uterus.

Tumors of the digestive tract include, but are not limited to anal, colon, colorectal, esophageal, gallbladder, gastric, pancreatic, rectal, small-intestine, and salivary gland cancers.

Tumors of the urinary tract include, but are not limited to bladder, penile, kidney, renal pelvis, ureter, urethral, and hereditary and sporadic papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer, and squamous cell cancer.

Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

The disorders mentioned above have been well characterized in humans, but also exist with a similar etiology in other animals, including mammals, and can be treated by administering pharmaceutical compositions of the present invention.

To treat any of the foregoing disorders, pharmaceutical compositions for use in accordance with the present invention may be formulated in a conventional manner using one or more physiologically acceptable carriers or excipients. An antibody of the invention or antigen-binding fragment thereof can be administered by any suitable means, which can vary, depending on the type of disorder being treated. Possible administration routes include parenteral (e.g., intramuscular, intravenous, intra-arterial, intraperitoneal, or subcutaneous), intrapulmonary and intranasal, and, if desired for local immunosuppressive treatment, intralesional administration. In addition, an antibody of the invention might be administered by pulse infusion, with, e.g., declining doses of the antibody. Preferably, the dosing is given by injections, most preferably intravenous or subcutaneous injections, depending in part on whether the administration is brief or chronic. The amount to be administered will depend on a variety of factors such as the clinical symptoms, weight of the individual, whether other drugs are administered. The skilled artisan will recognize that the route of administration will vary depending on the disorder or condition to be treated.

Determining a therapeutically effective amount of the novel polypeptide, according to this invention, largely will depend on particular patient characteristics, route of administration, and the nature of the disorder being treated. General guidance can be found, for example, in the publications of the International Conference on Harmonization and in REMINGTON'S PHARMACEUTICAL SCIENCES, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references. Efficacy may be determined utilizing the same guidance in conjunction with the methods described below in the Examples.

**Diagnostic Methods**

C4.4a antibodies or antigen-binding fragments thereof can be used for detecting the presence of C4.4a-expressing tumors. The presence of C4.4a-containing cells or shed C4.4a within various biological samples, including serum, and tissue biopsy specimens, may be detected with C4.4a antibodies. In addition, C4.4a antibodies may be used in various imaging methodologies such as immunoscintigraphy with a $^{99}$Tc (or other isotope) conjugated antibody. For example, an imaging protocol similar to the one recently described using a $^{111}$In conjugated anti-PSMA antibody may be used to detect pancreatic or ovarian carcinomas (Sodee et al., Clin. Nuc. Med. 21: 759-766, 1997). Another method of detection that can be used is positron emitting tomography by conjugating the antibodies of the invention with a suitable isotope (see Herzog et al., J. Nucl. Med. 34:2222-2226, 1993).

**Pharmaceutical Compositions and Administration**

An embodiment of the present invention are pharmaceutical compositions which comprise C4.4a antibodies or antigen-binding fragment thereof, alone or in combination with at least one other agent, such as stabilizing compound, which may be administered in any sterile, biocompatible pharmaceutical carrier, including, but not limited to, saline, buffered saline, dextrose, and water. A further embodiment are pharmaceutical compositions comprising a C4.4a binding antibody or antigen-binding fragment therof and a further pharmaceutically active compound that is suitable to treat C4.4a related diseases such as cancer. Any of these molecules can be administered to a patient alone, or in combination with other agents, drugs or hormones, in pharmaceutical compositions where it is mixed with excipient(s) or pharmaceutically acceptable carriers. In one embodiment of the present invention, the pharmaceutically acceptable carrier is pharmaceutically inert.

The present invention also relates to the administration of pharmaceutical compositions. Such administration is accomplished orally or parenterally. Methods of parenteral delivery include topical, intra-arterial (directly to the tumor), intramuscular, subcutaneous, intramedullary, intrathecal, intraventricular, intravenous, intraperitoneal, or intranasal administration. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Ed. Maack Publishing Co, Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, or other plants; cellulose such as methyl, cellulose, hydroxypropylmethylcellulose, or sodium carboxymethyl cellulose; and gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinyl pyrrolidone, carbopol gel, polyethylene glycol and/or titanium dioxide, lacquer solutions, and suitable organic solvents or solvent mixtures. Dyestuffs or pigments may be added to the tablets or dragee coatings for product identification or to characterize the quantity of active compound, i.e. dosage.

Pharmaceutical preparations that can be used orally include push-fit capsules made of gelatin, as well as soft, sealed capsules made of gelatin and a coating such as glycerol or sorbitol. Push-fit capsules can contain active ingredients mixed with a filler or binders such as lactose or starches, lubricants such as talc or magnesium stearate, and optionally, stabilizers. In soft capsules, the active compounds may be dissolved or suspended in suitable liquids, such as fatty oils, liquid paraffin, or liquid polyethylene glycol with or without stabilizers.

Pharmaceutical formulations for parenteral administration include aqueous solutions of active compounds. For injection, the pharmaceutical compositions of the invention may be formulated in aqueous solutions, preferably in physiologically compatible buffers such as Hank's solution, Ringer's solution, or physiologically buffered saline. Aqueous injection suspensions may contain substances that increase viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

**Kits**

The invention further relates to pharmaceutical packs and kits comprising one or more containers filled with one or more of the ingredients of the aforementioned compositions of the invention. Associated with such container(s) can be a notice in the form prescribed by a governmental agency regulating the manufacture, use or sale of pharmaceuticals or biological products, reflecting approval by the agency of the manufacture, use or sale of the product for human administration.

In another embodiment, the kits may contain DNA sequences encoding the antibodies of the invention. Preferably the DNA sequences encoding these antibodies are provided in a plasmid suitable for transfection into and expression by a host cell. The plasmid may contain a promoter (often an inducible promoter) to regulate expression of the DNA in the host cell. The plasmid may also contain appropriate restriction sites to facilitate the insertion of other DNA sequences into the plasmid to produce various antibodies. The plasmids may also contain numerous other elements to facilitate cloning and expression of the encoded proteins. Such elements are well known to those of skill in the art and include, for example, selectable markers, initiation codons, termination codons, and the like.

**Manufacture and Storage.**

The pharmaceutical compositions of the present invention may be manufactured in a manner that is known in the art, e.g., by means of conventional mixing, dissolving, granulating, dragee-making, levigating, emulsifying, encapsulating, entrapping or lyophilizing processes.

The pharmaceutical composition may be provided as a salt and can be formed with acids, including by not limited to hydrochloric, sulfuric, acetic, lactic, tartaric, malic, succinic, etc. Salts tend to be more soluble in aqueous or other protonic solvents that are the corresponding free base forms. In other cases, the preferred preparation may be a lyophilized powder in 1 mM-50 mM histidine, 0.1%-2% sucrose, 2%-7% mannitol at a pH range of 4.5 to 5.5 that is combined with buffer prior to use.

After pharmaceutical compositions comprising a compound of the invention formulated in an acceptable carrier have been prepared, they can be placed in an appropriate container and labeled for treatment of an indicated condition. For administration of C4.4a antibodies or antigen-binding fragment thereof, such labeling would include amount, frequency and method of administration.

**Therapeutically Effective Dose.**

Pharmaceutical compositions suitable for use in the present invention include compositions wherein the active ingredients are contained in an effective amount to achieve the intended purpose, i.e. treatment of a particular disease state characterized by C4.4a expression. The determination of an effective dose is well within the capability of those skilled in the art.

For any compound, the therapeutically effective dose can be estimated initially either in cell culture assays, e.g., neoplastic cells, or in animal models, usually mice, rabbits, dogs, pigs or monkeys. The animal model is also used to achieve a desirable concentration range and route of administration. Such information can then be used to determine useful doses and routes for administration in humans.

A therapeutically effective dose refers to that amount of antibody or antigen-binding fragment thereof, that ameliorate the symptoms or condition. Therapeutic efficacy and toxicity of such compounds can be determined by standard pharmaceutical procedures in cell cultures or experimental animals, e.g., $ED_{50}$ (the dose therapeutically effective in 50% of the population) and $LD_{50}$ (the dose lethal to 50% of the population). The dose ratio between therapeutic and toxic effects is the therapeutic index, and it can be expressed as the ratio, $ED_{50}/LD_{50}$. Pharmaceutical compositions that exhibit large therapeutic indices are preferred. The data obtained from cell culture assays and animal studies are used in formulating a range of dosage for human use. The dosage of such compounds lies preferably within a range of circulating concentrations what include the $ED_{50}$ with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors that may be taken into account include the severity of the disease state, e.g., tumor size and location; age, weight and gender of the patient; diet, time and frequency of administration, drug combination(s), reaction sensitivities, and tolerance/response to therapy. Long acting pharmaceutical compositions might be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature. See U.S. Pat. No. 4,657,760; 5,206,344; or 5,225,212. Those skilled in the art will employ different formulations for polynucleotides than for proteins or their inhibitors. Similarly, delivery of polynucleotides or polypeptides will be specific to particular cells, conditions, locations, etc. Preferred specific activities for a radiolabelled antibody may range from 0.1 to 10 mCi/mg of protein (Riva et al., Clin. Cancer Res. 5:3275-3280, 1999; Ulaner et al., 2008 Radiology 246(3):895-902)

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

All examples were carried out using standard techniques, which are well known and routine to those of skill in the art, except where otherwise described in detail. Routine molecular biology techniques of the following examples can be carried out as described in standard laboratory manuals, such as Sambrook et al., Molecular Cloning: A Laboratory Manual, 2nd Ed.; Cold Spring Harbor Laboratory Press, Cold Spring Harbor, N.Y., 1989.

A preferred embodiment of the invention is:

A. An isolated antibody or antigen-binding fragment thereof specifically binding to C4.4a, preferably to domain S1 of C4.4a.

B. The antibody or antigen-binding fragment thereof according to embodiment A wherein the antibody or antigen-binding fragment thereof is cross-reactive to rodent C4.4a, preferably to murine C4.4a.

C. The antibody or antigen-binding fragment thereof according to embodiments A or B wherein the antibody or antigen-binding fragment thereof is internalized following binding to C4.4a expressing cells.

D. The antibody or antigen-binding fragment according to any one of embodiments A to C, wherein the antibody or antigen-binding fragment competes in binding to C4.4a with antibody M31-B01 or M20-D02 S-A.

E. The antibody or antigen-binding fragment according to embodiment D, wherein the amino acid sequence of the antibody or antigen-binding fragment is at least 50%, 55%, 60% 70%, 80%, 90, or 95% identical to at least one CDR sequence depicted in table 7, or at least 50%, 60%, 70%, 80%, 90%, 92% or 95% identical to at least one VH or VL sequence depicted in table 7.

F. The antibody or antigen-binding fragment according to any one of embodiments D to E, wherein the amino acid sequence of the antibody or antigen-binding fragment is at least 50%, 55%, 60% 70%, 80%, 90, or 95% identical to at least one CDR sequence of M31-B01 or M20-D02 S-A, or at least 50%, 60%, 70%, 80%, 90%, 92% or 95% identical to the VH or VL sequence of M31-B01 or M20-D02 S-A.

G. The antibody or antigen-binding fragment according to any one of embodiments D to F, wherein the antibody or antigen-binding fragment thereof comprises at least one of the heavy chain CDR sequences that conforms to the consensus sequences SEQ ID NO: 297 or SEQ ID NO: 302 (CDR H1), SEQ ID NO: 298 or SEQ ID NO: 303 (CDR H2), or SEQ ID NO: 299 or SEQ ID NO: 304 (CDR H3), and/or at least one of the light chain CDR sequences that conform to the consensus sequences of SEQ ID NO: 300 or SEQ ID NO: 305 (CDR L1), SEQ ID NO: 22 or SEQ ID NO: 306 (CDR L2), or SEQ ID NO: 301 or SEQ ID NO: 307 (CDR L3).

H. The antibody or antigen-binding fragment according to any one of embodiments D to G,

a) wherein the antibody or antigen-binding fragment thereof comprises the heavy chain CDR sequences conforming to SEQ ID NO: 297 (CDR H1), SEQ ID NO: 298 (CDR H2) and SEQ ID NO: 299 (CDR H3), and the light chain CDR sequences conforming to SEQ ID NO: 300 (CDR L1), SEQ ID NO: 22 (CDR L2) and SEQ ID NO: 301 (CDR L3), or

b) wherein the antibody or antigen-binding fragment thereof comprises the heavy chain CDR sequences conforming to SEQ ID NO: 302 (CDR H1), SEQ ID NO: 303 (CDR H2) and SEQ ID NO: 304 (CDR H3), and the light chain CDR sequences conforming to SEQ ID NO: 305 (CDR L1), SEQ ID NO: 306 (CDR L2) and SEQ ID NO: 307 (CDR L3).

1. The antibody or antigen-binding fragment according to any one of embodiments D to H, comprising the variable heavy chain and variable light chain CDR sequences of antibody M31-B01 or modified therefrom, wherein

i. in the modified CDR-H1 the amino acid at position 3 is selected from the group consisting of N and S, and at position 4 is selected from the group consisting of A and Y;

ii. in the modified CDR-H2 the amino acid at position 10 is selected from the group consisting of T and S, and at position 11 is selected from the group consisting of I and T;

iii. in the modified CDR-H3 the amino acid at position 10 is selected from the group consisting of Y, K, G, W and N;

iv. in the modified CDR-L1 the amino acid at position 1 is selected from the group consisting of T and S;

v. in the modified CDR-L2 the amino acid at position 4 is selected from the group consisting of Q and K;

vi. in the modified CDR-L3 the amino acid at position 4 is selected from the group consisting of W, E, F and Y, at position 7 is selected from the group consisting of R, S and M, at position 9 is selected from the group consisting of N, K and S, at position 10 is selected from the group consisting of G and R, and at position 11 is selected from the group consisting of P and A.

J. The antibody or antigen-binding fragment according to embodiment G to H comprising the framework sequence of the variable heavy chain and variable light chain CDR sequences of antibody M31-B01 or modified therefrom, wherein in the modified variable light chain the amino acid at position 10 is selected from the group consisting of A and V and at position 13 is selected from the group consisting of A and T.

K. The antibody or antigen-binding fragment according to any ones of embodiments D - H, comprising the variable heavy chain and variable light chain CDR sequences of antibody M20-D02 S-A or modified therefrom, wherein

i. in the modified CDR-H1 the amino acid at position 3 is selected from the group consisting of D and S;

ii. in the modified CDR-H2 the amino acid at position 4 is selected from the group consisting of V and I, at position 9 is selected from the group consisting of A and G, and at position 10 is selected from the group consisting of R and S;

iii. in the modified CDR-H3 the amino acid at position 9 is selected from the group consisting of S, K and R, at position 10 is selected from the group consisting of A, S and R, at position 14 is selected from the group consisting of D, K, E and R, and at position 15 is selected from the group consisting of S and Y;

iv. in the modified CDR-L1 the amino acid at position 7 is selected from the group consisting of V and I;

v. in the modified CDR-L3 the amino acid at position 3 is selected from the group consisting of A, Q and R, at position 5 is selected from the group consisting of D and G, at position 7 is selected from the group consisting of R and S, at position 9 is selected from the group consisting of N, W and S, and at position 12 is selected from the group consisting of V, A and G.

L. The antibody or antigen-binding fragment according to embodiments D to K, wherein the antibody or antigen-binding fragment comprises at least one CDR sequence or at least one variable heavy chain or light chain sequence as depicted in table 7.

M. The antibody or antigen binding fragment according to any one of the preceding embodiments, wherein the antibody or antigen-binding fragment comprises the heavy and light chain CDR sequences or the variable heavy and light chain sequences of an antibody of table 7.

N. The antibody or antigen binding fragment according to any one of the preceding embodiments comprising
the variable heavy chain CDR sequences as presented by SEQ ID NO: 75-77 and the variable light chain CDR sequences presented by SEQ ID NO: 78-80, or
the variable heavy chain CDR sequences as presented by SEQ ID NO: 5, 9 and 13 and the variable light chain CDR sequences presented by SEQ ID NO: 17, 21 and 25, or
the variable heavy chain CDR sequences as presented by SEQ ID NO: 6, 10 and 14 and the variable light chain CDR sequences presented by SEQ ID NO: 18, 22 and 26, or
the variable heavy chain CDR sequences as presented by SEQ ID NO: 7, 11 and 15 and the variable light chain CDR sequences presented by SEQ ID NO: 19, 23 and 27, or
the variable heavy chain CDR sequences as presented by SEQ ID NO: 8, 12 and 16 and the variable light chain CDR sequences presented by SEQ ID NO: 20, 24 and 28. , or
the variable heavy chain CDR sequences as presented by SEQ ID NO: 45-47 and the variable light chain CDR sequences presented by SEQ ID NO: 48-50, or
the variable heavy chain CDR sequences as presented by SEQ ID NO: 55-57 and the variable light chain CDR sequences presented by SEQ ID NO: 58-60, or
the variable heavy chain CDR sequences as presented by SEQ ID NO: 65-67 and the variable light chain CDR sequences presented by SEQ ID NO: 68-70, or the variable heavy chain CDR sequences as presented by SEQ ID NO: 85-87 and the variable light chain CDR sequences presented by SEQ ID NO: 88-90, or
the variable heavy chain CDR sequences as presented by SEQ ID NO: 95-97 and the variable light chain CDR sequences presented by SEQ ID NO: 98-100, or
the variable heavy chain CDR sequences as presented by SEQ ID NO: 105-107 and the variable light chain CDR sequences presented by SEQ ID NO: 108-110, or
the variable heavy chain CDR sequences as presented by SEQ ID NO: 115-117 and the variable light chain CDR sequences presented by SEQ ID NO: 118-120, or
the variable heavy chain CDR sequences as presented by SEQ ID NO: 125-127 and the variable light chain CDR sequences presented by SEQ ID NO: 128-130, or
the variable heavy chain CDR sequences as presented by SEQ ID NO: 135-137 and the variable light chain CDR sequences presented by SEQ ID NO: 138-140.

O. The antibody or antigen binding fragment according to any one of the preceding embodiments comprising a variable heavy chain sequence as presented by SEQ ID NO: 81 and a variable light chain sequence as presented by SEQ ID NO: 82,
or a variable heavy chain sequence as presented by SEQ ID NO: 33 and a variable light chain sequence as presented by SEQ ID NO: 29,
or a variable heavy chain sequence as presented by SEQ ID NO: 34 and
a variable light chain sequence as presented by SEQ ID NO: 30, or a variable heavy chain sequence as presented by SEQ ID NO: 35 and a variable light chain sequence as presented by SEQ ID NO: 31, or a variable heavy chain sequence as presented by SEQ ID NO: 36 and a variable light chain sequence as presented by SEQ ID NO: 32, or a variable heavy chain sequence as presented by SEQ ID NO: 51 and a variable light chain sequence as presented by SEQ ID NO: 52, or a variable heavy chain sequence as presented by SEQ ID NO: 61 and a variable light chain sequence as presented by SEQ ID NO: 62, or a variable heavy chain sequence as presented by SEQ ID NO: 71 and a variable light chain sequence as presented by SEQ ID NO: 72, or a variable heavy chain sequence as presented by SEQ ID NO: 91 and a variable light chain sequence as presented by SEQ ID NO: 92, or a variable heavy chain sequence as presented by SEQ ID NO: 101 and a variable light chain sequence as presented by SEQ ID NO: 102, or a variable heavy chain sequence as presented by SEQ ID NO: 111 and a variable light chain sequence as presented by SEQ ID NO: 112, or a variable heavy chain sequence as presented by SEQ ID NO: 121 and a variable light chain sequence as presented by SEQ ID NO: 122, or a variable heavy chain sequence as presented by SEQ ID NO: 131 and a variable light chain sequence as presented by SEQ ID NO: 132, or a variable heavy chain sequence as presented by SEQ ID NO: 141 and a variable light chain sequence as presented by SEQ ID NO: 142.

P. The antibody according to any one of the preceding embodiments, which is an IgG antibody.

Q. The antigen-binding fragment according to any one of the preceding embodiments, which is a scFv, Fab, Fab' fragment or a $F(ab')_2$ fragment.

R. The antibody or antigen-binding fragment according to any one of the preceding embodiments, which is a monoclonal antibody or antigen-binding fragment.

S. The antibody or antigen-binding fragment according to any one of the preceding embodiments, which is a human, humanized or chimeric antibody or antigen-binding fragment.

T. An antibody-drug conjugate, comprising an antibody or antigen binding fragment thereof according to embodiments A to S. In a further embodiment the ADC comprises a cytotoxic agent, or a radioisotope, in a further preferred embodiment the cytotoxic agent or radioisotope is covalently linked to the antibody or antigen-binding fragment.

U. An isolated nucleic acid sequence that encodes the antibody or antigen-binding fragment according to embodiments A to S. A further embodiment is a nucleic acid as depicted in table 7.

V. A vector comprising a nucleic acid sequence according to embodiment U.

W. A cell expressing an antibody or antigen-binding fragment according to any one of the embodiments A to S and/or comprising a nucleic acid according to embodiment U or a vector according to embodiment V.

X. A cell according to embodiment W, wherein said cell is a prokaryotic or a eukaryotic cell.

Y. A method of producing an antibody or antigen-binding fragment according to any one of the embodiments A to S comprising culturing of a cell according to embodiment X and purification of the antibody or antigen-binding fragment.

Z. An antibody or antigen-binding fragment according to embodiments A to S or an antibody-drug conjugate according to embodiment T as a medicament.

AA. An antibody or antigen antigen-binding fragment according to embodiments A to S as a diagnostic agent.

BB. An antibody or antigen-binding fragment according to embodiments A to S or an antibody-drug conjugate according to embodiment T as a medicament for the treatment of cancer.

CC. A pharmaceutical composition comprising an antibody or antigen-binding fragment according to embodiments A to S or an antibody-drug conjugate according to embodiment T.

DD. A combination of an antibody or antigen-binding fragment according to embodiments A to S or a pharmaceutical composition according to embodiment CC and one or more therapeutically active compounds.

EE. A method for treating a disorder or condition associated with the undesired presence of C4.4a, comprising administering to a subject in need thereof an effective amount of the pharmaceutical composition according to embodiment CC or a combination according to embodiment DD. In a further preferred embodiment the disease is cancer.

## EXAMPLES

### EXAMPLE 1: Antibody Generation from n-CoDeR Libraries

The isolation of human antibodies against C4.4a was performed by phage display technology employing the naive antibody library n-CoDeR of BioInvent International AB (Lund, Sweden; described in Söderling et al., Nature Biotech. 2000, 18:853-856) in a cell selection approach. n-CoDeR is a Fab library in which all six CDRs are diversified. CDR sequences were initially obtained from healthy human donors.

Briefly, an aliquot of the Fab antibody library was depleted for unwanted surface binders by pre-incubation with $10^7$ non-C4.4a expressing parental CHO-S cells in PBS / 3% FCS / 0.01 % NaN3 (buffer A) at 4°C by end-over-end rotation for 15 min. After that cells were removed by centrifugation, the supernatant was used for 2 additional rounds of pre-incubation on CHO-S cells. Sub sequential panning on target cells ($10^7$ CHO-S:hC4.4a cells) was done by incubation with the phage preparation for 45 min at 4°C in buffer A followed by 10 times washing with buffer A (4°C). Bound phages were eluted by treating the cells during 5 min end-over-end rotation with 76 mM citric acid (4°C). The preparation containing eluted phages was neutralized by addition of 1M Tris/HCl, pH 7.5 and 2 additional rounds of cell panning were performed. In each round eluted phages were propagated and phage titers determined as previously described (Cicortas Gunnarsson et al., PEDS 2004, 17(3) 213-221). Briefly, aliquots of the eluate solution were saved for titration experiments while the rest was used to transform exponentially growing E. coli HB101' for preparation of new phage stocks. For each selection round, both input and output phages were titrated on exponentially growing E. coli HB101 and clones were picked from round 2 and 3 for analysis in Phage ELISA.

***Enzyme-linked immunosorbent assay (ELISA):***

*Phage ELISA:*

Selected phages from different selection rounds were analyzed for specificity using phage ELISA. Briefly, phage expression was performed by adding 10 μl of overnight culture (in LB-medium supplemented with 100 μg/ml ampicillin and 15 μg/ml tetracyclin) to 100 μl fresh medium (LB-medium supplemented with 100 μg/ml ampicillin, 15 μg/ml tetracyclin and 0,1% glucose) and shaking at 250 rpm and 37°C in 96-well MTP until an OD600 of 0.5 was reached. Subsequently helper phage M13KO7 (Invitrogen) was added and samples were incubated for another 15 min at 37°C without shaking. After addition of IPTG (f.c. of 0.25 mM) cells were incubated for 16 h at 30°C while shaking at 200 rpm.

96-well MTP (Nunc-maxisorb) were coated for 16 h at 4°C with recombinant 50 μl C4.4a (5 μg/ml human or mouse protein). The next day plates were washed 3 times with PBS/0.05% Tween 20 (buffer B), treated with blocking reagent (3% milk powder in buffer B), and washed again 3 times with buffer B. After that 50 μl aliquots from phage expressions were transferred per well and incubated for 1 h at 20 °C. After washing 3 times with buffer B anti M13 antibody coupled to HRP (GE Healthcare, 27-9421-01; 1:2500 diluted in buffer B) was added and incubated for 1 h at 20 °C. Color reaction was developed by addition of 50 μl TMB (Invitrogen) and stopped after 5-15 min at 20°C by adding 50 μl stopping solution (Invitrogen). Colorimetric reaction was recorded at 450 nM in a Tecan reader.

*Screening of sFabs by ELISA:*

For the generation of soluble Fabs (sFabs) Phagemid DNA from the selection rounds 2 and 3 was isolated and digested with restriction enzymes EagI and EcoRI according to the providers instructions in order to remove the gene III product. The resulting fragment was re-ligated and constructs were transformed into chemically competent E. coli Top10 using standard methods. A total of -1500 clones were picked, transferred to 96-well plates containing LB-media (100 μg/ml, 0.1% glucose) and shaken at 250 rpm and 37°C until an OD600 of 0.5 was reached. After that sFab production was induced by the addition of IPTG (final concentration 0.5 mM) and incubation was continued for 16 h at 30°C while shaking at 200 rpm. Next morning BEL-buffer was added to each well (24.7 g/l boric acid; 18.7 g/l NaCl; 1.49 g/l EDTA pH 8.0; 2.5 mg/ml lysozyme (Roche)) and 50 μl of the treated cultures were analyzed for sFab binding to the target in an ELISA essentially as described for phages, except that detection was performed with an anti-hIgG (Fab-specific) coupled to HRP (Sigma; Product No. A 0293)

*FACS:*

Cell binding of sFabs to target cells was analyzed on a FACSarray from BD. Briefly 50 μl cell suspension ($2x10^6$ cells/ml) were mixed with 50 μl E. coli supernatant for 1 h at 4°C and 300 rpm. Subsequently 100 μl buffer A was added, cells were centrifuged and washed another 2 times with buffer A. For the detection of bound Fabs R-Phycoerythrin conjugate AffiniPure $F(ab')_2$ fragment goat anti human $F(ab')_2$ fragment specific; Jackson Immuno Research; Product No. 109-116-097, diluted 1:100 in buffer A was added to the cells and incubated for 1 h at 4°C. After 3times washing with buffer A the final pellet was resuspended in 150 μl buffer A and 5000 FACS events were recorded per sample. Data analysis was performed using the BD FACSArray system software.

**EXAMPLE 2: Epitope Grouping**

Epitope grouping experiments were performed using surface plasmon resonance analysis on a Biacore T100 instrument (GE Healthcare Biacore, Inc.) by monitoring simultaneous binding of pairs of anti-C4.4a antibodies to C4.4a. All following steps were performed at 20°C. Approximately 2000 RU (one RU (resonance unit) represents the binding of 1 pg of protein per square mm) of the first antibody was covalently immobilized onto a CM5 sensor chip through primary amine coupling. The chip was activated with an 1:1 ration of 0,4 M N-ethyl-N-(3-diethylaminopropyl) carbodiimide (EDC) and 0.1 M n-hydroxysuccinamide (NHC) at a flow rate of 10μl/min.for 5-15 min.. Unoccupied binding sites on the surface were then blocked with an excess of 1 M ethanolamine pH 8.5. Soluble C4.4a (concentration of 400 nM in HEPES-EP buffer (GE Healthcare Biacore, Inc.) at a flow rate of 10 μl/min for 3 minutes) was captured on the surface via the immobilized antibody,. Therefore, the epitope of the capture antibody is blocked for all bound C4.4a molecules. Subsequently, a second antibody (at concentration of 200 nM in HEPES-EP buffer at a flow rate of 10 μl/min for 3 minutes) was immediately passed over the surface to bind to the captured C4.4a. Two antibodies recognizing the same or overlapping epitopes cannot bind to the C4.4a, whereas antibodies with distinct epitopes are able to bind. The antibody surface was regenerated with 3M MgCl2 (at a flow rate of 10 μl/min for 30 seconds), to remove all bound proteins and then the process was repeated with other antibodies.

Antibodies M31-B01 and M20-D02 S-A in IgG1 format were tested. Both antibodies were unable to bind simultaneously to C4.4a and therefore compete in binding (see Fig 1).

**EXAMPLE 3: Cross-reactivity to murine C4.4a**

Shown in Table 1 are results of Biacore and studies showing cross-reactivity and dissociation constants ($K_D$) of antibodies of the invention to murine C4.4a.

Binding affinities of anti C4.4 antibodies were determined by surface plasmon resonance analysis on a Biacore T100 instrument (GE Healthcare Biacore, Inc.). Antibodies were immobilized onto a CM5 sensor chip through an indirect capturing reagent, anti-human IgG Fc. Reagents from the "Human Antibody Capture Kit" (BR-1008-39, GE Healthcare Biacore, Inc.) were used as described by the manufacturer. Approximately 5000 RU monoclonal mouse anti-human IgG (Fc) antibody were immobilized per cell. Anti C4.4 antibodies were injected at a concentration of 5μg/ml at 10μl/min for 10 sec to reach a capturing level of approximately 200 to 600 RU. Various concentrations (400 nM, 200 nM, 100 nM, 50 nM, 25 nM, 12.5 nM, 6.25 nM, and 3.12 nM) in HEPES-EP buffer (GE Healthcare Biacore, Inc.) of human or murine C4.4a were injected over immobilized anti C4.4a antibodies at a flow rate of 60 μl/min for 3 minutes and the dissociation was allowed for 10 minutes. Sensograms were generated after in-line reference cell correction followed by buffer sample subtraction. The dissociation equilibrium constant (KD) was calculated based on the ratio of association ($k_{on}$) and dissociation rated ($k_{off}$) constants, obtained by fitting sensograms with a first order 1:1 binding model using Biavaluation Software (version 4.0).

**EXAMPLE 4: Binding to recombinant human and murine C4.4a**

Figure 2 depicts binding curves of anti-C4.4 antibody M31-B01 and M20 D02 S-A on recombinant human C4.4a (A) and murine C4.4a (B) respectively. Briefly, 96-well MTP were coated for 16 h at 4°C with recombinant human or mouse C4.4a (100 ng/well plates were washed 3 times with PBS/0.05% Tween 20 (=buffer B) treated with blocking reagent (PBS + 2 % BSA), and washed again 3 times with buffer B. After that anti C4.4a antibodies of the invention were added in concentrations of 0.39 ng to 400 ng /per well and incubated for one hour at 20°C. After washing 3 times with buffer B, 20 ng of Protein A were added in 100 μl buffer B to each well. Color reaction was developed by addition of 50 μl 3,3',5,5'-Tetramethylbenzidine (TMB) (Sigma). Data for $EC_{50}$ determination were recorded after 18 min by measuring extinction 360 nm a Tecan infinite plate reader. $EC_{50}$ values for M31-B01 were 0.24 and 0.29 nM on human and murine C4.4a, respectively. And for M20-D02 S-A $EC_{50}$ values were 0.3 and 0.38 nM on human and murine C4.4a, respectively. This indicates high affinity binding of anti C4.4a antibodies of the invention to both human and murine soluble recombinant C4.4a.

**EXAMPLE 5: Binding to different Tumor cells**

Figure 3 depicts binding curves and $EC_{50}$ values of antibodies M31-B01 and M20-D02 S-A to different tumor cells. Analyses were done by FACS using the transfected cell line A549:hC4.4a and natively C4.4a expressing tumor cell lines NCI H292, NCI H332, H1975/BCRP and BxPC3. Figure 3 f) shows that both antibodies of the invention bound specifically and with high affinities to this wide range of tumor cells. $EC_{50}$ values of further antibodies of the invention on mC4.4a: CHO cells and NCI H292 cells are depicted in Table 8.

FACS titration was performed in a 96 well microtiter plate, in which serial dilutions of the primary antibody in a volume of 50 μl of FACS buffer (3% FCS, in PBS) were mixed with 50 μl of a cell suspension consisting of 2x105 cells/ml which had been detached with Cell Dissociation Solution (1x) Non-enzymatic (Sigma), and resuspended in FACS buffer. Incubation was performed at 4 °C for 1 hour with agitation. Cells were pelleted, washed with FACS buffer and resuspended in 100 μl/well of Phycoerythrin labeled goat anti human IgG (Dianova) solution in FACS buffer. Incubation and washing was performed as before. Analysis of cell-bound antibodies was done at the respective detection wavelength using the FACS Array device. $EC_{50}$ values were determined from fluorescence medians of duplicates using Swift 8.0 software.

**EXAMPLE 6: Internalization**

Relative internalization of anti-C4.4a antibodies on transfected C4.4a:A549 cells is shown in Figure 4 and Table 9. Internalization assays were performed with fluorescence labeled C4.4a antibodies.

The pH-sensitive fluorescent dye CypHer 5E (GE Healthcare, PA15401) was chosen as a fluorescent marker, because only at acidic pH, present in endosomes, a fluorescence signal can be detected, while at neutral or basic pH values no fluorescence is measured. For coupling, anti- C4.4a antibodies were incubated for 1h at 20°C with a 2-molar excess of dye in PBS/Na-Carbonate pH 8.3 (9:1) . On average a Lys-coupled dye load of 1.6 was achieved. Effects of coupling on affinity were tested in FACS Assays and changes of $EC_{50}$ values were considered negligible. $1x10^4$ A549:hC4.4a cells were used to investigate specific C4.4a internalization upon antibody binding. Cells were treated with various concentrations (34nM - 6.6nM) of labeled antibodies at 37°C/ 5% $CO_2$. Internalization was kinetically measured for up to 24 h using the IN Cell Analyzer 1000. Analysis of internalization was performed microscopically at 40 x magnification and by determination of granularity (granule count/cell; 620 nm excitation, and 700 nm emission for CypHer5E). Nuclei were visualized with DNA staining using cell permeable stain Hoechst 33342 (0.5μlg/ml, 30min incubation, emission at 353-365 nm detection at 480 nm).

The corresponding non-C4.4a expressing vector cells were used as controls as well as A549 wild type (A549 wt) cells. A human IgG1 was selected as isotype control and was labeled in parallel. Three independent experiments were carried out; data points represent triplicate determinations. M31-B01 and M20-D02 S-A were internalized efficiently. The time of half maximal internalization was 31 min for M31-B01 and 49 min for M20-D02 S-A, respectively.

**EXAMPLE 7: Conversion from Fab to IgG format**

To transfer VH and VL regions from Fab to IgG format and/or to change the expression system from *E. coli* to a mammalian cell system VH and VL were amplified using PCR primers. Flanking restriction enzyme cleavage sites were introduced at both 5' and 3' ends of VH and VL, respectively. These restriction sites were used for cloning of VH and VL into an expression vector containing i.e. an IgG backbone.

*E. coli* cells were added to 100 μl water in a test tube and incubated at 95°C for 10 min and then kept on ice for 5 min. After vortexing, bacterial debris was pelleted by centrifugation. The supernatant was used for DNA amplification. PCR reactions were prepared separately for VH and VL using specific primer pairs with BamHI and HpaI restriction sites for VL and Blp1 and Mfe1 sites for VH respectively. PCR reactions were performed with AccuPrime Pfx polymerase (Invitrogen # 12344-024) according to the manufacturers instructions. PCR products were quality checked on an 1 % agarose gel. Expression vectors and PCR products were digested for 2 h at 37°C with the respective restriction endonucleases to create compatible ends, according to the providers instructions. The digestion reaction was stopped by incubation at 70°C for 15 min. Resulting fragments were ligated into an expression vector and constructs were transformed into *E. coli* or mammalian cells using standard methods.

**EXAMPLE 8: Subcutaneous Xenograft cancer Model:**

Antitumor effects of anti C4.4a antibodies will be evaluated using subcutaneous xenograft models in immmunodeficient mice. A431 cells are maintained as adherent cultures in DMEM supplemented with 10 % FBS. SCID mice of 6-7 weeks age will be inoculated subcutaneously in the right flank with 1 x 10e7 cells in 0.1 ml of medium. Monoclonal antibodies will be administered i.p. 3x every 3 days at a dose of 5-60 mg/kg. Control mice will be treated with PBS or an irrelevant monoclonal antibody. Tumor size will be measured every 2 days with a sliding caliper. Anti tumor efficacy will be evaluated by comparing tumor size of anti C4.4a antibody treatment versus control treatment.

**EXAMPLE 9: Subcutaneous Xenograft cancer Model with antibody drug conjugates:**

Anti C4.4a antibodies can be conjugated to cytotoxic small molecules using protocols that are known in the art (e.g. Liu et al., Proc Natl. Acad. Sci. (1996), 93, 8618-8623). A431 cells are maintained as adherent cultures in DMEM supplemented with 10 % FBS. SCID mice of 6-7 weeks age will be inoculated subcutaneously in the right flank with 1 x 10e7 cells in 0.1 ml of medium. When tumor sizes reach 50 $mm^3$ antibody drug conjugates will be administered i.p. 3x every 3 days at a dose of 1-60 mg/kg. Control mice will be treated with PBS, an irrelevant monoclonal antibody, or a free unconjugated drug Tumor size will be measured every 2 days with a sliding caliper. Anti tumor efficacy will be evaluated by comparing tumor size of anti C4.4a antibody drug conjugate treatment versus control treatment.

**EXAMPLE 10: Cloning, expression and quantification of Fab antibody fragment expression levels for affinity maturation.**

The heavy and light chain of the wild-type Fabs M31-B01 and M20-D02 S-A carrying a 3xHA-tag and a hexa-histidine tag at the C-terminus of the heavy chain were subcloned into the pET28a bacterial expression vector (Novagen/Merck Chemicals Ltd., Nottingham, UK) and transformed into Top10F' cells (Invitrogen GmbH, Karlsruhe, Germany). Alternatively, other bacterial expression vectors (e.g. pQE vector system, Qiagen GmbH, Hilden, Germany) and strains (e.g. DH5 α, Invitrogen GmbH, Karlsruhe, Germany) can be used. Variants were generated by standard oligo-based site-directed mutagenesis and confirmed by DNA sequencing. In particular, amino acid residues within or surrounding complementary determining regions were modified within the heavy and/or the light chain.

For expression, variants were transformed into the BL21starDE3 Escherichia coli strain (Invitrogen, C6010-03), inoculated into an overnight culture in LB medium including kanamycin (30 μg/ml) and incubated at 37 °C for 18 hours. Expression cultures were generated by inoculating the culture 1:20 into fresh LB medium with kanamycin (30 μg/ml). After 6 hours at 37°C, 1 mM IPTG was added to induce antibody expression and cultures were incubated for additional 18 hours at 30 °C.

For quantification of expression levels an ELISA approach was used. Briefly, MTP plates (Nunc maxisorp black, 460518) were incubated with a HA epitope tag specific mAb (Covance, MMS-101P) diluted in coating buffer (Candor Bioscience GmbH, 121125) at 4°C for 16h, washed with PBST (phosphate buffered saline containing: 137mM NaCl, 2.7mM KCl, 10mM Na2HPO4, 2mM KH2PO4, pH 7.4, 0.05% Tween 20), blocked with 100% Smart Block (Candor Bioscience GmbH, 113500) for 1h at 20°C and washed again. Cultures were diluted in 10% Smart Block in PBST and bound to the MTP plates for 1h at 20°C. After washing with PBST, captured antibodies were incubated with a HRP-coupled anti-lambda antibody (Sigma, A5175) and detected by incubating the plate with 10μM amplex red substrate (Invitrogen, A12222) for 30 minutes at 20°C in the dark followed by fluorescence measurement. Determined quantification signals were checked to be in the dynamic range of an calibration series built up with concentrated parental Fab (M31-B01 or M20-D02-S-A) expression culture supernatant allowing a relative quantification of each individual Fab expression sample.

**EXAMPLE 11: Determination of activity and interspecies cross reactivity of generated Fab antibody fragment variants**

To determine the activity of the mutated Fab variants on recombinant soluble human or mouse C4.4a a direct ELISA assay format was used. Briefly, MTP plates (Nunc maxisorp black, 460518) were coated with 2 μg/ml either human or mouse C4.4a diluted in coating buffer (Candor Bioscience GmbH, 121125) and incubated for 15 h at 4 °C. After washing 3 times with PBST, plates were blocked with 100% Smart Block (Candor Bioscience GmbH, 113500) for 1h at 20°C and the washing steps were repeated. For binding of the Fab antibody fragments 20 μl of culturing supernatants were added to the plates for 1h at 20°C followed by washing. In some cases the washing stringency was increased by an additional incubation step with 10% Smart Block in PBST for 90min followed by washing 3 times with PBST ("process B" in Table 10b). Bound parental Fabs and variants were then detected by a HA-epitope tag specific antibody_HRP conjugate (Bethyl, A190-108P). 10μM amplex red substrate (Invitrogen, A12222) was added to the plates and the fluorescence signal was detected using a common fluorescence reader, e.g. Tecan Ultra. Quantity normalized affinity signals were calculated as ratios of background corrected affinity and quantification signals in the respective ELISA: (Signalaffinity - Backgroundaffinity) / (Signalquantity - Backgroundquantity). The resulting values correlate mainly with the affinity in a positive manner, whereas not normalized values correlate additionally with the concentration of the binding Fab in the sample. Hence, the quantity normalized affinity signals serve as very good guidance for the determination of variants improved in affinity.

**EXAMPLE 12: Single and multiple amino acid substitutions**

Provided in Table l0a and 10b are several examples of single amino acid substitutions generated in the heavy and light chains of M20-D02 S-A. HC D101K, LC A90Q, LC V97A and LC V97G showed the strongest improvement regarding the normalized affinity signals on human C4.4a, with a corresponding signal on mouse C4.4a of at least 1/8 compared with the signal on human C4.4a (Table 10a). Beyond the CDR3s two substitutions, HC D31S and HC V51I showed the strongest improvement regarding the normalized affinity signals on human C4.4a, with a corresponding signal on mouse C4.4a of 30% in the case of HC V51I (Table 10b).

Table 10a. Analysis of single amino acid substitutions within the CDR3 of heavy and light chains of M20-D02 S-A. Normalized affinity signals on human and mouse C4.4a the average and respective standard deviation of two measurements in at least quadruplets are listed.

Tabel 10a:

| | | | | | human | | mouse | |
|---|---|---|---|---|---|---|---|---|
| pos | in SEQ ID NO: | pos | in SEQ ID NO: | Name | average | *stdev* | average | *stdev* |
| 9 | 14 | 105 | 34 | HC S100aR | 0.43 | *0.13* | 0.02 | *0.01* |
| 9 | 14 | 105 | 34 | HC S100aK | 0.45 | *0.21* | 0.06 | *0.01* |
| 10 | 14 | 106 | 34 | HC A100bR | 0.87 | *0.21* | 0.01 | *0.01* |
| 10 | 14 | 106 | 34 | HC A100bS | 0.34 | 0.17 | 0.02 | *0.00* |
| 14 | 14 | 110 | 34 | HC D101E | 0.31 | *0.24* | 0.05 | *0.00* |
| 14 | 14 | 110 | 34 | HC D101K | 1. 65 | *0.48* | 0.21 | *0.09* |
| 14 | 14 | 110 | 34 | HC D101R | 0.61 | *0.18* | 0.03 | *0.01* |
| 3 | 26 | 91 | 30 | LC A90Q | 1.41 | *0.67* | 0.29 | *0.15* |
| 3 | 26 | 91 | 30 | LC A90R | 0.94 | *0.47* | 0.17 | *0.04* |
| 5 | 26 | 93 | 30 | LC D92G | 0.64 | *0.25* | 0.10 | *0.04* |
| 9 | 26 | 97 | 30 | LC N95aW | 0.11 | *0.06* | 0.00 | *0.01* |
| 12 | 26 | 100 | 30 | LC V97A | 1.32 | *0.21* | 0.52 | *0.11* |
| 12 | 26 | 100 | 30 | LC V97G | 1.21 | *0.31* | 0.30 | *0.13* |
| | | | 34 - VH<br>30 - VL | M20-D02 S-A | 0.04 | *0.03* | 0.01 | *0.01* |

Table 10b. Analysis of single amino acid substitutions beyond the CDR3 of heavy and light chains of M20 D02-S-A. Normalized affinity signals on human and murine C4.4a, respectively are listed; given numbers are medians of a measurement in quadruplets. The difference between process B and A is an additional incubation step in buffer for 90 min in process B.

Table 10b:

| | | | | | process A | | process B | |
|---|---|---|---|---|---|---|---|---|
| pos | in SEQ ID NO: | pos | in SEQ ID NO: | name | human | mouse | Human | mouse |
| 3 | 6 | 31 | 34 | HC D31S | | | 0.84 | 0.04 |
| 4 | 10 | 51 | 34 | HC V51I | 0.52 | 0.16 | | |
| 9 | 10 | 56 | 34 | HC A55G | 0.15 | 0.04 | | |
| 10 | 10 | 57 | 34 | HC R56S | 0.21 | 0.06 | | |
| | | | 34 - VH<br>30 - VL | M20-D02 S-A | 0.15 | 0.04 | 0.06 | 0.00 |

Provided in Table 11a and 11b are several examples of single amino acid substitutions generated in the heavy and light chains of B01. LC W91E, LC W91F, LC W91Y and LC G95bR showed the strongest improvement regarding the normalized affinity signals on human C4.4a, with a corresponding signal on mouse C4.4a of at least 1/8 compared with the signal on human C4.4a, the latter three of 40% and more (Table 11a). Beyond the CDR3s the substitution HC A32Y showed the strongest improvement regarding the normalized affinity signals on human C4.4a (Table 11b).
Table 11 a. Analysis of single amino acid substitutions within the CDR3 of heavy and light chains of M31-B01. Normalized affinity signals on human and mouse C4.4a are listed; the average and respective standard deviation of two measurements result from at least quadruplets.

Table 11 a:

| | | | | | human | | mouse | |
|---|---|---|---|---|---|---|---|---|
| pos | in SEQ ID NO: | pos | in SEQ ID NO: | name | average | *stdev* | average | *stdev* |
| 10 | 13 | 106 | 33 | HC Y102K | 1.49 | *0.12* | 0.21 | *0. 02* |
| 10 | 13 | 106 | 33 | HC Y102W | 1.65 | *0.15* | 0.13 | *0. 03* |
| 4 | 25 | 93 | 29 | LC W91E | 3.17 | *0.44* | 0.40 | *0. 02* |
| 4 | 25 | 93 | 29 | LC W91F | 4.30 | *0.78* | 2.53 | *0.39* |
| 4 | 25 | 93 | 29 | LC W91Y | 4.97 | *0.91* | 2.79 | *0. 68* |
| 7 | 25 | 96 | 29 | LC R94M | 1.30 | *0.20* | 0.13 | *0. 02* |
| 9 | 25 | 98 | 29 | LC N95aK | 1.11 | *0.01* | 0.12 | *0. 04* |
| 10 | 25 | 99 | 29 | LC G95bR | 2.45 | *0.49* | 1.01 | *0.12* |
| 11 | 25 | 100 | 29 | LC P96A | 0.95 | 0.47 | 0.32 | *0.16* |
| | | | 33 - VH<br>29 - VL | M31- B01 | 1.11 | *0.34* | 0.13 | *0.07* |

Table 11b. Analysis of single amino acid substitutions beyond the CDR3 of heavy and light chains of B01. Normalized affinity signals on human and mouse C4.4a are listed; given numbers are medians of a measurement in at least triplicates.

Table 11b:

| pos | in SEQ ID NO: | pos | in SEQ ID NO: | name | human | mouse |
|---|---|---|---|---|---|---|
| 3 | 5 | 31 | 33 | HC N31S | 1.53 | 0.32 |
| 4 | 5 | 32 | 33 | HC A32Y | 2.31 | 0.01 |
| 10 | 9 | 57 | 33 | HC T56S | 1.58 | 0.74 |
| 11 | 9 | 58 | 33 | HC I57T | 1.50 | 0.73 |
| 1 | 17 | 23 | 29 | LC T24S | 0.77 | 0.23 |
| 4 | 21 | 55 | 29 | LC K53Q | 1.12 | 0.35 |
| | | | 33 - VH<br>29 - VL | B01 | 1.23 | 0.16 |

Provided in Table 12a and 12b are some examples of combined amino acid substitutions within M20-D02 S-A anti-C4.4a antibodies. Some substitutions were analyzed as single amino acid substitution (Table 10a and 10b), further were only analyzed as part of this combined substitutions. While not every combination is provided in Table 12a and 12b, it is contemplated that the anti-C4.4a antibody may comprise any combination of modifications provided in Table 10a and 10b.

| pos | in SEQ ID NO: | pos | in SEQ ID NO: | name | | D02-S-A | D02-04 | D02-05 | D02-ogl | D02-08 | D02-12 | D02-11 | D02-10 | D02-09 | D02-06 | D02-07 | D02-13 |
|---|---|---|---|---|---|---|---|---|---|---|---|---|---|---|---|---|---|
| 3 | 6 | 31 | 34 | HC | 31 | D | D | S | D | D | D | D | D | D | D | D | S |
| Not in CDR | | 40 | 34 | HC | 40 | T | T | T | T | T | T | T | T | T | T | T | A |
| 4 | 10 | 51 | 34 | HC | 51 | V | I | I | V | I | I | I | I | I | I | I | I |
| 9 | 10 | 56 | 34 | HC | 55 | A | G | G | A | G | G | G | G | G | G | G | G |
| 10 | 10 | 57 | 34 | HC | 56 | R | S | S | R | S | S | S | S | S | S | S | S |
| 9 | 14 | 105 | 34 | HC | 100 a | S | S | S | S | S | K | S | K | S | S | K | K |
| 10 | 14 | 106 | 34 | HC | 100 b | A | A | A | S | S | S | S | S | S | S | S | S |
| 14 | 14 | 110 | 34 | HC | 101 | D | D | D | K | K | K | K | K | K | K | K | K |
| 15 | 14 | 111 | 34 | HC | 102 | S | S | S | S | Y | S | S | S | S | S | S | S |

| pos | in SEQ ID NO: | pos | in SEQ ID NO: | name | | D02-S-A | D02-04 | D02-05 | D02-ogl | D02-08 | D02-12 | D02-11 | D02-10 | D02-09 | D02-06 | D02-07 | D02-13 |
|---|---|---|---|---|---|---|---|---|---|---|---|---|---|---|---|---|---|
| 7 | 18 | 29 | 30 | LC | 30 | V | I | I | V | I | I | I | I | I | I | I | I |
| 7 | 26 | 95 | 30 | LC | 94 | R | R | R | R | R | S | R | R | S | R | S | S |
| 9 | 26 | 97 | 30 | LC | 95A | N | N | N | N | N | N | S | S | N | S | S | S |
| 12 | 26 | 100 | 30 | LC | 97 | V | G | G | G | A | G | G | G | G | A | A | A |

Provided in Table 13a and 13b are some examples of combined amino acid substitutions within M31-B01 anti-C4.4a antibodies. Some substitutions were analyzed as single amino acid substitution (Table 11a and 11b), further were only analyzed as part of this combined substitutions. While not every combination is provided in Table 13a and 13b, it is contemplated that the anti-C4.4a antibody may comprise any combination of modifications provided in Table 11a and 11b.

Table 13a and 13b. Example of multiple amino acid substitutions within M31-B01.

| pos | in SEQ ID NO: | pos | in SEQ ID NO: | name | | B01 | B01-03 | B01-04 | B01-02 | B01-nn1 | B01-08 | B01-09 | B01-nn2 | B01-nn3 | B01-07 | B01-05 | B01-10 | B01-06 | B01-nn4 | B01-12 | B01-nn5 | B01-11 |
|---|---|---|---|---|---|---|---|---|---|---|---|---|---|---|---|---|---|---|---|---|---|---|
| **3** | **5** | 31 | 33 | **HC** | **31** | N | N | N | S | N | N | S | N | N | N | N | S | S | S | S | S | S |
| **10** | **9** | 57 | 33 | **HC** | **56** | T | T | T | S | T | T | T | T | T | T | T | T | S | S | S | S | S |
| **11** | **9** | 58 | 33 | **HC** | **57** | I | I | I | T | I | I | I | I | I | I | I | I | T | T | T | T | T |
| **10** | **13** | 106 | 33 | **HC** | **102** | Y | Y | Y | Y | K | K | K | K | K | Y | Y | Y | G | W | K | N | K |

| pos | in SEQ ID NO: | pos | in SEQ ID NO: | name | | B01 | B01-03 | B01-04 | B01-02 | B01-nn1 | B01-08 | B01-09 | B01-nn2 | B01-nn3 | B01-07 | B01-05 | B01-10 | B01-06 | B01-nn4 | B01-12 | B01-nn5 | B01-11 |
|---|---|---|---|---|---|---|---|---|---|---|---|---|---|---|---|---|---|---|---|---|---|---|
| **Not in CDR** | | 10 | 29 | **LC** | **11** | A | V | V | A | A | A | A | V | V | V | V | V | A | A | A | A | A |
| **Not in CDR** | | 13 | 29 | **LC** | **14** | T | A | A | T | T | T | T | A | A | A | A | A | T | T | T | T | T |
| **1** | **17** | 23 | 29 | **LC** | **24** | T | T | T | S | T | T | T | T | T | T | T | T | S | S | S | S | S |
| **4** | **21** | 55 | 29 | **LC** | **53** | K | K | K | Q | K | K | K | K | K | K | K | K | Q | Q | Q | Q | Q |
| **4** | **25** | 93 | 29 | **LC** | **91** | W | W | Y | W | Y | F | F | F | Y | F | Y | Y | Y | Y | F | Y | F |
| **7** | **25** | 96 | 29 | **LC** | **94** | R | R | R | R | S | R | R | S | S | S | S | S | S | S | R | S | S |
| **9** | **25** | 98 | 29 | **LC** | **95A** | N | N | N | N | K | N | N | N | S | N | S | S | N | S | S | N | N |
| **10** | **25** | 99 | 29 | **LC** | **95B** | G | G | G | G | G | G | G | G | G | G | G | G | R | G | G | G | G |

Provided in Table 14a and 14b are CDR sequence stretches with potential deamidation sites, specifically Asn-Gly ("NG") and Asn-Ala ("NA") in M20-D02 S-A and M31-B01, as well as the respective sequences of some corresponding variants with multiple amino acid substitutions.
In M20 D02-S-A there are two potential deamidation sites in CDRs, one in CDR-L3 and one in CDR-H2. In D02-11, -10, -06, -07 and -13 the site in CDR-L3 is removed by an N>S substitution.

Table 14a:

| pos | in SEQ ID NO: | pos | in SEQ ID NO: | name | | D02-S-A | D02-04 | D02-05 | D02-ogl | D02-08 | D02-12 | D02-11 | D02-10 | D02-09 | D02-06 | D02-07 | D02-13 |
|---|---|---|---|---|---|---|---|---|---|---|---|---|---|---|---|---|---|
| **6** | **26** | 94 | 30 | **LC** | **93** | D | D | D | D | D | D | D | D | D | D | D | D |
| **7** | **26** | 95 | 30 | **LC** | **94** | R | R | R | R | R | S | R | R | S | R | S | S |
| **8** | **26** | 96 | 30 | **LC** | **95** | L | L | L | L | L | L | L | L | L | L | L | L |
| **9** | **26** | 97 | 30 | **LC** | **95A** | **N** | **N** | **N** | **N** | **N** | **N** | S | S | **N** | S | S | S |
| **10** | **26** | 98 | 30 | **LC** | **95B** | **G** | **G** | **G** | **G** | **G** | **G** | G | G | **G** | G | G | G |
| **11** | **26** | 99 | 30 | **LC** | **96** | W | W | W | W | W | W | W | W | W | W | W | W |
| **12** | **26** | 100 | 30 | **LC** | **97** | V | G | G | G | A | G | G | G | G | A | A | A |
| **4** | **10** | 51 | 34 | **HC** | **51** | V | I | I | V | I | I | I | I | I | I | I | I |
| **5** | **10** | 52 | 34 | **HC** | **52** | S | S | S | S | S | S | S | S | S | S | S | S |
| **6** | **10** | 53 | 34 | **HC** | **52a** | W | W | W | W | W | W | W | W | W | W | W | W |
| **7** | **10** | 54 | 34 | **HC** | **53** | **N** | **N** | **N** | **N** | **N** | **N** | **N** | **N** | **N** | **N** | **N** | **N** |
| **8** | **10** | 55 | 34 | **HC** | **54** | **G** | **G** | **G** | **G** | **G** | **G** | **G** | **G** | **G** | **G** | **G** | **G** |
| **9** | **10** | 56 | 34 | **HC** | **55** | A | G | G | A | G | G | G | G | G | G | G | G |
| **10** | **10** | 57 | 34 | **HC** | **56** | R | S | S | R | S | S | S | S | S | S | S | S |

In M31-B01 there are two potential deamidation sites in CDRs, one in CDR-L3 and one in CDR-H1. In B01-nn3, -05, -10, -nn4 and -12 the site in CDR-L3 is removed by a N>S substitution, in B01-nn1 by a N>K substitution and in B01-06 by a G>R substitution. In B01-02, -09, -10, -06, -nn4, -12, -nn5 and -11 the site in CDR-H1 is removed by aN>S substitution. B01-10, -06, -nn4 and -12 show none of these both potential deamidations sites.

Table 14b:

| **pos** | **in SEQ ID NO:** | pos | in SEQ ID NO: | name | | **B01** | **B01-03** | **B01-04** | **B01-02** | **B01-nn1** | **B01-08** | **B01-09** | **B01-nn2** | **B01-nn3** | **B01-07** | **B01-05** | **B01-10** | **B01-06** | **B01-nn4** | **B01-12** | **B01-nn5** | **B01-11** |
|---|---|---|---|---|---|---|---|---|---|---|---|---|---|---|---|---|---|---|---|---|---|---|
| **6** | **25** | 95 | 29 | **LC** | **93** | D | D | D | D | D | D | D | D | D | D | D | D | D | D | D | D | D |
| **7** | **25** | 96 | 29 | **LC** | **94** | R | R | R | R | S | R | R | S | S | S | S | S | S | S | R | S | S |
| **8** | **25** | 97 | 29 | **LC** | **95** | L | L | L | L | L | L | L | L | L | L | L | L | L | L | L | L | L |
| **9** | **25** | 98 | 29 | **LC** | **95A** | **N** | **N** | **N** | **N** | K | **N** | **N** | **N** | S | **N** | S | S | N | S | S | **N** | **N** |
| **10** | **25** | 99 | 29 | **LC** | **95B** | **G** | **G** | **G** | **G** | G | **G** | **G** | **G** | G | **G** | G | G | R | G | G | **G** | **G** |
| **11** | **25** | 100 | 29 | **LC** | **96** | P | P | P | P | P | P | P | P | P | P | P | P | P | P | P | P | P |
| **12** | **25** | 101 | 29 | **LC** | **97** | V | V | V | V | V | V | V | V | V | V | V | V | V | V | V | V | V |
| **1** | **5** | 29 | 33 | **HC** | **29** | F | F | F | F | F | F | F | F | F | F | F | F | F | F | F | F | F |
| **2** | **5** | 30 | 33 | **HC** | **30** | S | S | S | S | S | S | S | S | S | S | S | S | S | S | S | S | S |
| **3** | **5** | 31 | 33 | **HC** | **31** | **N** | **N** | **N** | S | **N** | **N** | S | **N** | **N** | **N** | **N** | S | S | S | S | S | S |
| **4** | **5** | 32 | 33 | **HC** | **32** | **A** | **A** | **A** | A | **A** | **A** | A | **A** | **A** | **A** | **A** | A | A | A | A | A | A |
| **5** | **5** | 33 | 33 | **HC** | **33** | W | W | W | W | W | W | W | W | W | W | W | W | W | W | W | W | W |
| **6** | **5** | 34 | 33 | **HC** | **34** | M | M | M | M | M | M | M | M | M | M | M | M | M | M | M | M | M |

The methods described in examples 10-12 were used to generate affinity matured antibodies by single mutations and combinations of single mutations thereof. These methods are well suitable for generation of competing antibodies, derived from a parental antibody. The antibodies of the above mentioned examples are depicted in table 7. These examples provide further C4.4a binding antibodies or antigen-fragment-binding fragments which comprise CDR H1 sequences that are at least 77% identical to the CDR H1 of M31 B01, CDR H2 sequences that are at least 90% identical to the CDR H2 of M31 B01, CDR H3 sequences that are at least 90% identical to the CDR H3 of M31 B01, CDR L1 sequences that are at least 92% identical to the CDR L1 of M31 B01, CDR L2 sequences that are at least 85% identical to the CDR L2 of M31 B01 and CDR L3 sequences that are at least 58% identical to the CDR L3 of M31 B01 (those antibody variants compete in binding with M31 B01) and antibodies or antigen-fragment-binding proteins which comprise CDR H1 sequences that are at least 88% identical to the CDR H1 of M20 D02 S-A, CDR H2 sequences that are at least 85% identical to the CDR H2 of M20 D02 S-A, CDR H3 sequences that are at least 73% identical to the CDR H3 of M20 D02 S-A, CDR L1 sequences that are at least 92% identical to the CDR L1 of M20 D02 S-A, CDR L2 sequences that are at least 100% identical to the CDR L2 of M20 D02 S-A and CDR L3 sequences that are at least 58% identical to the CDR L3 M20 D02 S-A (those antibody variants compete in binding with M20 D02 S-A).

Table 15a: This table summarizes the amino acid variations of CDRs 1-3 of heavy and light chains for M20 D02 S-A as described in method 12.

The following represents the above results in form of a consensus sequence of the respective CDR sequences derived from M20 D02 S-A. The consensus for CDR H1 is depicted in Table 15a (i) (SEQ ID NO: 297), for CDR H2 is depicted in Table 15a (ii) (SEQ ID NO: 298), for CDR H3 is depicted in Table 15a (iii) (SEQ ID NO: 299), for CDR L1 is depicted in Table 15a (iv) (SEQ ID NO: 300), for CDR L2 is identical to SEQ ID NO: 22 (Table 15a (v)), for CDR L3 is depicted in Table 15a (v) (SEQ ID NO: 301), respectively.

Table 15a: This table summarizes the amino acid variations of CDRs 1-3 of heavy and light chains for M20 D02 S-A as described in example 12

**(i) CDR H1 M20 D02 S-A (SEQ ID NO:6)**

| position | 1 | 2 | 3 | 4 | 5 | 6 | 7 | 8 | 9 |
|---|---|---|---|---|---|---|---|---|---|
| amino acid | F | S | D | Y | Q | M | T | W | I |
| variant | | | S | | | | | | |
| consensus | F | S | D or S | Y | Q | M | T | W | I |

**(ii) CDR H2 M20 D02 S-A (SEQ ID NO:10)**

| position | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| amino acid | V | S | G | V | S | W | N | G | A | R | T | H | Y | A | D | S | V | k | G | R |
| variant | | | | I | | | | | G | S | | | | | | | | | | |
| consensus | V | S | G | V or I | S | W | N | G | A or G | R or S | T | H | Y | A | D | S | V | k | G | R |

**(iii) CDR H3 M20 D02 S-A (SEQ ID NO:14)**

| position | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| amino acid | A | K | G | D | Y | L | V | Y | S | A | Y | Y | F | D | S |
| variant | | | | | | | | | K or R | S or R | | | | K, E or R | Y |
| consensus | A | K | G | D | Y | L | V | Y | S, K or R | A, S or R | Y | Y | F | D, K, E or R | S or Y |

**(iv) CDR L1 M20 D02 S-A (SEQ ID NO:18)**

| position | 1 | 2 | 3 | 4 | 5 | 6 | 7 | 8 | 9 | 10 | 11 | 12 | 13 |
|---|---|---|---|---|---|---|---|---|---|---|---|---|---|
| amino acid | S | G | S | S | S | N | V | G | S | N | P | V | N |
| variant | | | | | | | I | | | | | | |
| consensus | S | G | S | S | S | N | V or I | G | S | N | P | V | N |

**(v) CDR L2 M20 D02 S-A (SEQ ID NO:22)**

| position | 1 | 2 | 3 | 4 | 5 | 6 | 7 |
|---|---|---|---|---|---|---|---|
| amino acid | R | N | N | Q | R | P | S |
| variant | | | | | | | |
| consensus | R | N | N | Q | R | P | S |

**(vi) CDR L3 M20 D02 S-A (SEQ ID NO:26)**

| position | 1 | 2 | 3 | 4 | 5 | 6 | 7 | 8 | 9 | 10 | 11 | 12 |
|---|---|---|---|---|---|---|---|---|---|---|---|---|
| amino acid | C | A | A | W | D | D | R | L | N | G | W | V |
| variant | | | Q or R | | G | | S | | W or S | | | A or G |
| consensus | C | A | A, Q or R | W | D or G | D | R or S | L | N, W or S | G | W | V, A or G |

The following represents the above results in form of a consensus sequence of the respective CDR sequences derived from M31 B01. The consensus for CDR H1 is depicted in Table 15b (i) (SEQ ID NO: 302), for CDR H2 is depicted in Table 15b (ii) (SEQ ID NO: 303), for CDR H3 is depicted in Table 15b (iii) (SEQ ID NO: 304), for CDR L1 is depicted in Table 15b (iv) (SEQ ID NO: 305), for CDR L2 is depicted in Table 15b (v) (SEQ ID NO: 306), for CDR L3 is depicted in Table 15b (v) (SEQ ID NO: 307), respectively.

Table 15b: This table summarizes the amino acid variations of CDRs 1-3 of heavy and light chains for M31 B01 as described in example 12

**(i) CDR H1 M31 B01 (SEQ ID NO:5)**

| position | 1 | 2 | 3 | 4 | 5 | 6 | 7 | 8 | 9 |
|---|---|---|---|---|---|---|---|---|---|
| amino acid | F | S | N | A | W | M | S | W | V |
| variant | | | S | Y | | | | | |
| consensus | F | S | N or S | A or Y | W | M | S | W | V |

**(ii) CDR H2 M31 B01 (SEQ ID NO:9)**

| position | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| amino acid | V | S | Y | I | S | S | S | G | S | T | I | Y | Y | A | D | S | V | K | G | R |
| variant | | | | | | | | | | S | T | | | | | | | | | |
| consensus | V | S | Y | I | S | S | S | G | S | T or S | I or T | Y | Y | A | D | S | V | K | G | R |

**(iii) CDR H3 M31 B01 (SEQ ID NO:13)**

| position | 1 | 2 | 3 | 4 | 5 | 6 | 7 | 8 | 9 | 10 |
|---|---|---|---|---|---|---|---|---|---|---|
| amino acid | A | R | E | G | L | W | A | F | D | Y |
| variant | | | | | | | | | | K, G, W or N |
| consensus | A | R | E | G | L | W | A | F | D | Y, K, G, W or N |

**(iv) CDR L1 M31 B01 (SEQ ID NO:17)**

| position | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| amino acid | T | G | S | S | S | N | I | G | A | G | Y | V | V | H |
| variant | S | | | | | | | | | | | | | |
| consensus | T or S | G | S | S | S | N | I | G | A | G | Y | V | V | H |

**(v) CDR L2 M31 B01 (SEQ ID NO:21)**

| position | 1 | 2 | 3 | 4 | 5 | 6 | 7 |
|---|---|---|---|---|---|---|---|
| amino acid | D | N | N | K | R | P | S |
| variant | | | | Q | | | |
| consensus | D | N | N | K or Q | R | P | S |

**(vi) CDR L3 M31 B01 (SEQ ID NO:25)**

| position | 1 | 2 | 3 | 4 | 5 | 6 | 7 | 8 | 9 | 10 | 11 | 12 |
|---|---|---|---|---|---|---|---|---|---|---|---|---|
| amino acid | C | A | A | W | D | D | R | L | N | G | P | V |
| variant | | | | E, F or Y | | | S or M | | K or S | R | A | |
| consensus | C | A | A | W, E, F or Y | D | D | R, S or M | L | N, K or S | G or R | P or A | V |

**EXAMPLE 13: Determination of antibody binding to C4.4a single domains by Western Blot**

To characterize the antibody binding domain of M31-B01 and M20-D02 S-A constructs consisting of C4.4a domain S1 amino acids 1-85 and C4.4 domain S2 108-193 of human C4.4a SEQ ID NO: 1 respectively, were generated as C-terminal human IgG1 Fc-6xHisTag fusions, cloned into a mammalian expression vector containing a CMV5 promotor and expressed transiently in HEK293 6E cells by standard methods. The expressed S1 and S2 proteins were then purified from cell supernatants by metal chelate chromatography using a 5 ml NiNTA superflow column (Qiagen) at 20°C. The samples were pH adjusted to pH 8.0 with 1M NaOH and then applied to the column previously equilibrated with 50mM NaH2PO4 + 300mM NaCl pH 8.0 at 1ml /min. The column was washed with 15 CV of equilibration buffer and bound HisTagged proteins were eluted with 50mM NaH2PO4 + 300mM NaCl + 250mM Imidazol pH 8.0. The eluted proteins were further purified on a Tricorn Superdex 75 10/300 GL (GE Healthcare) by size exclusion chromatography.

For further analysis full length (C4.4a SEQ ID NO: 1), the purified S1-Fc-6xHisTag fusion and S2 Fc-6xHisTag were applied to different lanes of a precast NuPage Bis-Tris 4 - 12% gradient gel (Invitrogen, No. NP0322Box) and separated by electrophoresis, using the NuPage MES SDS Running Puffer (Invitrogen NP0002) according to the manufacturer's instructions.

The SDS gel was then blotted to a iblot gel transfer membrane (Invitrogen IB3010-02) using an iblot device (Invitrogen). The membrane was blocked in DPBS pH 7.4 + 4 % milk powder + 0.1 % Tween 20 for 16 h at 4°C, followed by three washing steps for 5 min at 20°C in DPBS pH 7.4 + 0.1 % Tween 20. M31-B01 (5.76 mg/ml) or M20-D02 S-A (3.49mg/ml) were applied in dilutions of 1/5000 and 1/2000 respectively and incubated for 1.5 h at 20°C, followed by 2x washes in DPBS pH 7.4 + 0.1 % Tween 20. The second antibody (alk. Phosphatase. Conjugate Goat anti human IgG, Fab specific; 109-055-097 Jackson ImmunoResearch) was incubated at a dilution of 1/1000 for 1.5 h at 20°C. Followed by 3 washing steps for 5 min at 20°C in DPBS pH 7.4 + 0.1 % Tween 20. Staining was performed with Sigma Fast BCIP/NBT / 1 tablet in 10 ml water at 20°C till the bands became visible. Staining reaction was stopped by washing with water.

Both antibodies M31-B01 and M20-D02 S-A bind to full length human and mouse C4.4a. in addition they bind to domain S1 of C4.4a but not to domain S2 of C4.4a as depicted in Fig. 6. Binding can be exclusively detected in non DTT reduced samples, strongly indicating presence of a conformation dependent epitope which is stabilized by one or more disulfide bridges.

**EXAMPLE 14: Antibody dependent cell mediated cytotoxicity assays (ADCC assays)**

Anti tumor activity of anti-C4.4a IgGs can be mediated by ADCC. C4.4a expressing A549:hC4.4a cells and non C4.4a expressing parental cells are incubated with 250 ng/ml, 1000 ng/ml or 2000 ng/ml human anti-C4.4a or control IgG1 antibody (e.g. anti-digoxin antibody). Human PBMCs are added to theses cells at effector-target ratios of 50:1, 25:1 and 5:1 ratios. A chromium-51 release assay is performed to determine the level of target lysis. If the rate of lysis in the presence of anti-C4.4a antibody and PBMCs is higher than the rate of (spontaneous) lysis in the presence of mock antibody or no antibody and PBMCs this indicates ADCC is effective.

**EXAMPLE 15: Antibody dependent inhibition of proliferation - Cell Proliferation measured by the xCELLigence System**

The xCELLigence System (RTCA analyzer CatNo.05228972001, Roche) monitors cellular events in real time without the incorporation of labels. The system measures electrical impedance across interdigitated micro-electrodes integrated on the bottom of tissue culture E-Plates. Thee impedance measurement provides quantitative information about the biological status of the cells, including cell number, viability and morphology. To determine the rate of cell proliferation of A549:hC4.4a cells were incubated either with of an non-binding hIgG1 isotype control antibody or 100 nM of B01-3 (as hIgG1). First 50μl of cell culture medium (RPMI (Biochrom FG1640) + 10 % FCS (Biochrom #S0145) + 1 μg/ml Puromycin (Sigma 8833)) was added to each well of an E-Plate 96. The E-Plate 96 was inserted into the RTCA MP Station (CatNo.05331625001, Roche) and the background impedance of each well was determined. Solutions where removed again from the E-Plate 96 and 50μl of cell suspension (5000 cells/well of A549:hC4.4a in cell culture medium) were added to the wells. Plates were reinserted and measured for 4 hours at 37°C + 5 % CO2. The E-Plate 96 was removed again and 100μl of antibodies (100nM of B01-3 or non binding control hIgG1) in cell culture medium were added. All samples were run in triplicates. After reinsertion of the E-plate the measurement was conducted for 92 hours at 37°C + 5 % $CO_2$. Results were analyzed with the built-in software package. Results are depicted in figure 6. Antibody B01-3 clearly decreases cell proliferation compared to a non-binding control antibody. This shows that the antibodies of the invention effectively inhibit cellular proliferation of C4.4a expressing cells.

Table 7: Sequences of the antibodies

| Antibody | SEQ ID NO: HCDR1 | SEQ ID NO: HCDR2 | SEQ ID NO: HCDR3 | SEQ ID NO : LCDR1 | SEQ ID NO: LCDR2 | SEQ ID NO: LCDR3 | SEQ ID NO: VH Protein | SEQ ID NO: VL Protein | SEQ ID NO: VH Nucleotide | SEQ ID NO: VL Nucleotide |
|---|---|---|---|---|---|---|---|---|---|---|
| M31-B01 | 5 | 9 | 13 | 17 | 21 | 25 | 33 | 29 | 41 | 37 |
| M20-D02 S-A | 6 | 10 | 14 | 18 | 22 | 26 | 34 | 30 | 42 | 38 |
| M60-G03 | 7 | 11 | 15 | 19 | 23 | 27 | 35 | 31 | 43 | 39 |
| M36-H02 | 8 | 12 | 16 | 20 | 24 | 28 | 36 | 32 | 44 | 40 |
| B01-3 | 45 | 46 | 47 | 48 | 49 | 50 | 51 | 52 | 53 | 54 |
| B01-5 | 55 | 56 | 57 | 58 | 59 | 60 | 61 | 62 | 63 | 64 |
| B01-7 | 65 | 66 | 67 | 68 | 69 | 70 | 71 | 72 | 73 | 74 |
| B01-10 | 75 | 76 | 77 | 78 | 79 | 80 | 81 | 82 | 83 | 84 |
| B01-12 | 85 | 86 | 87 | 88 | 89 | 90 | 91 | 92 | 93 | 94 |
| D02-4 | 95 | 96 | 97 | 98 | 99 | 100 | 101 | 102 | 103 | 104 |
| D02-6 | 105 | 106 | 107 | 108 | 109 | 110 | 111 | 112 | 113 | 114 |
| D02-7 | 115 | 116 | 117 | 118 | 119 | 120 | 121 | 122 | 123 | 124 |
| D02-11 | 125 | 126 | 127 | 128 | 129 | 130 | 131 | 132 | 133 | 134 |
| D02-13 | 135 | 136 | 137 | 138 | 139 | 140 | 141 | 142 | 143 | 144 |
| B01-nn1 | 145 | 146 | 147 | 148 | 149 | 150 | 151 | 152 | 308 | 309 |
| B01-nn2 | 153 | 154 | 155 | 156 | 157 | 158 | 159 | 160 | 310 | 311 |
| B01-nn3 | 161 | 162 | 163 | 164 | 165 | 166 | 167 | 168 | 312 | 313 |
| B01-nn4 | 169 | 170 | 171 | 172 | 173 | 174 | 175 | 176 | 314 | 315 |
| B01-nn5 | 177 | 178 | 179 | 180 | 181 | 182 | 183 | 184 | 316 | 317 |
| B01-2 | 185 | 186 | 187 | 188 | 189 | 190 | 191 | 192 | 318 | 319 |
| B01-4 | 193 | 194 | 195 | 196 | 197 | 198 | 199 | 200 | 320 | 321 |
| B01-6 | 201 | 202 | 203 | 204 | 205 | 206 | 207 | 208 | 322 | 323 |
| B01-8 | 209 | 210 | 211 | 212 | 213 | 214 | 215 | 216 | 324 | 325 |
| B01-9 | 217 | 218 | 219 | 220 | 221 | 222 | 223 | 224 | 326 | 327 |
| B01-11 | 225 | 226 | 227 | 228 | 229 | 230 | 231 | 232 | 328 | 329 |
| B01-12 | 233 | 234 | 235 | 236 | 237 | 238 | 239 | 240 | 330 | 331 |
| D02-ogl | 241 | 242 | 243 | 244 | 245 | 246 | 247 | 248 | 332 | 333 |
| D02-5 | 249 | 250 | 251 | 252 | 253 | 254 | 255 | 256 | 334 | 335 |
| D02-8 | 257 | 258 | 259 | 260 | 261 | 262 | 263 | 264 | 336 | 337 |
| D02-9 | 265 | 266 | 267 | 268 | 269 | 270 | 271 | 272 | 338 | 339 |
| D02-10 | 273 | 274 | 275 | 276 | 277 | 278 | 279 | 280 | 340 | 341 |
| D02-11 | 281 | 282 | 283 | 284 | 285 | 286 | 287 | 288 | 342 | 343 |
| D02-12 | 289 | 290 | 291 | 292 | 293 | 294 | 295 | 296 | 344 | 345 |

## Claims

1. An isolated antibody or an antigen-binding fragment thereof specifically binding to C4.4a wherein the antibody or the antigen-binding fragment thereof comprises:

   a) the heavy chain CDR sequences conforming to SEQ ID NO: 297 (CDR H1), SEQ ID NO: 298 (CDR H2) and SEQ ID NO: 299 (CDR H3), and the light chain CDR sequences conforming to SEQ ID NO: 300 (CDR L1), SEQ ID NO: 22 (CDR L2) and SEQ ID NO: 301 (CDR L3), or
   b) the heavy chain CDR sequences conforming to SEQ ID NO: 302 (CDR H1), SEQ ID NO: 303 (CDR H2) and SEQ ID NO: 304 (CDR H3), and the light chain CDR sequences conforming to SEQ ID NO: 305 (CDR L1), SEQ ID NO: 306 (CDR L2) and SEQ ID NO: 307 (CDR L3).

2. The antibody or the antigen-binding fragment according to claim 1 comprising the variable heavy chain CDR sequences as presented by SEQ ID NO: 75-77 and the variable light chain CDR sequences presented by SEQ ID NO: 78-80, or
   the variable heavy chain CDR sequences as presented by SEQ ID NO: 5, 9 and 13 and the variable light chain CDR sequences presented by SEQ ID NO: 17, 21 and 25, or
   the variable heavy chain CDR sequences as presented by SEQ ID NO: 6, 10 and 14 and the variable light chain CDR sequences presented by SEQ ID NO: 18, 22 and 26, or
   the variable heavy chain CDR sequences as presented by SEQ ID NO: 7, 11 and 15 and the variable light chain CDR sequences presented by SEQ ID NO: 19, 23 and 27, or
   the variable heavy chain CDR sequences as presented by SEQ ID NO: 8, 12 and 16 and the variable light chain CDR sequences presented by SEQ ID NO: 20, 24 and 28. , or
   the variable heavy chain CDR sequences as presented by SEQ ID NO: 45-47 and the variable light chain CDR sequences presented by SEQ ID NO: 48-50, or
   the variable heavy chain CDR sequences as presented by SEQ ID NO: 55-57 and the variable light chain CDR sequences presented by SEQ ID NO: 58-60, or
   the variable heavy chain CDR sequences as presented by SEQ ID NO: 65-67 and the variable light chain CDR sequences presented by SEQ ID NO: 68-70, or
   the variable heavy chain CDR sequences as presented by SEQ ID NO: 85-87 and the variable light chain CDR sequences presented by SEQ ID NO: 88-90, or
   the variable heavy chain CDR sequences as presented by SEQ ID NO: 95-97 and the variable light chain CDR sequences presented by SEQ ID NO: 98-100, or the variable heavy chain CDR sequences as presented by SEQ ID NO: 105-107 and the variable light chain CDR sequences presented by SEQ ID NO: 108-110, or

the variable heavy chain CDR sequences as presented by SEQ ID NO: 115-117 and the variable light chain CDR sequences presented by SEQ ID NO: 118-120, or
the variable heavy chain CDR sequences as presented by SEQ ID NO: 125-127 and the variable light chain CDR sequences presented by SEQ ID NO: 128-130, or
the variable heavy chain CDR sequences as presented by SEQ ID NO: 135-137 and the variable light chain CDR sequences presented by SEQ ID NO: 138-140.

**3.** The antibody or the antigen-binding fragment according to any one of the preceding claims comprising
a variable heavy chain sequence as presented by SEQ ID NO: 81 and a variable light chain sequence as presented by SEQ ID NO: 82,
or a variable heavy chain sequence as presented by SEQ ID NO: 33 and a variable light chain sequence as presented by SEQ ID NO: 29,
or a variable heavy chain sequence as presented by SEQ ID NO: 34 and a variable light chain sequence as presented by SEQ ID NO: 30,
or a variable heavy chain sequence as presented by SEQ ID NO: 35 and a variable light chain sequence as presented by SEQ ID NO: 31,
or a variable heavy chain sequence as presented by SEQ ID NO: 36 and a variable light chain sequence as presented by SEQ ID NO: 32,
or a variable heavy chain sequence as presented by SEQ ID NO: 51 and a variable light chain sequence as presented by SEQ ID NO: 52,
or a variable heavy chain sequence as presented by SEQ ID NO: 61 and a variable light chain sequence as presented by SEQ ID NO: 62,
or a variable heavy chain sequence as presented by SEQ ID NO: 71 and a variable light chain sequence as presented by SEQ ID NO: 72,
or a variable heavy chain sequence as presented by SEQ ID NO: 91 and a variable light chain sequence as presented by SEQ ID NO: 92,
or a variable heavy chain sequence as presented by SEQ ID NO: 101 and a variable light chain sequence as presented by SEQ ID NO: 102,
or a variable heavy chain sequence as presented by SEQ ID NO: 111 and a variable light chain sequence as presented by SEQ ID NO: 112,
or a variable heavy chain sequence as presented by SEQ ID NO: 121 and a variable light chain sequence as presented by SEQ ID NO: 122,
or a variable heavy chain sequence as presented by SEQ ID NO: 131 and a variable light chain sequence as presented by SEQ ID NO: 132,
or a variable heavy chain sequence as presented by SEQ ID NO: 141 and a variable light chain sequence as presented by SEQ ID NO: 142.

**4.** The antibody according to any one of the preceding claims, which is an IgG antibody.

**5.** The antigen-binding fragment according to any one of the preceding claims, which is an scFv, Fab, Fab' fragment or a $F(ab')_2$ fragment.

**6.** An antibody-drug conjugate, comprising the antibody or the antigen-binding fragment according to any one of the claims 1 to 5.

**7.** An isolated nucleic acid that encodes the antibody or the antigen-binding fragment according to any one of the claims 1 to 5.

**8.** A vector comprising the nucleic acid according to claim 7.

**9.** An isolated cell expressing the antibody or the antigen-binding fragment according to any one of the claims 1 to 5 and /or comprising the nucleic acid according to claim 7 or the vector according to claim 8.

**10.** The isolated cell according to claim 9, wherein said cell is a prokaryotic or a eukaryotic cell.

**11.** A method of producing the antibody or the antigen-binding fragment according to any one of the claims 1 to 5 comprising culturing of the cell according to claim 10 and purification of the antibody or antigen-binding fragment.

**12.** An antibody or an antigen-binding fragment according to any one of the claims 1 to 5 or an antibody-drug conjugate according to claim 6 for use as a medicament.

**13.** An antibody or an antigen-binding fragment according to any one of the claims 1 to 5 or an antibody-drug conjugate according to claim 6 for use as a medicament for the treatment of cancer.

**14.** An antibody or an antigen antigen-binding fragment according to any one of the claims 1 to 5 for use as a diagnostic agent.

**15.** A pharmaceutical composition comprising the antibody or the antigen-binding fragment according to any one of the claims 1 to 5 or the antibody-drug conjugate according to claim 6.

**Figure 1**

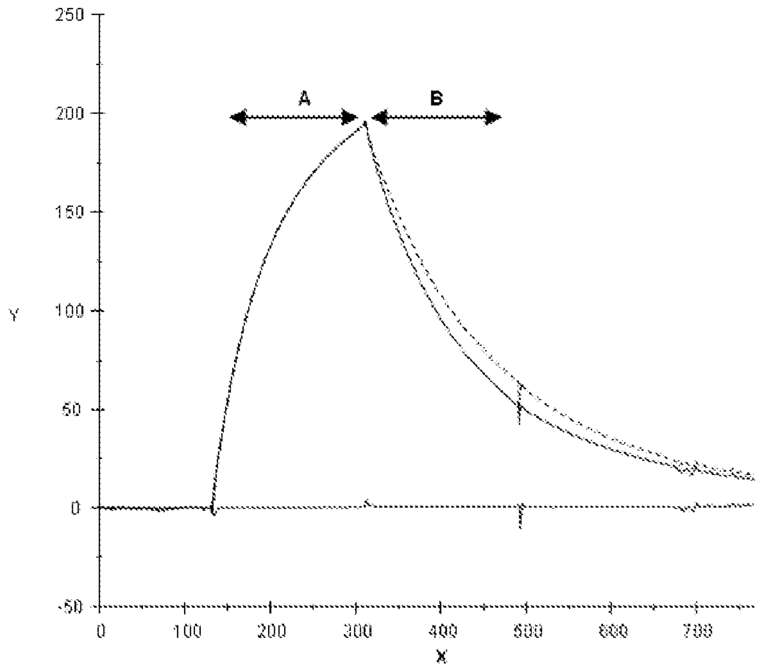

Figure 2

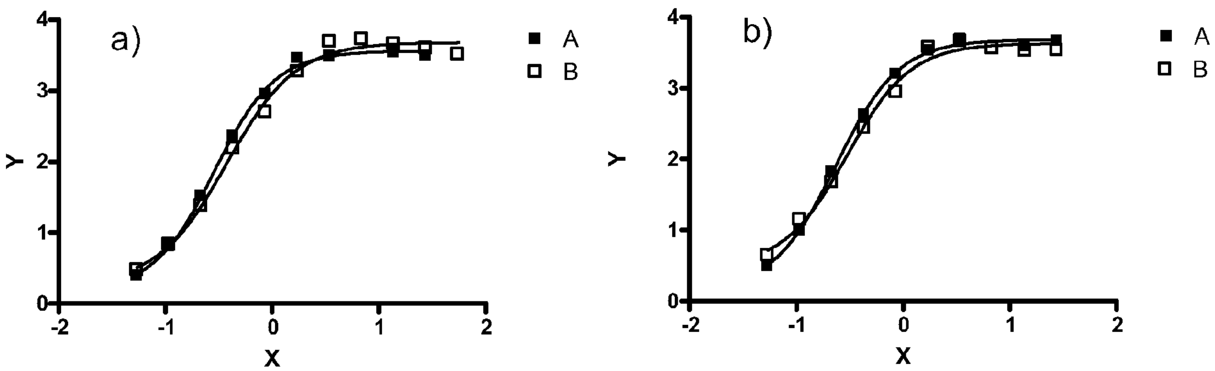

# Figure 3

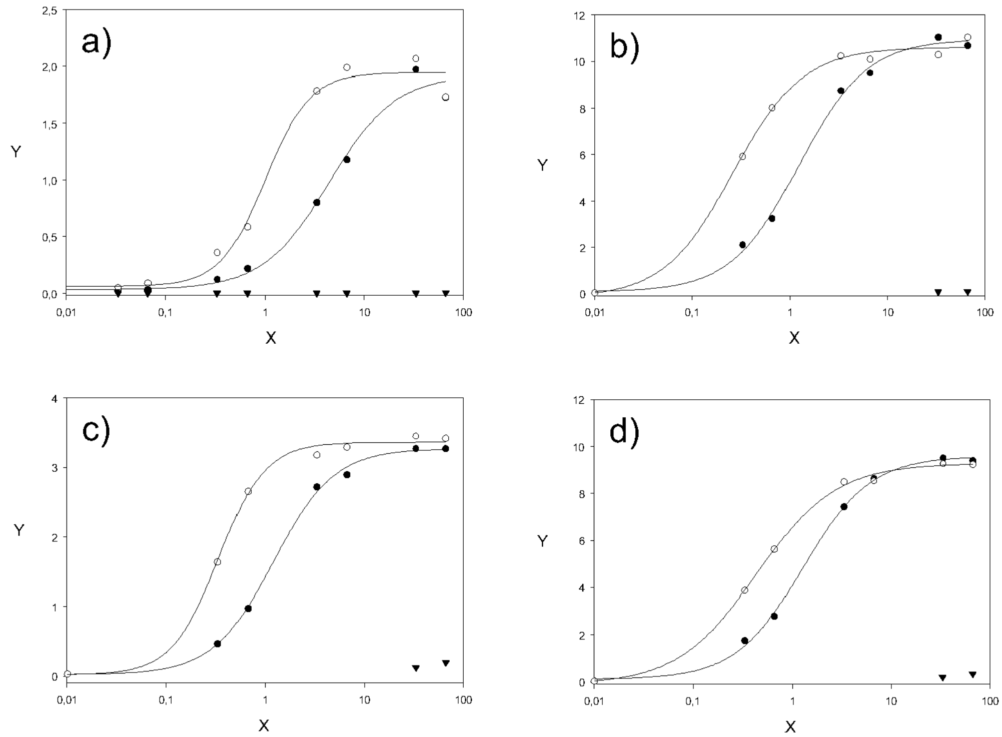


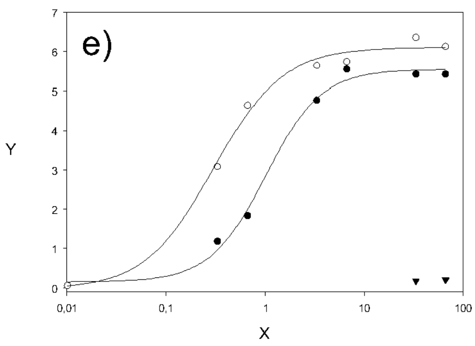


**f)**

| Tumor celllines | EC50 (nM) M31-B01 | M20-D02S-A |
|---|---|---|
| A549:C4.4a | 1.9 | 4.3 |
| NCI H322 | 0.3 | 1.2 |
| NCI H292 | 0.3 | 1.3 |
| H1975/BCRP | 0.4 | 1.3 |
| BxPC3 | 0.3 | 1.0 |

**Figure 4**

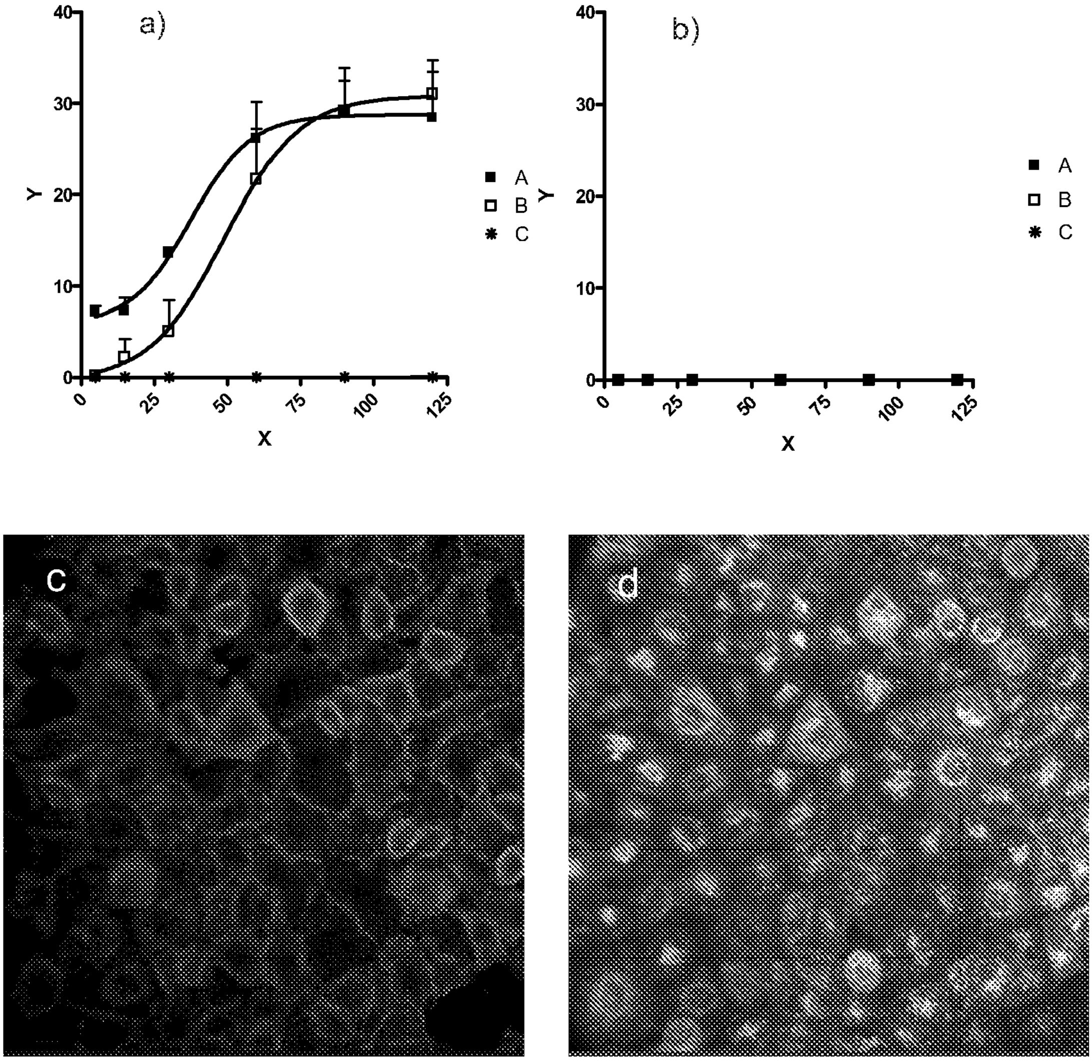

**Figure 5**

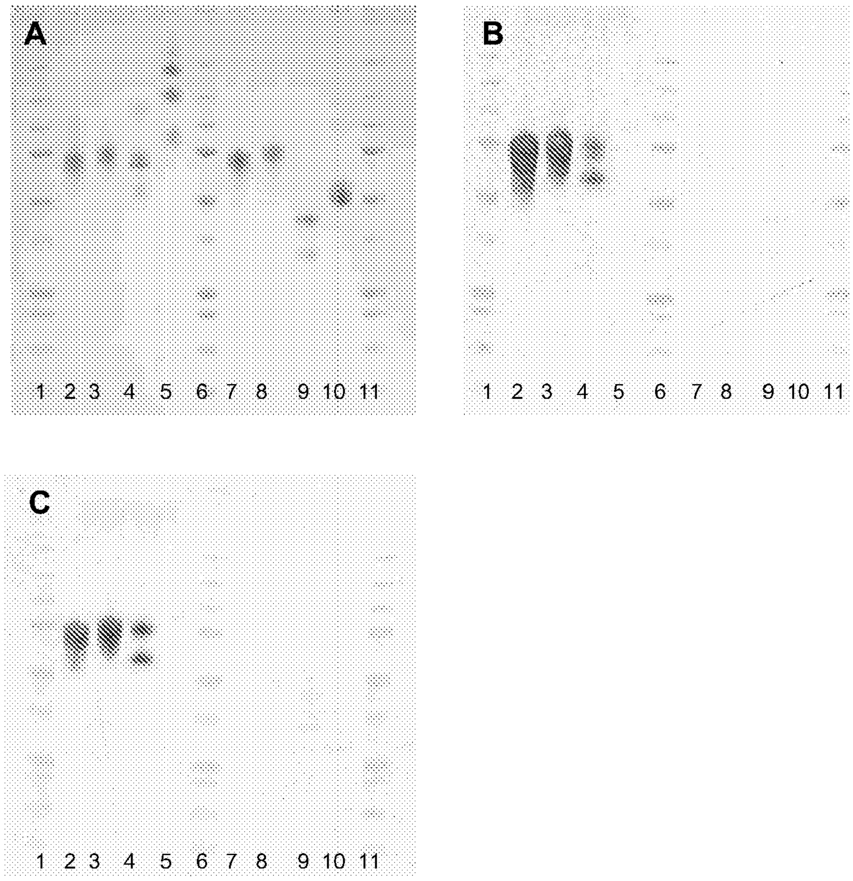

**Figure 6:**

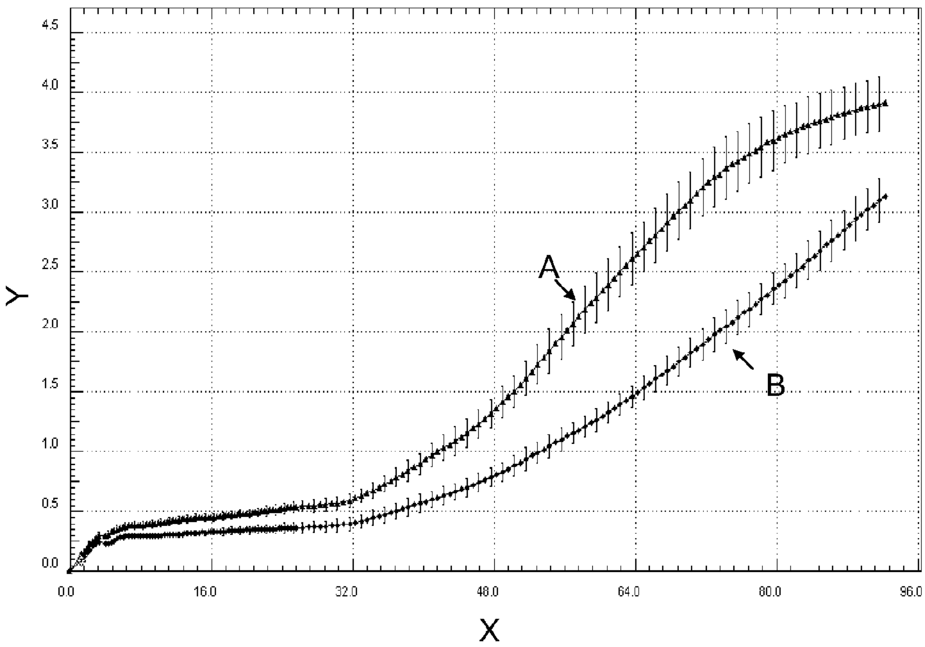


**Figure 7:**

SEQ ID NO:1 (polypeptide):
lecyscvqkaddgcspnkmktvkcapgvdvcteavgavetihgqfslavrgcgsglpgkndrgldlh
gllafiqlqqcaqdrcnaklnltsraldpagnesayppngvecyscvglsreacqgtsppvvscyna
sdhvykgcfdgnvtltaanvtvslpvrgcvqdefctrdgvtgpgftlsgsccqgsrcnsdlrnktyf
spripplvrlpppepttvasttsvttstsapvrptsttkpmpaptsqtprqgveheasrdeeprltg
gaaghqdrsn

SEQ ID NO:2 (polypeptide):
lecyscvqkaddgcsphrmktvkcgpgvdvcteavgavetihgqfsvavrgcgsgipgkndrgldlh
gllaffqlqqcsedrcnaklnltlrglnpagnesayepngaecyscvglsrekcqgsmppvvncyna
sgrvykgcfdgnvtltaanvtvslpvrgcvqdetctrdgvtgpgftlsgsccqgprcnadlrnktyf
spripplvllppttaapstraqnsssttstaaptttsiikpttaqashtsphemdleviqeegas
lsggaaghggtaghggaaghqdrsn

SEQ ID NO:3 (polypeptide):
mdparkagaqamiwtagwllllllrggaqalecyscvqkaddgcspnkmktvkcapgvdvcteavga
vetihgqfslavrgcgsglpgkndrgldlhgllafiqlqqcaqdrcnaklnltsraldpagnesayp
pngvecyscvglsreacqgtsppvvscynasdhvykgcfdgnvtltaanvtvslpvrgcvqdefctr
dgvtgpgftlsgsccqgsrcnsdlrnktyfspripplvrlpppepttvasttsvttstsapvrptst
tkpmpaptsqtprqgveheasrdeeprltggaaghqdrsnsgqypakggpqqphnkgcvaptaglaa
lllavaagvll

SEQ ID NO:4 (polypeptide):
mdaarrgdtqpvmwttgwllllplllcegaqalecyscvqkaddgcsphrmktvkcgpgvdvcteav
gavetihgqfsvavrgcgsgipgkndrgldlhgllaffqlqqcsedrcnaklnltlrglnpagnesa
yepngaecyscvglsrekcqgsmppvvncynasgrvykgcfdgnvtltaanvtvslpvrgcvqdetc
trdgvtgpgftlsgsccqgprcnadlrnktyfspripplvllppttaapstraqnsssttstaapt
tttsiikpttaqashtsphemdleviqeegaslsggaaghggtaghggaaghqdrsnmekypgkgga
qipakggsgtlgswlsavlltvvagaml

SEQ ID NO:5 (polypeptide):

FSNAWMSWV

SEQ ID NO:6 (polypeptide):
FSDYQMTWI

SEQ ID NO:7 (polypeptide):
FGHYYMFWI

SEQ ID NO:8 (polypeptide):
FSSNYMSW

SEQ ID NO:9 (polypeptide):
VSYISSSGSTIYYADSVKGR

SEQ ID NO:10 (polypeptide):
VSGVSWNGARTHYADSVKGR

SEQ ID NO:11 (polypeptide):
VSAISGSGYSTHYADSVKGR

SEQ ID NO:12 (polypeptide):
VSAISSSGSSTYYADSVKGR

SEQ ID NO:13 (polypeptide):
AREGLWAFDY

SEQ ID NO:14 (polypeptide):
AKGDYLVYSAYYFDS

SEQ ID NO:15 (polypeptide):
ARLPYGSQSGVDY

SEQ ID NO:16 (polypeptide):
ARESGGSGPNYYYGMDV

SEQ ID NO:17 (polypeptide):
TGSSSNIGAGYVVH

SEQ ID NO:18 (polypeptide):
SGSSSNVGSNPVN

SEQ ID NO:19 (polypeptide):
TGSSSNIGAGYVVH

SEQ ID NO:20 (polypeptide):
TGSSSNIGAGYVVH

SEQ ID NO:21 (polypeptide):
DNNKRPS

SEQ ID NO:22 (polypeptide):
RNNQRPS

SEQ ID NO:23 (polypeptide):
SNNQRPS

SEQ ID NO:24 (polypeptide):
SNNQRPS

SEQ ID NO:25 (polypeptide):
CAAWDDRLNGPV

SEQ ID NO:26 (polypeptide):

CAAWDDRLNGWV

SEQ ID NO:27 (polypeptide):
CQSYDSSHVL

SEQ ID NO:28 (polypeptide):
CQSYDRSLRGWV

SEQ ID NO:29 (polypeptide):
QSvltqppsasgtpgqrvtisctgsssnigagyvvhwyqqlpgtapklliydnnkrpsgvpdrfsgs
ksgtsaslaisglrsedeadyycaawddrlngpvfgggtkltvlgq

SEQ ID NO:30 (polypeptide):
QSVLTQPPSASGTPGQRVTISCSGSSSNVGSNPVNWYQQLPGTAPKLLIYRNNQRPSGVPDRFSGSK
SGTSASLAISGLRSEDEADYYCAAWDDRLNGWVFGGGTKLTVLGQ

SEQ ID NO:31 (polypeptide):
qsvltqppsasgtpgqrvtisctgsssnigagyvvhwyqqlpgtapklliysnnqrpsgvpdrfsgs
ksgtsaslaisglrsedeadyycqsydsshvlfgggtkltvlgq

SEQ ID NO:32 (polypeptide):
qsvltqppsasgtpgqrvtisctgsssnigagyvvhwyqqlpgtapklliysnnqrpsgvpdrfsgs
ksgtsaslaisglrsedeadyycqsydrslrgwvfgggtkltvlgq

SEQ ID NO:33 (polypeptide):
evqllesggglvqpggslrlscaasgftfsnawmswvrqapgkglewvsyisssgstiyyadsvkgr
ftisrdnskntlylqmnslraedtavyycareglwafdywgqgtlvtvts

SEQ ID NO:34 (polypeptide):
evqllesggglvqpggslrlscaasgftfsdyqmtwirqtpgkglewvsgvswngArthyadsvkgr
ftisrdnskntlylqmnslraedtavyycakgdylvysayyfdswgqgtlvtvts

SEQ ID NO:35 (polypeptide):
evqllesggglvqpggslrlscaasgftfghyymfwirqapgkglewvsaisgsgysthyadsvkgr
ftisrdnskntlylqmnslraedtavyycarlpygsqsgvdywgqgtlvtvts

SEQ ID NO:36 (polypeptide):
evqllesggglvqpggslrlscaasgftfssnymswvrqapgkglewvsaisssgsstyyadsvkgr
ftisrdnskntlylqmnslraedtavyycaresggsgpnyyygmdvwgqgtlvtvts

SEQ ID NO:37 (DNA):
cagtctgtgctgactcagccaccctcagcgtctgggacccctgggcagagggtcaccatctcctgca
ctgggagcagctccaacattggggcgggttatgttgtacattggtatcagcagctcccaggaacggc
ccccaaactcctcatctatgacaataataagcgaccctcaggggtccctgaccgattctctggctcc
aagtctggcacctcagcctccctggccatcagtgggctccggtccgaggatgaggctgattattact
gtgcagcatgggatgacaggctgaatggtccggtgttcggcggaggaaccaagttaaccgtcctagg
tcag

SEQ ID NO:38 (DNA):
cagtctgtgctgactcagccaccctcagcgtctgggacccccgggcagagggtcaccatctcttgtt
ctggaagcagctccaacgtcgggagtaatcctgtaaactggtatcagcagctcccaggaacggcccc
caaactcctcatctataggaataatcagcggccctcaggggtccctgaccgattctctggctccaag
tctggcacctcagcctccctggccatcagtgggctccggtccgaggatgaggctgattattactgtg
cagcatgggatgacaggctgaatggttgggtgttcggcggaggaaccaagctgacggtcctaggtca
g

SEQ ID NO:39 (DNA):
cagtctgtgctgactcagccaccctcagcgtctgggacccccgggcagagggtcaccatctcctgca
ctgggagcagctccaacattggggcgggttatgttgtacattggtatcagcagctcccaggaacggc
ccccaaactcctcatctatagtaataatcagcggccctcaggagtccctgaccgattctctggctcc
aagtctggcacctcagcctccctggccatcagtgggctccggtccgaggatgaggctgattattact
gccagtcctatgacagcagccatgttttattcggcggaggaaccaagctgacggtcctaggtcag

SEQ ID NO:40 (DNA):
cagtctgtgctgactcagccaccctcagcgtctgggacccccgggcagagggtcaccatctcctgca
ctgggagcagctccaacattggggcgggttatgttgtacattggtatcagcagctcccaggaacggc
ccccaaactcctcatctatagtaataatcagcggccctcaggggtccctgaccgattctctggctcc
aagtctggcacctcagcctccctggccatcagtgggctccggtccgaggatgaggctgattattact
gccagtcctatgacagaagcctgcgtggttgggtgttcggcggaggaaccaagctgacggtcctagg
tcag

SEQ ID NO:41 (DNA):
GAGGTGCAGCTGTTGGAGTCTGGGGGAGGCTTGGTACAGCCTGGGGGGTCCCTGAGACTCTCCTGTG
CAGCCTCTGGATTCACCTTCAGTAACGCCTGGATGAGCTGGGTCCGCCAGGCTCCAGGGAAGGGGCT
GGAGTGGGTTTCATACATTAGTAGTAGTGGTAGTACCATATACTACGCAGACTCTGTGAAGGGCCGA
TTCACCATCTCCAGAGACAATTCCAAGAACACGCTGTATCTGCAAATGAACAGCCTGAGAGCCGAGG
ACACTGCCGTGTATTACTGTGCGAGAGAAGGGTTATGGGCCTTTGACTACTGGGGCCAGGGTACCCT
GGTCACCGTGACTAGT

SEQ ID NO:42 (DNA):
gaggtgcagctgttggagtctgggggaggcttggtacagcctggggggtccctgagactctcctgtg
cagcctctggattcaccttcagtgactatcagatgacctggatccgccagactccagggaaggggct
ggagtgggtatcgggtgttagttggaatggcGCtaggacgcactatgcagactctgtgaagggccga
ttcaccatctccagagacaattccaagaacacgctgtatctacaaatgaacagcctgagagccgagg
acactgccgtgtattactgtgcgaagggcgactacctggtttactccgcatactactttgactcctg
gggccagggtaccctggtcaccgtgactagt

SEQ ID NO:43 (DNA):
gaggtgcagctgttggagtctgggggaggcttggtacagcctggggggtccctgagactctcctgtg
cagcctctggattcaccttcggtcactactatatgttctggatccgtcaggctccagggaaggggct
ggagtgggtctcagctattagtggtagtggttatagcacacactacgcagactccgtgaagggccgg
ttcaccatctccagagacaattccaagaacacgctgtatctgcaaatgaacagcctgagagccgagg
acactgccgtgtattactgtgcgagactgccatatggttcgcagagtggcgttgactactggggcca
gggtaccctggtcaccgtgactagt

SEQ ID NO:44 (DNA):
gaggtgcagctgttggagtctgggggaggcttggtacagcctggggggtccctgagactctcctgtg
cagcctctggattcaccttcagtagcaactacatgagctgggtccgccaggctccagggaaggggct
ggagtgggtctcagctattagtagtagtggtagtagcacatactacgcagactccgtgaagggccgg
ttcaccatctccagagacaattccaagaacacgctgtatctgcaaatgaacagcctgagagccgagg
acactgccgtgtattactgtgcgagagaatctggtgggagcggaccgaactactactacggtatgga
cgtctggggccaaggtaccctggtcaccgtgactagt

SEQ ID NO:45 (polypeptide):
FSNAWMSWV

SEQ ID NO:46 (polypeptide):
VSYISSSGSTIYYADSVKGR

SEQ ID NO:47 (polypeptide):
AREGLWAFDY

SEQ ID NO:48 (polypeptide):
TGSSSNIGAGYVVH

SEQ ID NO:49 (polypeptide):
DNNKRPS

SEQ ID NO:50 (polypeptide):
CAAWDDRLNGPV

SEQ ID NO:51 (polypeptide):
EVQLLESGGGLVQPGGSLRLSCAASGFTFSNAWMSWVRQAPGKGLEWVSYISSSGSTIYYADSVKGR
FTISRDNSKNTLYLQMNSLRAEDTAVYYCAREGLWAFDYWGQGTLVTVSS

SEQ ID NO:52 (polypeptide):

ESVLTQPPSVSGAPGQRVTISCTGSSSNIGAGYVVHWYQQLPGTAPKLLIYDNNKRPSGVPDRFSGS
KSGTSASLAISGLRSEDEADYYCAAWDDRLNGPVFGGGTKLTVLGQ

SEQ ID NO:53 (DNA):
gaggtgcagctgctggaaagcggcggaggactggtgcagcctggaggcagcctgagactgtcttgcg
ccgccagcggcttcaccttcagcaacgcctggatgagctgggtccgacaggctcctggcaagggcct
ggaatgggtgtcctacatcagcagcagcggcagcaccatctactacgccgacagcgtgaagggccgg
ttcaccatcagccgggacaacagcaagaacaccctgtacctgcagatgaacagcctgcgggccgagg
acaccgccgtgtactactgcgccagagaaggcctgtgggccttcgactactggggccagggcaccct
ggtcaccgtgtctagc

SEQ ID NO:54 (DNA):
gagagcgtgctgacccagcctcctagcgtgtccggcgctcctggccagagagtgaccatcagctgca
ccggcagcagcagcaacatcggagccggctacgtggtgcactggtatcagcagctgcccggcaccgc
ccccaagctgctgatctacgacaacaacaagcggcctagcggcgtgcccgacagattcagcggcagc
aagagcggcaccagcgccagcctggccatcagcggcctgagaagcgaggacgaggccgactactact
gcgccgcctgggacgacagactgaacggccctgtgttcggcggaggcaccaagctgaccgtgctggg
acag

SEQ ID NO:55 (polypeptide):
FSNAWMSWV

SEQ ID NO:56 (polypeptide):
VSYISSSGSTIYYADSVKGR

SEQ ID NO:57 (polypeptide):
AREGLWAFDY

SEQ ID NO:58 (polypeptide):
TGSSSNIGAGYVVH

SEQ ID NO:59 (polypeptide):
DNNKRPS

SEQ ID NO:60 (polypeptide):
CAAYDDSLSGPV

SEQ ID NO:61 (polypeptide):
EVQLLESGGGLVQPGGSLRLSCAASGFTFSNAWMSWVRQAPGKGLEWVSYISSSGSTIYYADSVKGR
FTISRDNSKNTLYLQMNSLRAEDTAVYYCAREGLWAFDYWGQGTLVTVSS

SEQ ID NO:62 (polypeptide):
QSVLTQPPSVSGAPGQRVTISCTGSSSNIGAGYVVHWYQQLPGTAPKLLIYDNNKRPSGVPDRFSGS
KSGTSASLAISGLRSEDEADYYCAAYDDSLSGPVFGGGTKLTVLGQ

SEQ ID NO:63 (DNA):
gaggtgcagctgctggaatccggcggaggcctggtgcagcctggcggatctctgagactgtcctgcg
ccgccagcggctttaccttctccaacgcctggatgtcctgggtccgacaggcccctggcaagggact
ggaatgggtgtcctacatctcctcctccggctccaccatctactacgccgactccgtgaagggccgg
ttcaccatctcccgggacaactccaagaacaccctgtacctgcagatgaactccctgcgggccgagg
acaccgccgtgtactactgcgcccgagagggcctgtgggccttcgattattggggccagggcaccct
ggtcaccgtcagctca

SEQ ID NO:64 (DNA):
cagtccgtgctgacccagccccttctgtgtctggcgcccctggccagagagtgaccatctcttgca
ccggctcctccagcaacatcggcgctggctacgtggtgcactggtatcagcagctgcccggcaccgc
ccccaagctgctgatctacgacaacaacaagcggccctccggcgtgcccgacagattctccggctcc
aagtccggcacctccgcctccctggccatctccggcctgagatctgaggacgaggccgactactact
gcgccgcctacgacgactccctgtccggccctgtgttcggcggaggcacaaagttaaccgtgctggg
ccag

SEQ ID NO:65 (polypeptide):
FSNAWMSWV

SEQ ID NO:66 (polypeptide):
VSYISSSGSTIYYADSVKGR

SEQ ID NO:67 (polypeptide):
AREGLWAFDY

SEQ ID NO:68 (polypeptide):
TGSSSNIGAGYVVH

SEQ ID NO:69 (polypeptide):
DNNKRPS

SEQ ID NO:70 (polypeptide):
CAAFDDSLNGPV

SEQ ID NO:71 (polypeptide):
EVQLLESGGGLVQPGGSLRLSCAASGFTFSNAWMSWVRQAPGKGLEWVSYISSSGSTIYYADSVKGR
FTISRDNSKNTLYLQMNSLRAEDTAVYYCAREGLWAFDKWGQGTLVTVSS

SEQ ID NO:72 (polypeptide):
QSVLTQPPSASGTPGQRVTISCTGSSSNIGAGYVVHWYQQLPGTAPKLLIYDNNKRPSGVPDRFSGS
KSGTSASLAISGLRSEDEADYYCAAFDDRLNGPVFGGGTKLTVLGQ

SEQ ID NO:73 (DNA):
gaggtgcagctgctggaatccggcggaggcctggtgcagcctggcggatctctgagactgtcctgcg
ccgcctccggctttaccttctccaacgcctggatgtcctgggtccgacaggctcctggcaagggcct
ggaatgggtgtcctacatctcctcctccggctccaccatctactacgccgactccgtgaagggccgg
ttcaccatctcccgggacaactccaagaacaccctgtacctgcagatgaactccctgcgggccgagg
acaccgccgtgtactactgcgcccgagagggcctgtgggccttcgataagtggggccagggcaccct
ggtcaccgtcagctca

SEQ ID NO:74 (DNA):
cagtccgtgctgacccagcctccttccgcctctggcacccctggccagagagtgaccatctcctgca
ccggctcctccagcaacatcggcgctggctacgtggtgcactggtatcagcagctgcccggcaccgc
ccccaagctgctgatctacgacaacaacaagcggccctccggcgtgcccgacagattctccggctcc
aagtccggcacctccgcctccctggccatctccggcctgagatctgaggacgaggccgactactact
gcgccgccttcgacgaccggctgaacggccctgtgttcggcggaggcacaaagttaaccgtgctggg
ccag

SEQ ID NO:75 (polypeptide):
FSSAWMSWV

SEQ ID NO:76 (polypeptide):
VSYISSSGSTIYYADSVKGR

SEQ ID NO:77 (polypeptide):
AREGLWAFDY

SEQ ID NO:78 (polypeptide):
TGSSSNIGAGYVVH

SEQ ID NO:79 (polypeptide):
DNNKRPS

SEQ ID NO:80 (polypeptide):
CAAYDDSLSGPV

SEQ ID NO:81 (polypeptide):
EVQLLESGGGLVQPGGSLRLSCAASGFTFSSAWMSWVRQAPGKGLEWVSYISSSGSTIYYADSVKGR
FTISRDNSKNTLYLQMNSLRAEDTAVYYCAREGLWAFDYWGQGTLVTVSS

SEQ ID NO:82 (polypeptide):

QSVLTQPPSVSGAPGQRVTISCTGSSSNIGAGYVVHWYQQLPGTAPKLLIYDNNKRPSGVPDRFSGS
KSGTSASLAISGLRSEDEADYYCAAYDDSLSGPVFGGGTKLTVLGQ

SEQ ID NO:83 (DNA):
gaggtgcagctgctggaatccggcggaggcctggtgcagcctggcggatctctgagactgtcctgcg
ccgccagcggctttaccttctccagcgcctggatgtcctgggtccgacaggcccctggcaagggact
ggaatgggtgtcctacatctcctcctccggctccaccatctactacgccgactccgtgaagggccgg
ttcaccatctcccgggacaactccaagaacaccctgtacctgcagatgaactccctgcgggccgagg
acaccgccgtgtactactgcgcccgagagggcctgtgggccttcgattattggggccagggcaccct
ggtcaccgtcagctca

SEQ ID NO:84 (DNA):
cagtccgtgctgacccagccccccttctgtgtctggcgcccctggccagagagtgaccatctcttgca
ccggctcctccagcaacatcggcgctggctacgtggtgcactggtatcagcagctgcccggcaccgc
ccccaagctgctgatctacgacaacaacaagcggccctccggcgtgcccgacagattctccggctcc
aagtccggcacctccgcctccctggccatctccggcctgagatctgaggacgaggccgactactact
gcgccgcctacgacgactccctgtccggccctgtgttcggcggaggcacaaagttaaccgtgctggg
ccag

SEQ ID NO:85 (polypeptide):
FSSAWMSWV

SEQ ID NO:86 (polypeptide):
VSYISSSGSSTYYADSVKGR

SEQ ID NO:87 (polypeptide):
AREGLWAFDK

SEQ ID NO:88 (polypeptide):
SGSSSNIGAGYVVH

SEQ ID NO:89 (polypeptide):
DNNQRPS

SEQ ID NO:90 (polypeptide):
CAAFDDRLSGPV

SEQ ID NO:91 (polypeptide):
EVQLLESGGGLVQPGGSLRLSCAASGFTFSSAWMSWVRQAPGKGLEWVSYISSSGSSTYYADSVKGR
FTISRDNSKNTLYLQMNSLRAEDTAVYYCAREGLWAFDKWGQGTLVTVSS

SEQ ID NO:92 (polypeptide):
QSVLTQPPSASGTPGQRVTISCSGSSSNIGAGYVVHWYQQLPGTAPKLLIYDNNQRPSGVPDRFSGS
KSGTSASLAISGLRSEDEADYYCAAFDDRLSGPVFGGGTKLTVLGQ

SEQ ID NO:93 (DNA):
gaggtgcagctgctggaaagcggcggaggcctggtgcagcctggcggaagcctgagactgagctgtg
ccgccagcggcttcaccttcagcagcgcctggatgagctgggtccgacaggcccctggcaagggcct
ggaatgggtgtcctacatcagcagcagcggcagcagcacctactacgccgacagcgtgaagggccgg
ttcaccatcagccgggacaacagcaagaacaccctgtacctgcagatgaacagcctgcgggccgagg
acaccgccgtgtactactgcgccagagaaggcctgtgggccttcgataagtggggccagggcaccct
ggtcaccgtcagctca

SEQ ID NO:94 (DNA):
cagagcgtgctgacccagcctcctagcgcctctggcacccctggccagagagtgaccatcagctgca
gcggcagcagcagcaacatcggagccggctacgtggtgcactggtatcagcagctgcccggcaccgc
ccccaagctgctgatctacgacaacaaccagcggcccagcggcgtgcccgacagattttccggcagc
aagagcggcaccagcgccagcctggccatcagcggcctgagaagcgaggacgaggccgactactact
gcgccgccttcgacgacagactgagcggccctgtgttcggcggaggcacaaagttaaccgtgctggg
ccag

SEQ ID NO:95 (polypeptide):
FSDYQMTWI

SEQ ID NO:96 (polypeptide):
VSGVSWNGARTHYADSVKGR

SEQ ID NO:97 (polypeptide):
AKGDYLVYSAYYFDS

SEQ ID NO:98 (polypeptide):
SGSSSNVGSNPVN

SEQ ID NO:99 (polypeptide):
RNNQRPS

SEQ ID NO:100 (polypeptide):
CAAWDDRLNGWV

SEQ ID NO:101 (polypeptide):
EVQLLESGGGLVQPGGSLRLSCAASGFTFSDYQMTWIRQTPGKGLEWVSGISWNGGSTHYADSVKGR
FTISRDNSKNTLYLQMNSLRAEDTAVYYCAKGDYLVYSAYYFDSWGQGTLVTVSS

SEQ ID NO:102 (polypeptide):
ESVLTQPPSASGTPGQRVTISCSGSSSNIGSNPVNWYQQLPGTAPKLLIYRNNQRPSGVPDRFSGSK
SGTSASLAISGLRSEDEADYYCAAWDDRLNGWGFGGGTKLTVLGQ

SEQ ID NO:103 (DNA):
gaggtgcagctgctggaaagcggcggaggactggtgcagcctggaggcagcctgagactgtcttgcg
ccgccagcggcttcaccttcagcgactaccagatgacctggatccgacagacccctggcaagggcct
ggaatgggtgtccggcatcagctggaacggaggcagcacccactacgccgacagcgtgaagggccgg
ttcaccatcagccgggacaacagcaagaacaccctgtacctgcagatgaacagcctgcgggccgagg
acaccgccgtgtactactgcgccaagggcgactacctggtgtacagcgcctactacttcgacagctg
ggccagggcaccctggtcaccgtgtctagc

SEQ ID NO:104 (DNA):
gagagcgtgctgacccagcctcctagcgcctctggcacccctggccagagagtgaccatcagctgct
ctggcagcagcagcaacatcggaagcaaccccgtgaactggtatcagcagctgcccggcaccgcccc
caagctgctgatctaccggaacaaccagcggcctagcggcgtgcccgacagattcagcggcagcaag
agcggcaccagcgccagcctggccatcagcggcctgagaagcgaggacgaggccgactactactgcg
ccgcctgggacgacagactgaacggctggggcttcggcggaggcaccaagctgaccgtgctgggaca
g

SEQ ID NO:105 (polypeptide):
FSDYQMTWI

SEQ ID NO:106 (polypeptide):
VSGISWNGGSTHYADSVKGR

SEQ ID NO:107 (polypeptide):
AKGDYLVYSSYYFKS

SEQ ID NO:108 (polypeptide):
SGSSSNIGSNPVN

SEQ ID NO:109 (polypeptide):
RNNQRPS

SEQ ID NO:110 (polypeptide):
CAAWDDRLSGWA

SEQ ID NO:111 (polypeptide):
EVQLLESGGGLVQPGGSLRLSCAASGFTFSDYQMTWIRQTPGKGLEWVSGISWNGGSTHYADSVKGR
FTISRDNSKNTLYLQMNSLRAEDTAVYYCAKGDYLVYSSYYFKSWGQGTLVTVSS

SEQ ID NO:112 (polypeptide):

QSVLTQPPSASGTPGQRVTISCSGSSSNIGSNPVNWYQQLPGTAPKLLIYRNNQRPSGVPDRFSGSK
SGTSASLAISGLRSEDEADYYCAAWDDRLSGWAFGGGTKLTVLGQ

SEQ ID NO:113 (DNA):
gaggtgcagctgctggaatccggcggaggcctggtgcagcctggcggatctctgagactgtcctgcg
ccgcctccggcttcaccttctccgactaccagatgacctggatcagacagacccccggcaagggcct
ggaatgggtgtccggcatctcctggaacggcggctccacccactacgccgactctgtgaagggccgg
ttcaccatctcccgggacaactccaagaacaccctgtacctgcagatgaactccctgcgggccgagg
acaccgccgtgtactactgcgccaagggcgactacctggtgtactcctcctactacttcaagtcctg
gggccagggcaccctggtcaccgtcagctca

SEQ ID NO:114 (DNA):
cagtccgtgctgacccagcctccttccgcctctggcacccctggccagagagtgaccatctcctgct
ccggctcctcctccaacatcggctccaaccccgtgaactggtatcagcagctgcccggcaccgcccc
caagctgctgatctaccggaacaaccagcggccctccggcgtgcccgacagattctccggctccaag
tccggcacctccgcctccctggccatctccggcctgagatctgaggacgaggccgactactactgcg
ccgcctgggacgaccggctgtctggctgggcttttggcggcggaacaaagttaaccgtgctgggcca
g

SEQ ID NO:115 (polypeptide):
FSDYQMTWI

SEQ ID NO:116 (polypeptide):
VSGISWNGGSTHYADSVKGR

SEQ ID NO:117 (polypeptide):
AKGDYLVYKSYYFKS

SEQ ID NO:118 (polypeptide):
SGSSSNIGSNPVN

SEQ ID NO:119 (polypeptide):
RNNQRPS

SEQ ID NO:120 (polypeptide):
CAAWDDSLSGWA

SEQ ID NO:121 (polypeptide):
EVQLLESGGGLVQPGGSLRLSCAASGFTFSDYQMTWIRQTPGKGLEWVSGISWNGGSTHYADSVKGR
FTISRDNSKNTLYLQMNSLRAEDTAVYYCAKGDYLVYKSYYFKSWGQGTLVTVSS

SEQ ID NO:122 (polypeptide):
QSVLTQPPSASGTPGQRVTISCSGSSSNIGSNPVNWYQQLPGTAPKLLIYRNNQRPSGVPDRFSGSK
SGTSASLAISGLRSEDEADYYCAAWDDSLSGWAFGGGTKLTVLGQ

SEQ ID NO:123 (DNA):
gaggtgcagctgctggaatccggcggaggcctggtgcagcctggcggatctctgagactgtcctgcg
ccgcctccggcttcaccttctccgactaccagatgacctggatcagacagacccccggcaagggcct
ggaatgggtgtccggcatctcctggaacggcggctccacccactacgccgactctgtgaagggccgg
ttcaccatctcccgggacaactccaagaacaccctgtacctgcagatgaactccctgcgggccgagg
acaccgccgtgtactactgcgccaagggcgactacctggtgtacaagtcctactacttcaagtcctg
gggccagggcaccctggtcaccgtcagctca

SEQ ID NO:124 (DNA):
cagtccgtgctgacccagcctccttccgcctctggcacccctggccagagagtgaccatctcctgct
ccggctcctcctccaacatcggctccaaccccgtgaactggtatcagcagctgcccggcaccgcccc
caagctgctgatctaccggaacaaccagcggccctccggcgtgcccgacagattctccggctccaag
tccggcacctccgcctccctggccatctccggcctgagatctgaggacgaggccgactactactgcg
ccgcctgggacgactccctgtctggctgggcttttggcggcggaacaaagttaaccgtgctgggcca
g

SEQ ID NO:125 (polypeptide):
FSDYQMTWI

SEQ ID NO:126 (polypeptide):
VSGISWNGGSTHYADSVKGR

SEQ ID NO:127 (polypeptide):
AKGDYLVYSSYYFKS

SEQ ID NO:128 (polypeptide):
SGSSSNIGSNPVN

SEQ ID NO:129 (polypeptide):
RNNQRPS

SEQ ID NO:130 (polypeptide):
CAAWDDRLSGWG

SEQ ID NO:131 (polypeptide):
EVQLLESGGGLVQPGGSLRLSCAASGFTFSDYQMTWIRQTPGKGLEWVSGISWNGGSTHYADSVKGR
FTISRDNSKNTLYLQMNSLRAEDTAVYYCAKGDYLVYSSYYFKSWGQGTLVTVSS

SEQ ID NO:132 (polypeptide):
QSVLTQPPSASGTPGQRVTISCSGSSSNIGSNPVNWYQQLPGTAPKLLIYRNNQRPSGVPDRFSGSK
SGTSASLAISGLRSEDEADYYCAAWDDRLSGWGFGGGTKLTVLGQ

SEQ ID NO:133 (DNA):
gaggtgcagctgctggaatccggcggaggcctggtgcagcctggcggatctctgagactgtcctgcg
ccgcctccggcttcaccttctccgactaccagatgacctggatcagacagacccccggcaagggcct
ggaatgggtgtccggcatctcctggaacggcggctccacccactacgccgactctgtgaagggccgg
ttcaccatctcccgggacaactccaagaacaccctgtacctgcagatgaactccctgcgggccgagg
acaccgccgtgtactactgcgccaagggcgactacctggtgtactcctcctactacttcaagtcctg
gggccagggcaccctggtcaccgtcagctca

SEQ ID NO:134 (DNA):
cagtccgtgctgacccagcctccttccgcctctggcacccctggccagagagtgaccatctcctgct
ccggctcctcctccaacatcggctccaaccccgtgaactggtatcagcagctgcccggcaccgcccc
caagctgctgatctaccggaacaaccagcggccctccggcgtgcccgacagattctccggctccaag
tccggcacctccgcctccctggccatctccggcctgagatctgaggacgaggccgactactactgcg
ccgcctgggacgaccggctgtctggctggggatttggcggcggaacaaagttaaccgtgctgggcca
g

SEQ ID NO:135 (polypeptide):
FSSYQMTWI

SEQ ID NO:136 (polypeptide):
VSGISWNGGSTHYADSVKGR

SEQ ID NO:137 (polypeptide):
AKGDYLVYKSYYFKS

SEQ ID NO:138 (polypeptide):
SGSSSNIGSNPVN

SEQ ID NO:139 (polypeptide):
RNNQRPS

SEQ ID NO:140 (polypeptide):
CAAWDDSLSGWA

SEQ ID NO:141 (polypeptide):
EVQLLESGGGLVQPGGSLRLSCAASGFTFSSYQMTWIRQAPGKGLEWVSGISWNGGSTHYADSVKGR
FTISRDNSKNTLYLQMNSLRAEDTAVYYCAKGDYLVYKSYYFKSWGQGTLVTVSS

SEQ ID NO:142 (polypeptide):

QSVLTQPPSASGTPGQRVTISCSGSSSNIGSNPVNWYQQLPGTAPKLLIYRNNQRPSGVPDRFSGSK
SGTSASLAISGLRSEDEADYYCAAWDDSLSGWAFGGGTKLTVLGQ

SEQ ID NO:143 (DNA):
gaggtgcagctgctggaaagcggcggaggcctggtgcagcctggcggaagcctgagactgagctgtg
ccgccagcggcttcaccttcagcagctaccagatgacctggatcagacaggcccctggcaagggcct
ggaatgggtgtccggcatcagctggaacggcggcagcacccactacgccgacagcgtgaagggccgg
ttcaccatcagccgggacaacagcaagaacaccctgtacctgcagatgaacagcctgcgggccgagg
acaccgccgtgtactactgcgccaagggcgactacctggtgtacaagagctactacttcaagagctg
gggccagggcacactggtcaccgtcagctca

SEQ ID NO:144 (DNA):
cagagcgtgctgacccagcctcctagcgcctctggcacccctggccagagagtgaccatcagctgca
gcggcagcagcagcaacatcggcagcaaccccgtgaactggtatcagcagctgcccggcaccgcccc
caagctgctgatctaccggaacaaccagcggcccagcggcgtgcccgacagattttccggcagcaag
agcggcaccagcgccagcctggccatcagcggcctgagaagcgaggacgaggccgactactactgcg
ccgcctgggacgatagcctgagcggctgggcctttggcggcggaacaaagttaaccgtgctgggcca
g

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0123553 A
- WO 9708320 A
- US 5500362 A
- US 5821337 A
- US 6737056 B, Presta
- US 6194551 B1
- WO 199951642 A
- US 4816397 A, Boss
- US 5168062 A, Stinski
- US 4510245 A, Bell
- US 4968615 A, Schaffner
- US 4399216 A
- US 4634665 A
- US 5179017 A, Axel
- US 4657760 A
- US 5206344 A
- US 5225212 A


### Non-patent literature cited in the description


- **RÖSEL M. et al.** *Oncogene,* 1998, vol. 17 (15), 1989-2002
- **WÜRFEL, J.** *Gene,* 2001, vol. 262, 35-41
- **JACOBSEN B. ; PLOUG M.** *Current Medicinal Chemistry,* 2008, vol. 15, 2559-2573
- **HANSEN L. et al.** *Biochem J.,* 2004, vol. 380, 845-857
- **WÜRFEL J.** *Gene,* 2001, vol. 262, 35-41
- **HANSEN L. et al.** *Lung Cancer,* 2007, vol. 58, 260-266
- **SEITER S. et al.** *J Invest Dermatol.,* 2001, vol. 116 (2), 344-347
- **FLETCHER G.C.** *Br. J. Cancer,* 2003, vol. 88 (4), 579-585
- **SMITH B. A. et al.** *Cancer Res,* 2001, vol. 61 (4), 1678-1685
- **K. KONISHI et al.** *Cancer Science,* 2010
- **PARET C. et al.** *British Journal of Cancer,* 2007, vol. 97, 1146-1156
- **PARET C.** *Int. J. Cancer,* 2005, vol. 115, 724-733
- **FLETCHER GC.** *Brit. J. Cancer,* 2003, vol. 88, 579-585
- **SÖDERLIND et al.** *Nature Biotech.,* 2000, vol. 18, 853-856
- **KABAT et al.** Sequences of Proteins of Immulological Interest. Public Health Service, National Institutes of Health, 1991
- **CHOTHIA ; LESK.** *J Mol Biol,* 1987, vol. 196, 901-917
- Antibody Engineering (Breakthroughs in Molecular Biology). Oxford University Press, 1995
- Antibody Engineering (Springer Laboratory Manual). Springer Verlag, 2001
- **GAZZANO-SANTORO et al.** *J. Immunol. Methods,* 1996, vol. 202, 163
- **LIU et al.** *Proc Natl. Acad. Sci.,* 1996, vol. 93, 8618-8623
- **SAMBROOK, J. ; FRITSCH, E. F. ; MANIATIS, T.** Molecular Cloning: A laboratory manual. Cold Spring Harbor Laboratory Press, 1989
- **ROBERT K. SCOPES.** Protein Purification, - Principles and Practice. Springer Science and Business Media LLC, 1994
- **KNAPPIK A. et al.** *JMB,* 2000, vol. 296, 57-86
- **VIRNEKÄS B. et al.** *Nucl. Acids Res.,* 1994, vol. 22, 5600
- Current Protocols in Molecular Biology. John Wiley and Sons, 1995
- **KHORANA et al.** *J. Mol. Biol.,* 1971, vol. 72, 209-217
- Molecular Cloning; A Laboratory Manual. Cold Spring Harbor, 1989
- Current Protocols in Molecular Biology. Greene Publishing Associates, 1989
- **KABAT, E. A.** Sequences of Proteins of Immunological Interest. U.S. Department of Health and Human Services, 1991
- **BIRD et al.** *Science,* 1988, vol. 242, 423-426
- **HUSTON et al.** *Proc. Natl. Acad. Sci. USA,* 1988, vol. 85, 5879-5883
- **MCCAFFERTY et al.** *Nature,* 1990, vol. 348, 552-554
- Gene Expression Technology. **GOEDDEL.** Methods in Enzymology. Academic Press, 1990, vol. 185
- **URLAUB ; CHASIN.** *Proc. Natl. Acad. Sci. USA,* 1980, vol. 77, 4216-4220
- **R. J. KAUFMAN ; P. A. SHARP.** *Mol. Biol.,* 1982, vol. 159, 601-621
- **DUROCHER et al.** *Nucl.Acids Res.,* 2002, vol. 30, e9

- **T. BENTON et al.** *Cytotechnology,* 2002, vol. 38, 43-46
- **COLLIGAN.** Current Protocols in Immunology, or Current Protocols in Protein Science. John Wiley & Sons, 1997
- International Conference on Harmonization. REMINGTON'S PHARMACEUTICAL SCIENCES. Mack Pub. Co, 1990, 484-528
- **SODEE et al.** *Clin. Nuc. Med.,* 1997, vol. 21, 759-766
- **HERZOG et al.** *J. Nucl. Med.,* 1993, vol. 34, 2222-2226
- Remington's Pharmaceutical Sciences
- **RIVA et al.** *Clin. Cancer Res.,* 1999, vol. 5, 3275-3280
- **ULANER et al.** *Radiology,* 2008, vol. 246 (3), 895-902
- **SAMBROOK et al.** Molecular Cloning: A Laboratory Manual. Cold Spring Harbor Laboratory Press, 1989
- **SÖDERLING et al.** *Nature Biotech.,* 2000, vol. 18, 853-856
- **CICORTAS GUNNARSSON et al.** *PEDS,* 2004, vol. 17 (3), 213-221